# EUROPEAN PATENT APPLICATION

(11) **EP 3 821 888 A1**
(43) Date of publication of application: **19.05.2021**
(21) Application number: 20205336.9
(22) Date of filing: 03.11.2020
(51) Int. Cl.: A61K 31/138, A61K 31/575, A61P 25/00, A61P 25/22, A61P 25/24, A61K 45/06

(54) **LXR AGONISTS FOR TREATING PSYCHIATRIC STRESS DISORDERS**

(30) Priority: 07.11.2019 US 201916676495
(71) Applicant: Yeda Research and Development Co. Ltd, 7610002 Rehovot (IL)
(72) Inventor: FAINZILBER, Michael, 7642414 Rehovot (IL); PANAYOTIS, Nicolas, 7610002 Rehovot (IL)
(74) Representative: Dennemeyer & Associates S.A.

(57) **Abstract**

Agents and methods of treating a psychiatric stress disorder, Rett syndrome, MeCP2 duplication syndrome or multiple sclerosis are provided. Accordingly there is provided an agent capable of down-regulating activity and/or expression of importin alpha5 for use in the treatment of a medical condition selected from the group consisting of psychiatric stress disorder, Rett syndrome, MeCP2 duplication syndrome and multiple sclerosis. Also provided are methods of identifying an agent that inhibits activity of importin alpha5. A combination of an agent capable of up-regulating activity and/or expression of liver X receptor (LXR) and a selective serotonin reuptake inhibitor (SSRI), for use in treating a psychiatric stress disorder in a subject in need thereof.

## Description

### SEQUENCE LISTING STATEMENT

The ASCII file, entitled 84750 SequenceListing.txt, created on 19 October 2020, comprising 2,894 bytes, submitted concurrently with the filing of this application is incorporated herein by reference.

### FIELD AND BACKGROUND OF THE INVENTION

The present invention, in some embodiments thereof, relates to methods of treating psychiatric stress disorders.

Anxiety and stress-related conditions represent a significant health burden in modern society, representing the most common psychiatric disorders, affecting between 10 to 30 percent of the general population. Anxiety disorders are currently treated with a variety of agents targeting synaptic mechanisms, such as Benzodiazepines and Selective Serotonin Uptake Inhibitors (SSRIs). These agents either directly affect neurotransmitter receptor systems or modulate neurotransmitter levels or availability, but their long-term use is limited by problematic side effects and suboptimal efficacy.

Importins are a group of proteins that transports protein molecules into the nucleus by binding to specific recognition sequences, called nuclear localization sequences (NLS). Importins are expressed in all neuronal compartments, including axons, dendrites and synapses; and their dependent transport mechanisms link synapse to nucleus in a diversity of physiological contexts. Importin has two subunits, importin α and importin β, wherein members of the importin-β subfamily can bind NLS cargo proteins and transport them by themselves, or can form heterodimers with importin-a. There are 6-7 importin α family members in any given mammal and individual cell types express different subsets of this ensemble⁷, often in a tightly regulated manner^{8,9}. Injury in peripheral neurons¹⁰ or activity in central neurons¹¹ can activate importin-dependent transport mechanisms in axons or dendrites to link both pre- and postsynaptic compartments to soma and nucleus^{4,12}. Assigning specific roles for individual importin α's in brain functions is challenging due to functional redundancies in cargo binding^{13,14} and compensatory expression regulation of different family members¹⁵.

Additional background art includes:
Moreau, T. et al. European neurology 77, 47-55 (2017);
Stearns, N. A. et al. Neuroscience 146, 907-921 (2007);
Samaco, R. C. et al. Human Molecular Genetics 17, 1718-1727 (2008);
Goffin, D. et al. Nature Neuroscience 15, 274-283 (2011);
Na, E. S. et al. Journal of Neuroscience 32, 3109-3117 (2012);
Sztainberg, et al. Nature; 528(7580): 123-126 (2015);
US Patent Nos: US9492544; US 8604011;
International Patent Application Publication Nos: WO2016141145, WO2014210389 and WO2013170147;
US Patent Application Publication No: US 20050003998, US 20140309291 and US20150025052; and
Chinese Patent No: CN100441191.

### SUMMARY OF THE INVENTION

According to an aspect of some embodiments of the present invention there is provided an agent capable of down-regulating activity and/or expression of importin α5, for use in the treatment of a medical condition selected from the group consisting of psychiatric stress disorder, Rett syndrome, MeCP2 duplication syndrome and multiple sclerosis, with the proviso that the psychiatric stress disorder is not post-traumatic stress disorder mediated by inflammation.

According to an aspect of some embodiments of the present invention there is provided a method of treating a medical condition selected from the group consisting of psychiatric stress disorder, Rett syndrome, MeCP2 duplication syndrome and multiple sclerosis in a subject in need thereof, the method comprising administering to the subject a therapeutically effective amount of an agent capable of down-regulating activity and/or expression of importin α5, with the proviso that the psychiatric stress disorder is not post-traumatic stress disorder mediated by inflammation, thereby treating the medical condition in the subject.

According to some embodiments of the invention, the medical condition is selected from the group consisting of psychiatric stress disorder, Rett syndrome and MeCP2 duplication syndrome.

According to an aspect of some embodiments of the present invention there is provided a composition comprising as the active agent alvespimycin or a structural analog thereof for use in the treatment of a psychiatric stress disorder.

According to an aspect of some embodiments of the present invention there is provided a composition comprising as the active agent alvespimycin or a structural analog thereof for use in the treatment of a psychiatric stress disorder, wherein the psychiatric stress disorder is not substance induced anxiety and/or in co-morbidity with Alzheimer's disease.

According to an aspect of some embodiments of the present invention there is provided a composition comprising as the active agent alvespimycin or a structural analog thereof for use in the treatment of a psychiatric stress disorder selected from the group consisting of acute stress disorder, post-traumatic stress disorder (PTSD), phobic anxiety disorder, separation anxiety disorder, obsessive-compulsive anxiety disorder, adjustment disorder with anxiety and selective mutism.

According to an aspect of some embodiments of the present invention there is provided a method of treating a psychiatric stress disorder in a subject in need thereof, the method comprising administering to the subject a therapeutically effective amount of a composition comprising as the active agent alvespimycin or a structural analog thereof, thereby treating the psychiatric stress disorder in the subject.

According to an aspect of some embodiments of the present invention there is provided a method of treating a psychiatric stress disorder in a subject in need thereof, wherein said psychiatric stress disorder is not substance induced anxiety and/or in co-morbidity with Alzheimer's disease, the method comprising administering to the subject a therapeutically effective amount of a composition comprising as the active agent alvespimycin or a structural analog thereof, thereby treating the psychiatric stress disorder in the subject.

According to an aspect of some embodiments of the present invention there is provided a method of treating a psychiatric stress disorder selected from the group consisting of acute stress disorder, post-traumatic stress disorder (PTSD), phobic anxiety disorder, separation anxiety disorder, obsessive-compulsive anxiety disorder, adjustment disorder with anxiety and selective mutism in a subject in need thereof, the method comprising administering to the subject a therapeutically effective amount of a composition comprising as the active agent alvespimycin or a structural analog thereof, thereby treating the psychiatric stress disorder in the subject.

According to some embodiments of the invention, the composition consists of alvespimycin or a structural analog thereof as the active agent.

According to an aspect of some embodiments of the present invention there is provided a combination of at least two agents selected from the group consisting of:
(i) an agent capable of down-regulating activity and/or expression of importin α5;
(ii) an agent capable of down-regulating activity and/or expression of MeCP2;
(iii) an agent capable of up-regulating activity and/or expression of liver X receptor (LXR);
(iv) an agent capable of up-regulating activity and/or expression of SIP receptor; and
(v) alvespimycin or a structural analog thereof;
for the treatment of a medical condition that can benefit from up-regulating activity of S1P receptor.

According to an aspect of some embodiments of the present invention there is provided a method of treating a medical condition that can benefit from up-regulating activity of SIP receptor in a subject in need thereof, the method comprising administering to the subject a therapeutically effective amount of at least two agents selected from the group consisting of:
(i) an agent capable of down-regulating activity and/or expression of importin α5;
(ii) an agent capable of down-regulating activity and/or expression of MeCP2;
(iii) an agent capable of up-regulating activity and/or expression of liver X receptor (LXR);
(iv) an agent capable of up-regulating activity and/or expression of SIP receptor; and
(v) alvespimycin or a structural analog thereof;
thereby treating the medical condition in the subject.

According to some embodiments of the invention, each of the at least two agents is packaged in a separate container.

According to some embodiments of the invention, the at least two agents are packaged in a co-formulation.

According to some embodiments of the invention, the medical condition is selected from the group consisting of psychiatric stress disorder, multiple sclerosis, Rett syndrome and MeCP2 duplication syndrome.

According to some embodiments of the invention, the medical condition is not multiple sclerosis.

According to some embodiments of the invention, the at least two agents comprise agent (i), agent (iii) and/or agent (iv) and the medical condition is not multiple sclerosis.

According to some embodiments of the invention, the medical condition is not MeCP2 duplication syndrome or in co-morbidity with MeCP2 duplication syndrome.

According to some embodiments of the invention, the at least two agents comprise agent (ii) and the medical condition is not MeCP2 duplication syndrome or in co-morbidity with MeCP2 duplication syndrome.

According to some embodiments of the invention, the psychiatric stress disorder is not substance induced anxiety and/or in co-morbidity with Alzheimer's disease.

According to some embodiments of the invention, the at least two agents comprise agent (v) and the psychiatric stress disorder is not substance induced anxiety and/or in co-morbidity with Alzheimer's disease.

According to some embodiments of the invention, the psychiatric stress disorder is selected from the group consisting of acute stress disorder, post-traumatic stress disorder (PTSD), phobic anxiety disorder, separation anxiety disorder, obsessive-compulsive anxiety disorder, adjustment disorder with anxiety and selective mutism.

According to some embodiments of the invention, the at least two agent comprise agent (v) and the psychiatric stress disorder is selected from the group consisting of acute stress disorder, post-traumatic stress disorder (PTSD), phobic anxiety disorder, separation anxiety disorder, obsessive-compulsive anxiety disorder, adjustment disorder with anxiety and selective mutism.

According to some embodiments of the invention, the at least two agents comprise agent (iii) and agent (iv).

According to an aspect of some embodiments of the present invention there is provided an agent capable of down-regulating activity of MeCP2, for use in the treatment of a medical condition selected from the group consisting of psychiatric stress disorder and multiple sclerosis.

According to an aspect of some embodiments of the present invention there is provided a method of treating a medical condition selected from the group consisting of psychiatric stress disorder and multiple sclerosis in a subject in need thereof, the method comprising administering to the subject a therapeutically effective amount of an agent capable of down-regulating activity of MeCP2, thereby treating the medical condition in the subject.

According to some embodiments of the invention, the medical condition is not in co-morbidity with MeCP2 duplication syndrome.

According to some embodiments of the invention, the MeCP2 activity is translocation into the nucleus.

According to some embodiments of the invention, the MeCP2 activity is binding to importin α5.

According to some embodiments of the invention, the MeCP2 activity is binding to the promoter of sphingosine kinase 1 (Sphkl).

According to some embodiments of the invention, the LXR activity is translocation into the nucleus.

According to some embodiments of the invention, the LXR activity is binding to the promoter of sphingosine kinase 1 (Sphkl).

According to an aspect of some embodiments of the present invention there is provided an agent capable of up-regulating expression of liver X receptor (LXR), for use in the treatment of a medical condition selected from the group consisting of a psychiatric stress disorder and multiple sclerosis.

According to an aspect of some embodiments of the present invention there is provided a method of treating a medical condition selected from the group consisting of psychiatric stress disorder and multiple sclerosis in a subject in need thereof, the method comprising administering to the subject a therapeutically effective amount of an agent capable of up-regulating expression of liver X receptor (LXR), thereby treating the medical condition in the subject.

According to an aspect of some embodiments of the present invention there is provided a method of treating a psychiatric stress disorder in a subject in need thereof, the method comprising administering to the subject a therapeutically effective amount of an agent capable of up-regulating activity and/or expression of liver X receptor (LXR) and a selective serotonin reuptake inhibitor (SSRI), thereby treating the psychiatric stress disorder in the subject.

According to some embodiments of the invention, the SSRI is fluoxetine (Prozac®).

According to some embodiments of the invention, the agent capable of up-regulating activity and/or expression of LXR and/or the SSRI is administered in less than the FDA recommended does for treatment as a monotherapy.

According to some embodiments of the invention, the SSRI is administered in a dose of 0.5-5 mg/kg.

According to some embodiments of the invention, the agent capable of up-regulating activity and/or expression of LXR is administered in a dose of 2-20 mg/kg.

According to some embodiments of the invention, the agent is selected from the group consisting of a small molecule, a peptide and an antibody.

According to some embodiments of the invention, the agent capable of up-regulating activity and/or expression of LXR is formulated for penetrating the blood brain barrier (BBB).

According to some embodiments of the invention, the agent capable of up-regulating activity of LXR is selected from the group consisting of β-sitosterol, campesterol, stigmasterol, fucosterol and brassicasterol, cholic acid (CA) and 24,25 EC.

According to some embodiments of the invention, the agent capable of up-regulating activity of LXR is β-sitosterol.

According to some embodiments of the invention, the S1P receptor comprises S1P receptor 5 (S1PR5).

According to some embodiments of the invention, the agent capable of up-regulating activity of SIP receptor is selected from the group consisting of fingolimod, siponimod and A 971432.

According to some embodiments of the invention, the agent capable of up-regulating activity of SIP receptor is selected from the group consisting of fingolimod, siponimod, TC-G 1006 and A 971432.

According to some embodiments of the invention, the agent capable of up-regulating activity of SIP receptor is selected from the group consisting of fingolimod, TC-G 1006 and A 971432.

According to some embodiments of the invention, the agent capable of up-regulating activity of SIP receptor is fingolimod and/or siponimod.

According to some embodiments of the invention, the agent capable of up-regulating activity of SIP receptor is fingolimod.

According to some embodiments of the invention, the structural analog is selected from the group consisting of geldanamycin and tanespimycin (17-AAG).

According to some embodiments of the invention, the psychiatric stress disorder is anxiety.

According to an aspect of some embodiments of the present invention there is provided a method of identifying an agent that inhibits activity of importin α5, the method comprising:
(i) contacting a polypeptide sequence containing an NLS sequence of MeCP2 with a importin α5 polypeptide under conditions which allow binding of the importin α5 polypeptide to the polypeptide containing the NLS sequence;
(ii) determining an effect of a test agent on binding of the importin α5 polypeptide to the polypeptide containing the NLS sequence;
wherein a decrease in the binding as compared to same in the absence of the test agent indicates the test agent inhibits activity of importin α5.

According to some embodiments of the invention, the NLS sequence of MeCP2 comprises an amino acid sequence as set forth in SEQ ID NO: 2.

According to some embodiments of the invention, the method further comprising providing the test agent and testing an anxiolytic activity of same.

According to some embodiments of the invention, the determining is effected *in-vitro* or *ex-vivo.*

According to some embodiments of the invention, the method further comprising treating a medical condition that can benefit from up-regulating activity of SIP receptor with the test agent wherein the determining indicates the test agent inhibits activity of importin α5.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

The patent or application file contains at least one drawing executed in color. Copies of this patent or patent application publication with color drawing(s) will be provided by the Office upon request and payment of the necessary fee.

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

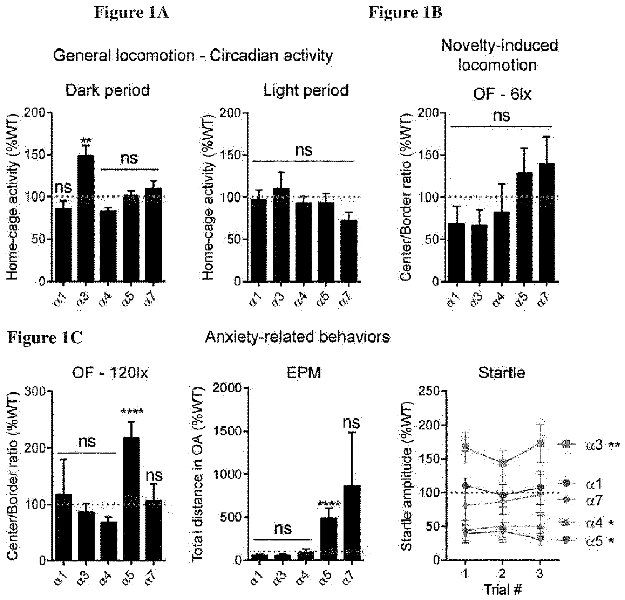

Figures 1A-C demonstrate behavioral phenotyping of different importin α knockout (KO) mice as compared to their wild type (WT) littermates. The tests used cover general locomotion and circadian activity (Figure 1A), novelty-induced locomotion (Figure 1B) and anxiety-related behaviors (Figure 1C). Figure 1A is a graph demonstrating basal home-cage activity of the mice asses by a home cage locomotion test. The InfraMot system was used to monitor the activity of the mice over 72 consecutive hours. Most of importin α-KO mice do not exhibit significant modification of their activity during the dark period (α1, α4, α5, α7; p > 0.05) and the light period (α1, α4, α5, α7; p > 0.05). Importin α3 knockouts showed an elevated activity compared to WT during the dark phase (148 % ± 11.9; p < 0.05) but not the light phase (93.11% ± 7.4; p > 0.05). Figure 1B is a graph demonstrating novelty-induced locomotion assessed in an open-field (OF) test performed under dim light (OF-6 lux). No significant differences were observed for all importin α mutants compared to their WT littermates. Figure 1C show graphs demonstrating anxiety measures assessed by the OF test (OF-120 lux), the Elevated plus maze (EPM) test and the Acoustic startle reflex (ASR) test. The anxiety measures from the OF test (OF-120 lux) show increased center/border ratio for importin α5 null mice. In the EPM, importin α5 mice spent more time in the open (i.e. unsecured/anxiogenic) arms. Importin α5 null mice displayed a delayed and decreased baseline acoustic startle, in accordance with a reduction in anxiety-related behaviors in this line. Importin α3 results indicate an increased startle and Importin α4 nulls present a deficit in the startle response. 8-15 animals were used for each line in different assays in this data-set. *p < 0.05; ** p < 0.01, *** p <0.001, **** p <0.0001 (two-tailed t-test), bars represent average ± S.E.M.

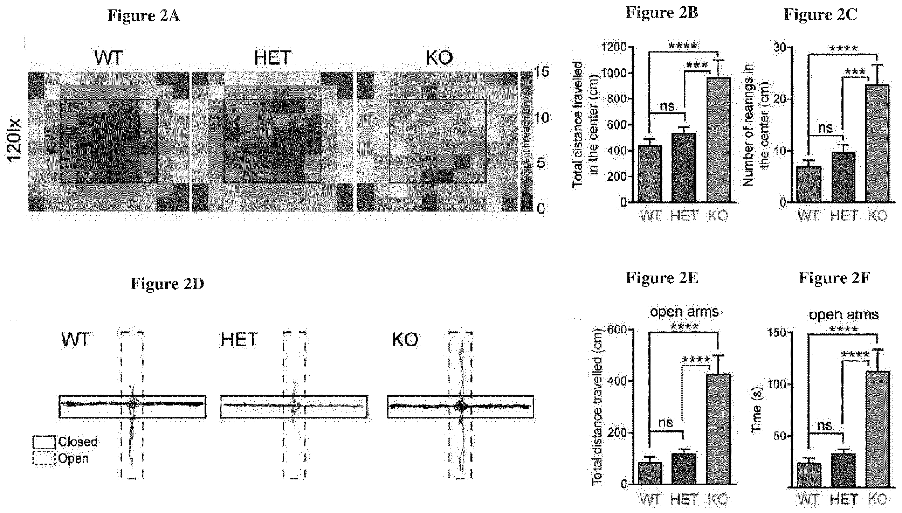

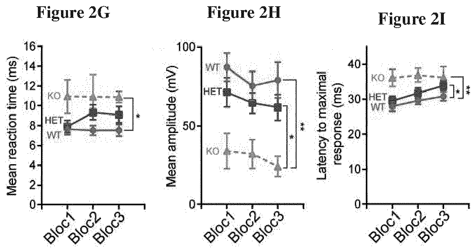

Figures 2A-I demonstrate reduced anxiety in Importin α5 KO mice as compared to their WT and heterologous (HET) littermates. Figures 2A-C demonstrate mice activity in the OF test. Shown are group heat-map representation (Figure 2A) and the respective graphs demonstrating the distance travelled (Figure 2B) and rearing activity in the OF center (Figure 2C) over 10 minutes in the OF. Importin α5 KO mice exhibit significant increase in the time spent, the distance travelled and in rearing activity in the OF center. Figures 2D-F demonstrate mice activity in the EPM test. Importin α5 KO mice show increased exploration of the open arms of an EPM as shown by representative track traces (Figure 2D), a significantly higher distance coverage (Figure 2E) and time spent (Figure 2F) in the open arms. Figures 2G-I demonstrate the results of an ASR test monitoring the motor reflex to a 120 dB auditory stimulation. Importin α5 KO mice show a significant increase in reaction time to the auditory stimulus (Figure 2G), a significant decrease in response amplitude (Figure 2H) and a delay of the peak response (Figure 2I). Bars represent average ± S.E.M., n ≥ 9 animals for each genotype per test, * p < 0.05, ** p < 0.01, *** p <0.001, **** p <0.0001 (one-way ANOVA followed by Tukey's HSD post hoc correction for multiple comparisons).

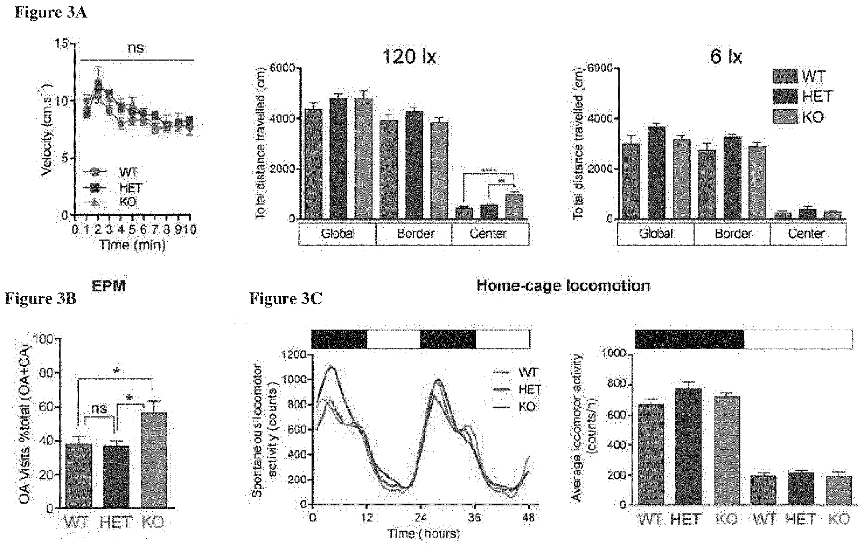

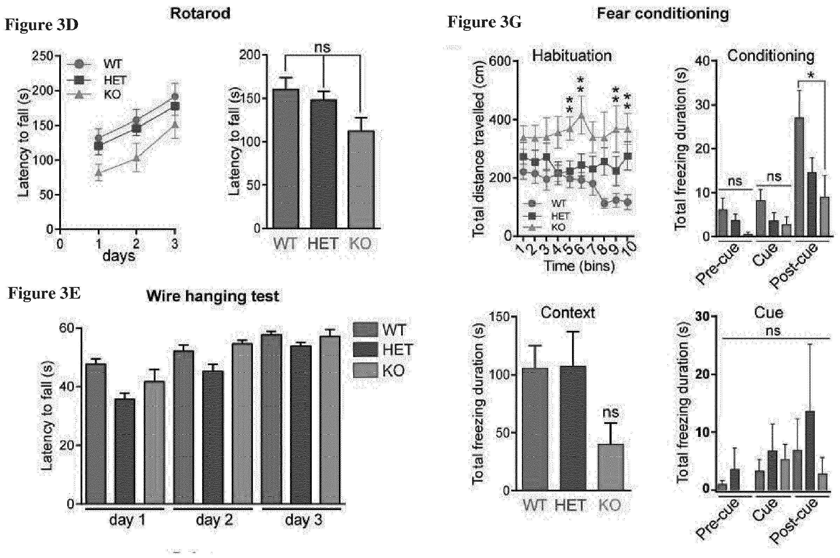

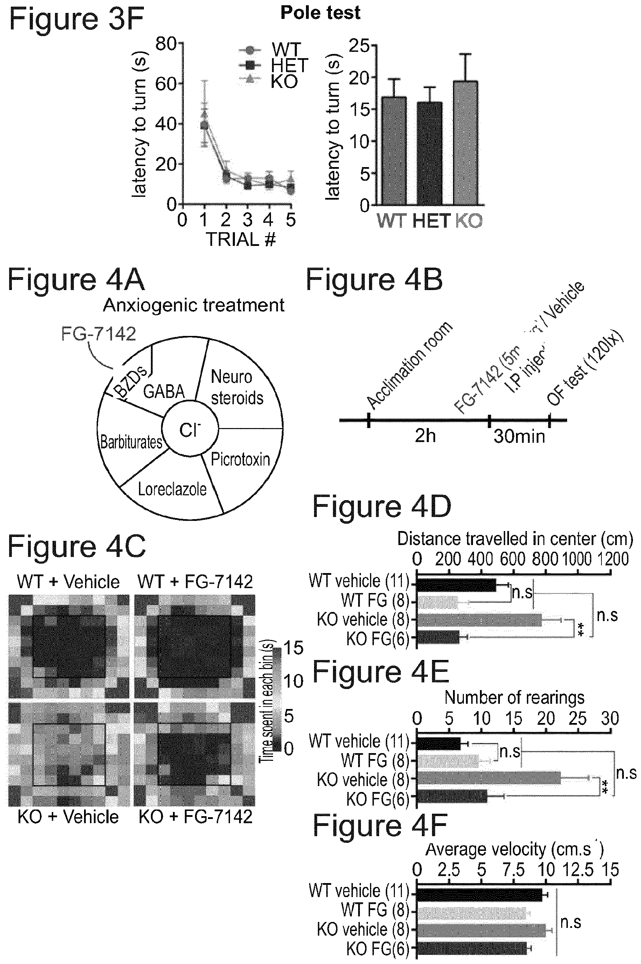

Figures 3A-G demonstrate preserved sensory and motor performance in importin α5 KO mice as compared to their WT and HET littermates. Figure 3A demonstrates mice activity in the OF test as assessed by velocity and total distance travelled. Importin α5 KO movement speed (green) was not significantly different over the 10 minutes of the OF session compared to WT (red) and HET (blue) littermates. Reducing OF illumination from 120 to 6 lux abolished the differences between WT and importin α5 KO animals in the distance travelled in the OF center area. Figure 3B demonstrates mice activity in the EPM test. Importin α5 KO mice show increased exploration of the EPM open arms, expressed as a percentage of open arm visits from the total number of visits to all arms. Figure 3C demonstrates mice activity in the home-cage locomotion test. The graphs show spontaneous and average locomotor circadian activity over 48 hours. The home-cage activity of importin α5 KO was not different from WT and HET during the dark and light phases (black and white boxes above the graphs, respectively). Figures 3D-F demonstrate the absence of locomotion and coordination/balance defects in importin α5 KO animals, as assessed by accelerated rotarod (Figure 3D) wire-hanging (Figure 3E) and pole (Figure 3F) tests. Figure 3G demonstrates mice activity in the fear conditioning test as assessed by total distance travelled and total freezing duration in the different phases of the test. Strikingly, importin α5 KO animals moved significantly more than their WT (red) and HET (blue) littermates during the habituation phase, and consequently froze less during the conditioning (P<0.05 during the post-cue phase). The total freezing time (totaling all pause periods > 3 sec each) did not differ significantly for context and the cue tests. Bars represent average ± S.E.M., n ≥ 6 animals for each genotype per test, * p < 0.05, ** p < 0.01, *** p <0.001, **** p <0.0001 (one-way ANOVA followed by Tukey's HSD post hoc correction for multiple comparisons (Figures 3A-F) and two-way repeated measure ANOVA followed by Sidak's multiple comparisons test (Figure 3G, habituation phase).
Figures 4A-F demonstrate the effects of the anxiogenic drug FG-7142 on WT and importin α5 KO mice. Figure 4A is a schematic representation of the GABA-A receptor showing the benzodiazepine binding site where FG-7142 acts as an inverse partial agonist. Figure 4B shows the treatment protocol: The pro-anxiogenic drug FG-7142 (5 mg / kg) or vehicle was injected intraperitoneally and its effects were assessed in OF after 30min. Figure 4C shows activity heat-map representations of the OF test, demonstrating that the anxiolytic phenotype of importin α5 KO mice is reversed by FG-7142 treatment. Figure 4D-F are graphs demonstrating that FG-7142 treatment significantly decreases the distance travelled in the center of the OF (Figure 4D) and the number of rearing events in the center (Figure 4E), but does not perturb average movement velocity (Figure 4F). Bars represent average ± S.E.M., the number of animals per group is indicated in parentheses; * p <0.05; ** p <0.01, *** p <0.001 (one-way ANOVA followed by Tukey's HSD post hoc correction for multiple comparisons (Figure 4D-F).

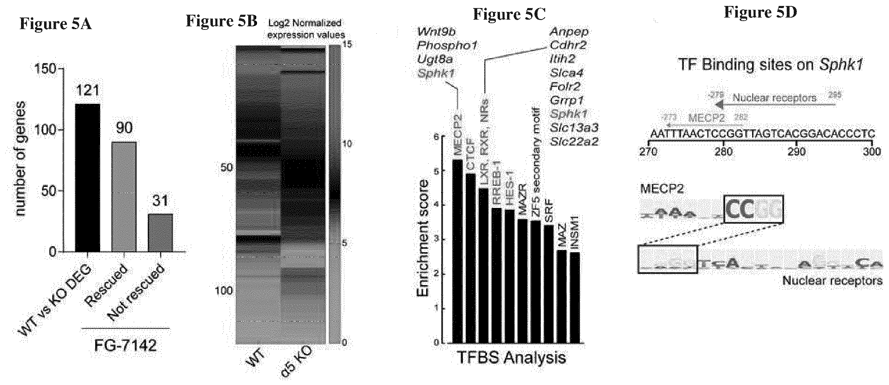

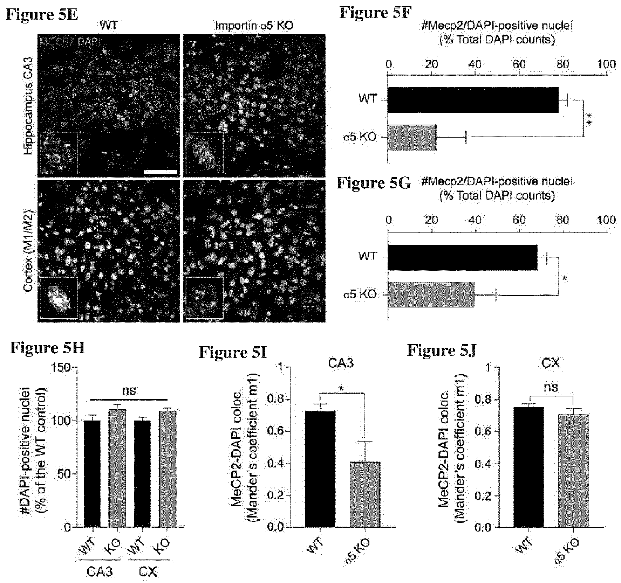

Figures 5A-J demonstrate the effects of the importin α5 KO on transcription and nuclear localization. Figure 5A is a bar graph illustrating the number of deregulated genes (fold-change, -1.5≤ FC ≥1.5) in importin α5 KO hippocampus and the ratio of genes for which the levels of expression were rescued or not under an anxiogenic treatment (5mg/kg FG7142). Figure 5B is a heat map showing log2-normalized expression values for 121 genes with significantly changed expression in importin α5 KO hippocampus (n = 3 mice per group). Figure 5C is a bar graph illustrating the top 10 transcription factor candidates identified by FMatch (geneXplain) search for factors regulating expression of genes differentially expressed in importin α5 KO. Genes regulated by the indicated factors are shown in insets. Figure 5D shows the overlapping binding sequences for MECP2 and nuclear receptors on the Sphkl promoter. Figure 5E shows representative photomicrographs of hippocampus CA3 and motor cortex immunostained with MECP2 (red) and DAPI nuclear staining (blue), demonstrating a clear punctate heterochromatic pattern in neuronal nuclei in hippocampus CA3 and motor cortex of WT mice, with reduced colocalization in importin α5 KO. Scale bar 50 µm. Figure 5F-G are bar graphs showing quantification of the number of nuclei co-labelled with MECP2 and DAPI in the hippocampus (HPC, CA3 field, Figure 5F) and the cortex (CX, M1/M2 area, Figure 5G). The data show a significant reduction of co-labeled nuclei in both the hippocampus (p <0.01) and the cortex (p <0.05) of importin α5 KO mice (n ≥ 4 for each genotype). Figure 5H is a bar graph showing total counts of DAPI-positive nuclei in the CA3 region of the hippocampus (HPC) and the M1/M2 cortical area (CX) as the % of the WT values (n ≥ 3 for each genotype). Figures 5I-J are bar graphs showing colocalization analyses of MECP2 with DAPI in hippocampal (Figure 5I) and cortical (Figure 5J) neurons of WT versus importin α5 KO (n ≥ 3 for each genotype). * p <0.05; ** p <0.01 [two-tailed t-test (Figures 5F, 5G, 5I, 5J) and Kruskal-Wallis test followed by Dunn's multiple comparisons test (Figure 5H)], bars represent average ± S.E.M..

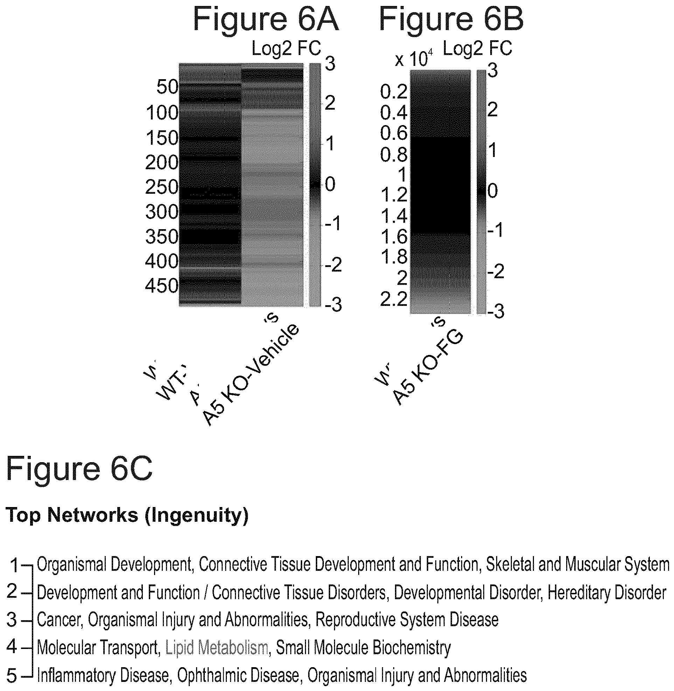

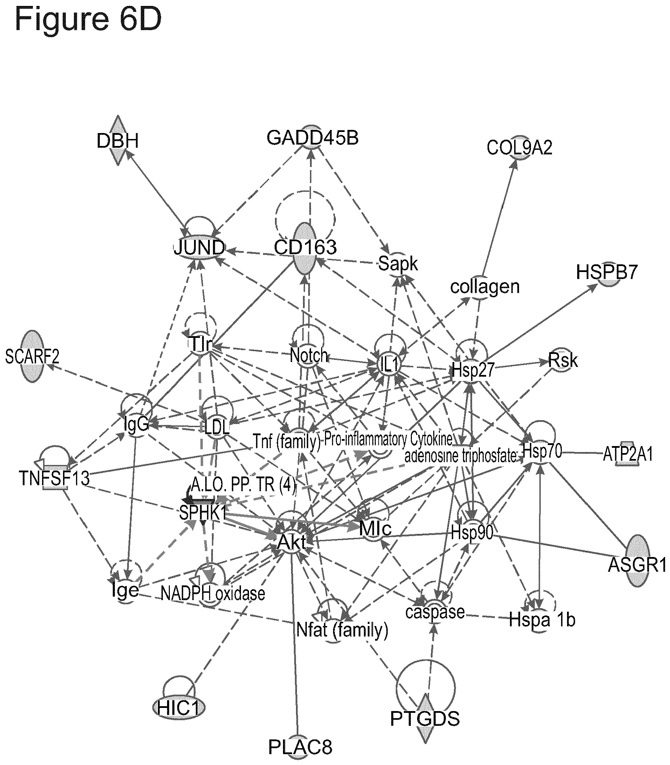

Figures 6A-D demonstrate the effects of the importin α5 KO on transcription. Figure 6A is a heat map showing log2-normalized fold-change of gene expression in WT+ FG7142 (marked as FG) vs WT-vehicle, importin α5 KO (marked as A5 KO) + FG7142 vs importin α5 KO-vehicle. Figure 6B is a heat map showing log2-normalized fold-change of gene expression in WT- FG7142 vs. with importin α5 KO-FG7142, demonstrating a larger cohort of almost 600 transcripts that were differentially regulated by FG7142 treatment in KO versus WT mice (n = 3 mice per group). Figure 6C demonstrates the identification of signaling networks using the Ingenuity Pathway Analysis program and the candidate genes from the top ranked pathway. Figure 6D demonstrates the lipid metabolism network identified as one of the top five signaling networks by Ingenuity and containing the Sphkl gene (red triangle) up-regulated in importin α5 KO hippocampi.

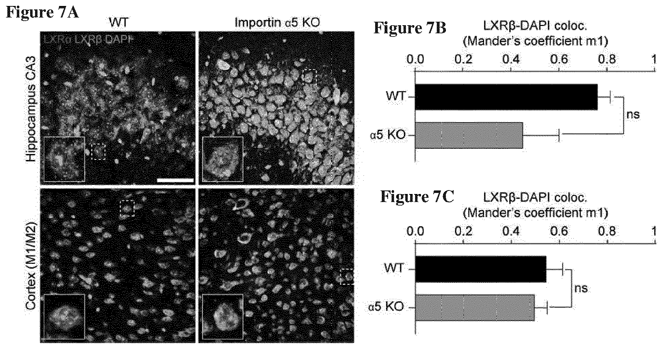

Figures 7A-C demonstrate no significant effect of importin α5 KO on LXR localization. Figure 7A shows representative photomicrographs of hippocampus CA3 and motor cortex immunostained with LXRα (red), LXRβ (green) and DAPI nuclear staining (blue). Scale bar 50 µm. Figures 7B-C are bar graphs showing quantification od the number of nuclei co-labelled with LXRβ and DAPI in the hippocampus (HPC, CA3 field, Figure 7B) and the cortex (CX, M1/M2 area, Figure 7C). The data indicate no significant differences between the colocalization of LXRβ with DAPI in hippocampal and cortical neurons in WT compared to importin α5 KO neurons, as determined by m1 Mander's colocalization coefficient per genotype and per brain areas. Bars represent average ± S.E.M., n ≥ 3 [Mann-Whitney test (Figures 7B-C)].

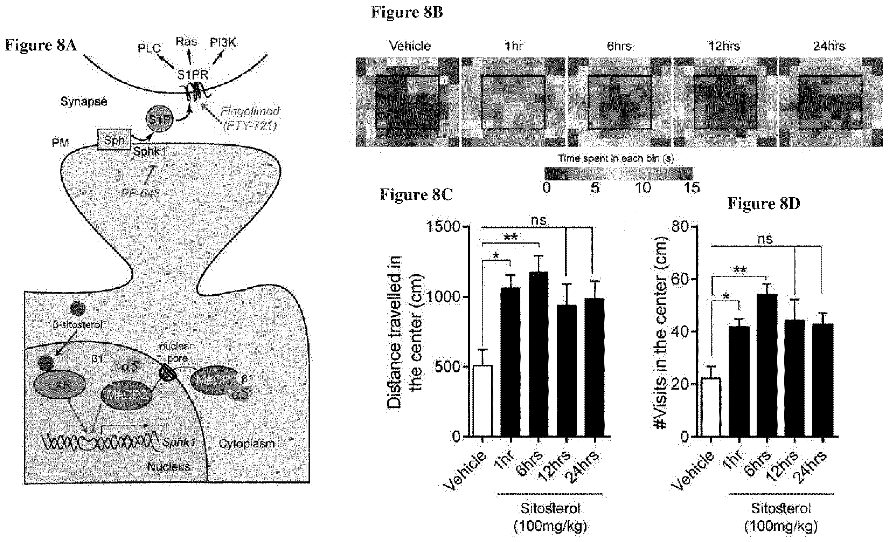

Figures 8A-D demonstrate a newly discovered anxiolysis mechanism based on importins, transcription factors and lipid signaling. Figure 8A is a schematic model of the proposed mechanism, indicating potential pharmacological challenges and their predicted outcomes. Figures 8B-D show heat map representations and bar graphs demonstrating the effects of the LXR agonist β-sitosterol (100 mg/kg) or vehicle, as assessed in the OF at the indicated time points following IP injection. β-sitosterol exerts anxiolytic properties by increasing the distance travelled (Figure 8C) and the number of visits (Figure 8D) in the OF center at 1 and 6 hours following injection. Bars represent average ± S.E.M., n ≥ 5, * p <0.05; ** p <0.01, *** p <0.001 [one-way ANOVA followed by Tukey's HSD post hoc correction for multiple comparisons (Figure 8C-D)].

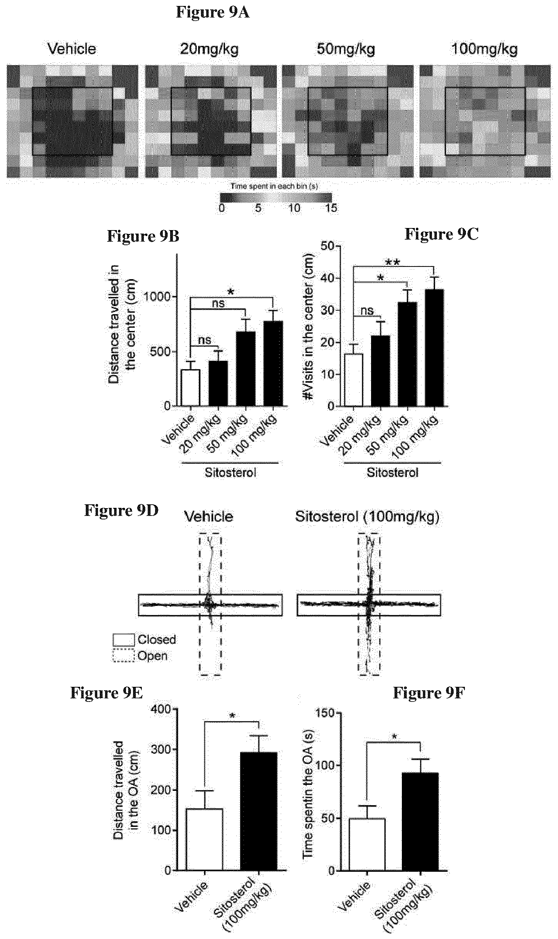

Figures 9A-F demonstrate the anxiolytic effect of the LXR agonist β-sitosterol. β-sitosterol or vehicle was injected intraperitoneally at the indicated doses. Figures 9A-C demonstrate mice activity in the OF test 1 hour following injection. Shown are heat map representations (Figure 9A) and bar graphs (Figures 9B-C), demonstrating a dose response effect of β-sitosterol, reaching significance for the highest dose tested. Figures 9D-F demonstrate mice activity in the EPM test 1 hour following injection at a dose of 100 mg/ kg. Shown are representative track traces (Figure 9D) and bar graphs of the distance coverage (Figure 9E) and time spent (Figure 9F) in the open arms. The results show a significant increase in the distance and the time spent in the open arms of the EPM in β-sitosterol-treated mice compared to their vehicle-treated littermates. Bars represent average ± S.E.M., n ≥ 5, * p <0.05, ** p <0.01 [one-way ANOVA followed by Tukey's HSD post hoc correction for multiple comparisons (Figure 9C-D) and two-tailed t-test (Figures 9E-F)].

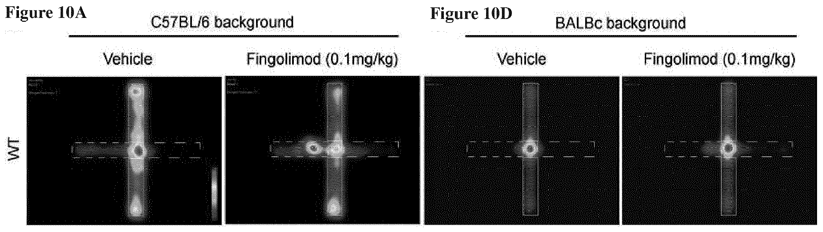

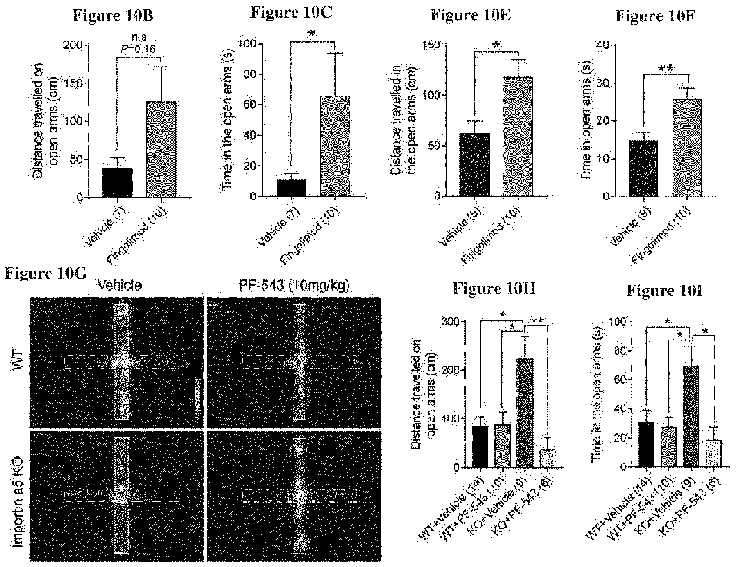

Figures 10A-I demonstrate the anxiolytic effect of fingolimod and the anxiogenic effect of the Sphkl inhibitor PF-543, as assessed by the EPM test. Figures 10A-F show density maps (Figures 10A and 10D) and bar graphs (Figures 10B-C and 10E-D) of mouse exploratory activity in the EPM test showing the effects of fingolimod on wild type C57BL6 mice (Figures 10A-C) and Balb/c mice (Figure 10E-F). Strikingly, Fingolimod has clear and robust anxiolytic effects on both C57BL6 and Balb/c mice, increasing the time spent in the open arms of the EPM. Figures 10G-I show density maps (Figure 10G) and bar graphs (Figures 10H-I) of mouse exploratory activity in the EPM showing the potential of the Sphkl inhibitor PF-543 (10 mg / kg) to reverse the anxiolytic response of importin α5 KOs towards WT values. Bars represent average ± S.E.M., number of animals per group indicated in parentheses, * p <0.05, ** p <0.01 [two-tailed t-test (Figure 10B, 10C, 10E, 10F) and one-way ANOVA followed by Tukey's HSD post hoc correction for multiple comparisons (Figures 10H, 10I)].

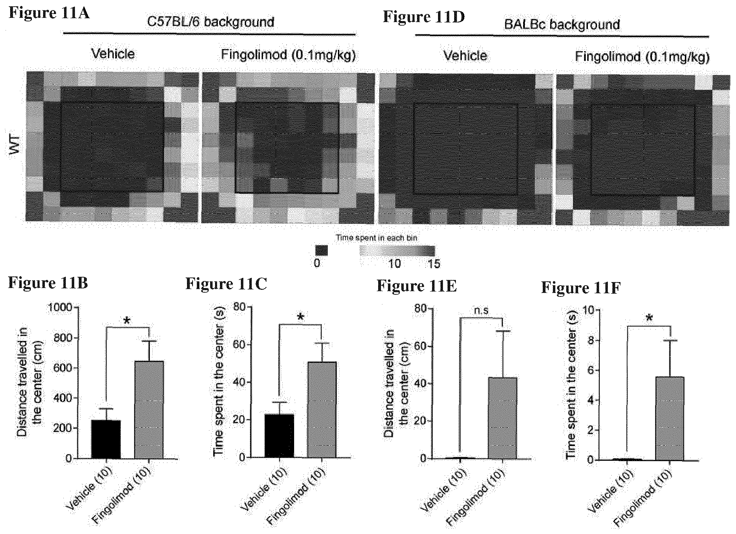

Figures 11A-F demonstrate the anxiolytic effect of fingolimod, as assessed by the OF test. Shown are hit-map representations (Figures 11A and 11D) and the respective bar graphs demonstrating the distance travelled (Figures 11B and 11E) and rearing activity in the OF center (Figures 11C and 11F) showing the effects of fingolimod on wild type C57BL6 mice (Figures 11A-C) and Balb/c mice (Figure 11E-F). Fingolimod has clear and robust anxiolytic effects on both C57BL6 and Balb/c mice, increasing the distance travelled and the time spent in the center of the OF arena Bars represent average ± S.E.M., number of animals per group indicated in parentheses, * p <0.05 [two-tailed t-test (Figures 11B-C and 11E-F)].

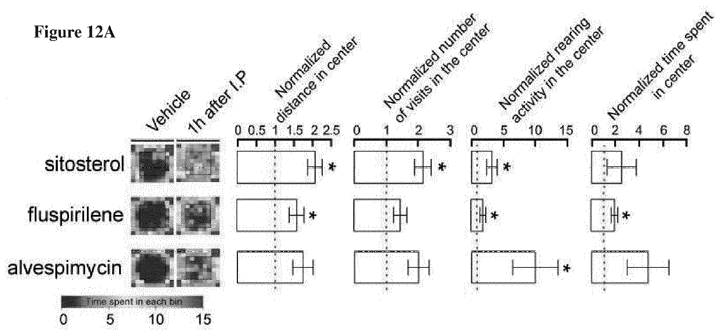

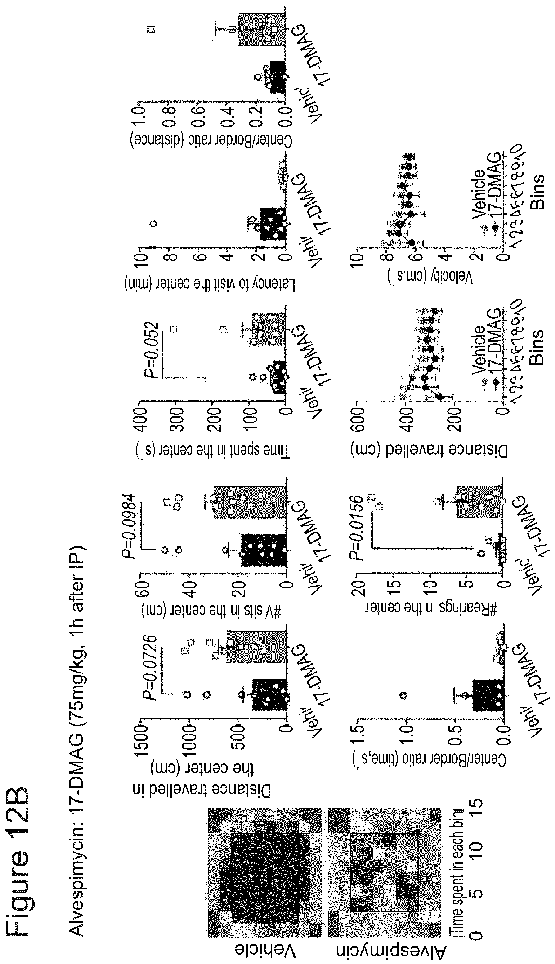

Figures 12A-B demonstrate the effect the anxiolytic effect of β-sitosterol, fluspirilene and alvespimycin. Figure 12A demonstrate the anxiolytic properties of the three drugs using the OF test 1 hour following i.p. injection, as compared to vehicle. Data is shown for Sitosterol (100 mg/kg), n=5, vehicle, n=8; Alvespimycin (17-DMAG; 75 mg/kg), n=10, vehicle, n=10; Fluspirilene (10 mg/kg) n=5, vehicle, n=5. Figure 12B shows data obtained from an independent cohort of animals treated with Alvespimycin (17-DMAG; n=10) or its vehicle (n=10) and tested in the OF test conducted to further evaluate alvespimycin anxiolytic properties. Shown are heat map representations and the respective graphs, demonstrating the potential of the drug to increase the number of rearings in the center area of the OF without adverse effects on their motor performances. Data is represented as average ± S.E.M. *P<0.05; **P<0.01, ***P<0.001.

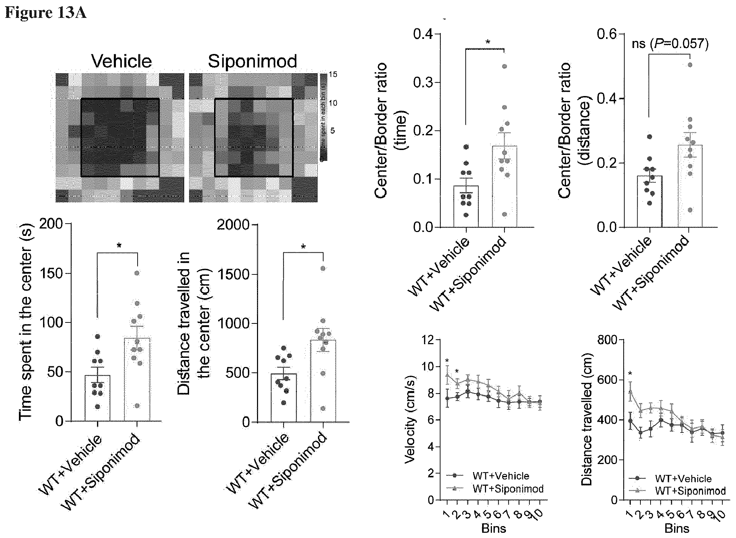

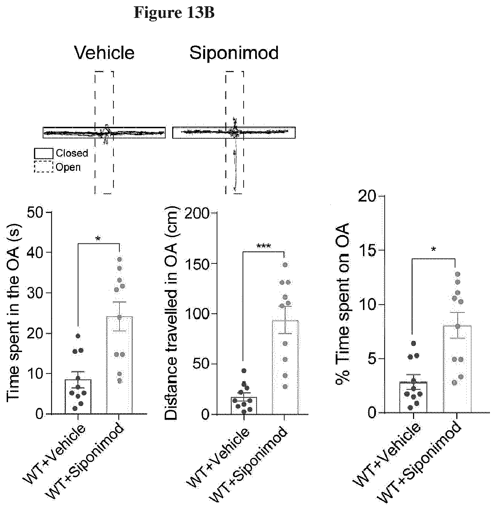

Figures 13A-B demonstrate the anxiolytic effect of siponimod. 0.1 mg / kg Siponimod or vehicle (saline control) was injected intraperitoneally to wild-type C57BL6 mice every 3 days during 4 weeks. Figure 13A demonstrates mice activity during the 10 minutes OF test. Shown are heat map representations and bar graphs, demonstrating a significant increase in the time spent, the distance travelled and a higher center/border ratio (for the exploration time) in the center of the OF. The lower panel demonstrates that the average velocity and distance was elevated during the first 1-2 minutes of exploration in the siponimod-treated group. Figure 13B demonstrates mice activity in the EPM test. Shown are representative track traces and bar graphs of the distance coverage and time spent in the open arms. The results show a significant increase in the distance and the time spent in the open arms of the EPM in siponimod-treated mice compared to their vehicle-treated littermates. Bars represent average ± S.E.M., n=9-10 mice in each groups for each test, * p <0.05, ** p <0.01.

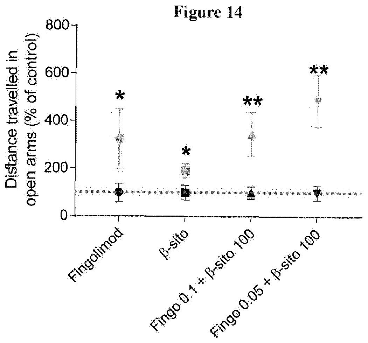

Figure 14 demonstrates an additive anxiolytic effect of a combined treatment with Fingolimod and β-sitosterol, as assessed by the EPM test. Each drug was administered intraperitoneally to wild-type C57BL6 mice. Fingolimod (0.1 mg / kg, every 3 days for 4 weeks), β-sitosterol (100 mg / kg, 1h before test), or a combination of Fingolimod + β-sitosterol at two different dosage (0.1 mg / kg Fingolimod + 100 mg / kg β-sitosterol or 0.05 mg / kg Fingolimod + 100 mg / kg β-sitosterol; both every 3 days for 4 weeks). n = 10 in Fingolimod treated group, n ≥ 5 in β-sitosterol treated group, n = 8-10 in the combination groups. Orange symbols represent mice treated with drugs; black symbols represent vehicle-treated mice. Data were normalized as percentage of the control response. * P < 0.05; ** P < 0.01.

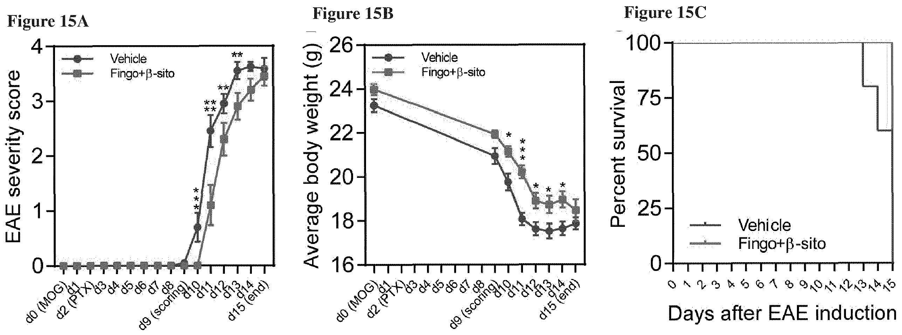

Figures 15A-C demonstrate the effect of a combined treatment with Fingolimod and β-sitosterol on the severity of EAE mouse model of Multiple Sclerosis. 0.1 mg / kg Fingolimod + 100 mg / kg β-sitosterol or vehicle control were injected intraperitoneally every 3 days for 4 weeks prior to induction of EAE (n = 10 mice per group). All surviving mice were sacrificed on Day 15. Figure 15A is a graph demonstrating EAE severity scores. The mice injected with Fingolimod + β-sitosterol exhibit an improvement of the EAE pathology with a delayed 1 day onset of the pathology and a significant reduction of the averaged score up to 13 days following EAE induction. Figure 15B is a graph demonstrating body weight indicating that the combined treatment reduces the weight loss from day 10 to day 14 compared to the vehicle control treatment. Figure 15C is a graph demonstrating mice survival up to day 15. Analysis of the survival curves shows a significant amelioration of survival in the combined treatment group compared to the vehicle control treatment group (Log-rank Mantel Cox test: P=0.0293 - Gehan-Breslow-Wilcoxon test: P=0.0301). Data is presented as mean ± S.E.M. * P < 0.05; ** P < 0.01, *** P < 0.001 [two-way analysis of variance (ANOVA)].

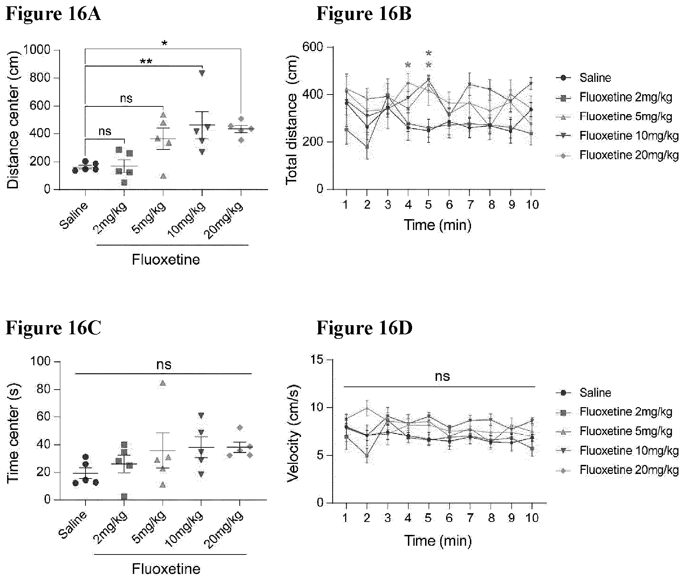

Figure 16A-D demonstrate a dose dependent anxiolytic effect of fluoxetine, as assessed by the OF test. Shown are the distance travelled in the OF center (Figure 16A), the total distance travelled (per minute) in the entire OF (Figure 16B), the time spent in the OF center (Figure 16C) and the movement velocity (in cm/s) (Figure 16D) of mice injected with 2, 5, 10 or 20 mg / kg fluoxetine as compared to a vehicle control (n=5 per each group) during the 10 minutes OF test. Data error bars represent mean +/- S.E.M. * p<0.05, ** p<0.01, *** p<0.001 (1-way ANOVA followed by Dunnett's multiple comparisons test in Figures 16A and 16C; 2-way ANOVA followed by Sidak's multiple comparisons test in Figures 16B and 16D).

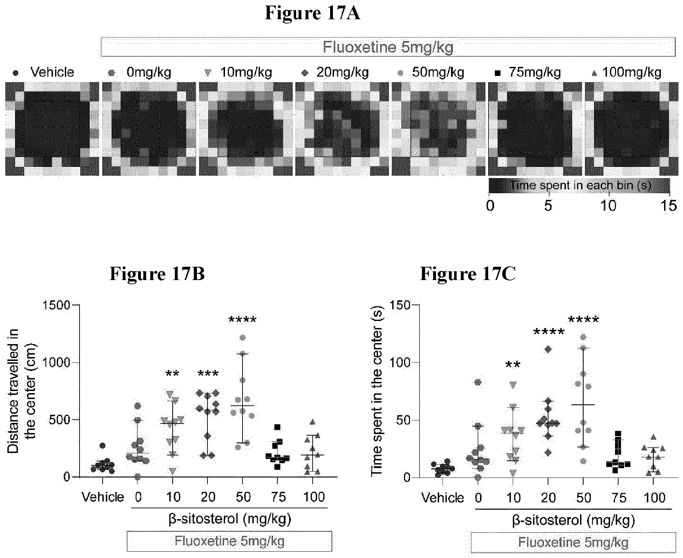

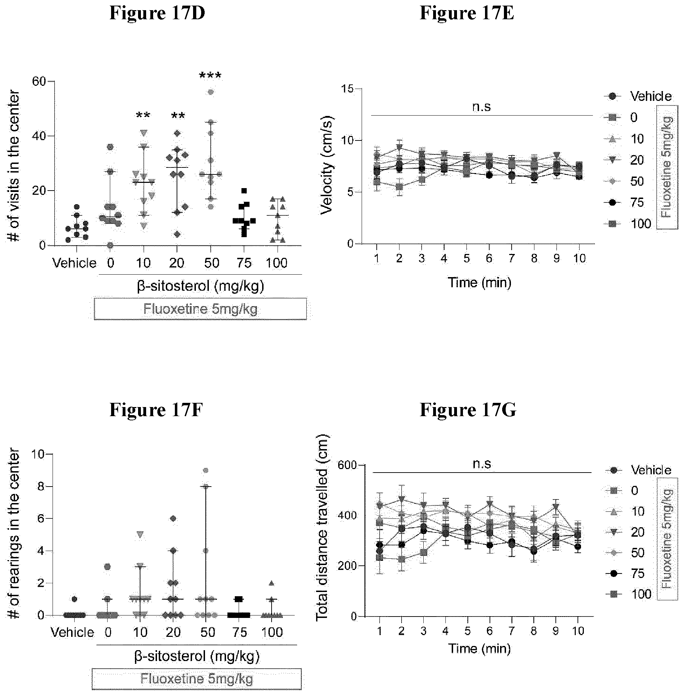

Figures 17A-G demonstrate an anxiolytic effect of a combined fluoxetine and β-sitosterol, as assessed by the OF test. 5 mg/ kg fluoxetine or vehicle control was injected intraperitoneally for 3 weeks and the indicated doses of β-sitosterol or vehicle control were injected intraperitoneally 1 hour prior to testing. Figure 17A shows hit-map representations demonstrating mice activity during the 10 minutes OF test. Figures 17B-G show graphs demonstrating the distance travelled (Figure 17B), the time spent in the OF center (Figure 17C), the number of visits in the OF center (Figure 17D), the movement velocity (in cm/s) (Figure 17E), the number of rearings (Figure 17F) and the total distance travelled (per minute) in the entire OF (Figure 17G) over the 10 minutes OF test, for mice receiving one of the 6 combinations as indicated (n ≥ 9 per combination) as compared to vehicle-injected animals (n = 9). In Figures 17B-D and 17F: data error bars represent the median with 95 % confidence interval (CI); *p<0.0332, **p<0.0021, ***p<0.0002, ****p<0.0001 (two-sided Kruskal-Wallis test). In Figures 17E and 17G: data error bars represent mean +/-S.E.M; * p<0.05, ** p<0.01, *** p<0.001 (2-way ANOVA followed by Sidak's multiple comparisons test).

### DESCRIPTION OF SPECIFIC EMBODIMENTS OF THE INVENTION

The present invention, in some embodiments thereof, relates to methods of treating psychiatric stress disorders.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Anxiety and stress-related conditions represent a significant health burden in modern society, currently treated with a variety of medicaments targeting synaptic mechanisms, such as Benzodiazepines and Selective Serotonin Uptake Inhibitors (SSRIs).

Importins are a group of proteins that transport protein molecules into the nucleus by binding to specific recognition sequences, called nuclear localization sequences (NLS). Importin polypeptide has two subunits, importin α and importin β, wherein members of the importin-β subfamily can bind NLS cargo proteins as homodimers or can form heterodimers with importin-a. There are 6-7 importin α family members in any given mammal and individual cell types express different subsets of this ensemble in a tightly regulated manner.

Whilst reducing the present invention to practice, the present inventors have now uncovered an importin α5 - MeCP2/LXR - Sphkl - SIP receptor pathway that provides molecular control of anxiety levels and demonstrate that targeting this pathway indeed modulates anxiety levels. In addition, the present inventors have also uncovered that alvespimycin has an anxiolytic effect.

As is illustrated hereinunder and in the examples section, which follows, the present inventors show, using a panel of behavioral assays, that importin α5 knockout (KO) mice present an anxiolytic phenotype (Examples 1-2, Figures 1A-C, 2A-I, 3A-G and 4A-F). While elucidating the mechanisms underlying the observed reduction in anxiety, the present inventors demonstrate significant changes in the expression of genes transcriptionally co-regulated by MeCP2 and Liver X Receptor (LXR), including Sphingosine kinase 1 (Sphkl) in the importin α5 KO mice (Example 2, Figures 5A-D and 6A-D). Immunofluorescent staining further show a significant reduction of nuclear localization of MeCP2 in the KO mice in specific brain regions such as the hippocampus, with no apparent effect on LXR nuclear localization (Example 2, Figures 5E-J and 7A-C). Without being bound by theory, these data suggest that expression levels of Sphkl are determined by the relative amounts of MeCP2 and LXRs in neuronal nuclei; and that removal of importin α5 reduces MeCP2 levels in the nucleus, thereby enabling LXR-induced up-regulation of Sphkl and subsequent increase of SIP activation of SIP receptors (Example 2, Figure 8A).

To verify this model and the possible option of treating anxiety and stress disorders by targeting this importin α5 - MeCP2/LXR - Sphkl - SIP receptor pathway, the present inventors show that administration of β-sitosterol (a plant-derived nutraceutical that can target LXR), fingolimod (FTY720, an SIP receptor agonist) or siponimod (an SIP receptor agonist) had significant anxiolytic effects in wild type mice, while on the contrary administration of PF-543 (a selective inhibitor of Sphkl) attenuated anxiolysis in the importin α5 KO mice (Example 3, Figure 8C-D, 9A-F, 10A-I, 11A-F, 13A-B and 14). Hence, the present inventors provide both molecular and pharmacological evidence for the involvement of the importin α5 - MeCP2/LXR - Sphkl - SIP receptor pathway in anxiety.

In addition, based on the importin α5 KO mice transcriptome, the present inventors were able to identify an additional compound, alvespimycin, and demonstrated that indeed this compound can serve as an anxiolytic agent (Example 4, Figures 12A-B).

Furthermore, the present inventors show that combined treatment with Fingolimod and β-sitosterol reduced severity of an EAE mouse model of multiple sclerosis (Example 5, Figures 15A-C).

Based on the above, the present inventors suggest that targeting the importin α5 - MeCP2/LXR - Sphkl - SIP receptor pathway using agents which down-regulate activity or expression of importin α5 or MeCP2 or up-regulate activity or expression of LXR or SIP receptor; and/or compositions comprising alvespimycin, can be used for the treatment of medical disorders that can benefit from up-regulating SIP receptor activity, in general, and psychiatric stress disorders, multiple sclerosis, Rett syndrome and MeCP2 duplication syndrome, in particular.

Thus, according to a first aspect of the present invention, there is provided an agent capable of down-regulating activity and/or expression of importin α5, for use in the treatment of a medical condition selected from the group consisting of psychiatric stress disorder, Rett syndrome, MeCP2 duplication syndrome and multiple sclerosis, with the proviso that said psychiatric stress disorder is not post-traumatic stress disorder mediated by inflammation.

According to another aspect of the present invention, there is provided a method of treating a medical condition selected from the group consisting of psychiatric stress disorder, Rett syndrome, MeCP2 duplication syndrome and multiple sclerosis in a subject in need thereof, the method comprising administering to the subject a therapeutically effective amount of an agent capable of down-regulating activity and/or expression of importin α5, with the proviso that said psychiatric stress disorder is not post-traumatic stress disorder mediated by inflammation, thereby treating the medical condition in the subject.

According to another aspect of the present invention, there is provided an agent capable of down-regulating activity of MeCP2, for use in the treatment of a medical condition selected from the group consisting of psychiatric stress disorder and multiple sclerosis.

According to another aspect of the present invention, there is provided a method of treating a medical condition selected from the group consisting of psychiatric stress disorder and multiple sclerosis in a subject in need thereof, the method comprising administering to the subject a therapeutically effective amount of an agent capable of down-regulating activity of MeCP2, thereby treating the medical condition in the subject.

According to another aspect of the present invention, there is provided an agent capable of up-regulating expression of liver X receptor (LXR), for use in the treatment of a medical condition selected from the group consisting of a psychiatric stress disorder and multiple sclerosis.

According to another aspect of the present invention, there is provided a method of treating a medical condition selected from the group consisting of psychiatric stress disorder and multiple sclerosis in a subject in need thereof, the method comprising administering to the subject a therapeutically effective amount of an agent capable of up-regulating expression of liver X receptor (LXR), thereby treating the medical condition in the subject.

According to another aspect of the present invention, there is provided a composition comprising as the active agent alvespimycin or a structural analog thereof for use in the treatment of a psychiatric stress disorder.

According to another aspect of the present invention, there is provided a method of treating a psychiatric stress disorder in a subject in need thereof, the method comprising administering to the subject a therapeutically effective amount of a composition comprising as the active agent alvespimycin or a structural analog thereof, thereby treating the psychiatric stress disorder in the subject.

According to another aspect of the present invention, there is provided a composition comprising as the active agent alvespimycin or a structural analog thereof for use in the treatment of a psychiatric stress disorder, wherein said psychiatric stress disorder is not substance induced anxiety and/or in co-morbidity with Alzheimer's disease.

According to another aspect of the present invention, there is provided a method of treating a psychiatric stress disorder in a subject in need thereof, wherein said psychiatric stress disorder is not substance induced anxiety and/or in co-morbidity with Alzheimer's disease, the method comprising administering to the subject a therapeutically effective amount of a composition comprising as the active agent alvespimycin or a structural analog thereof, thereby treating the psychiatric stress disorder in the subject.

According to another aspect of the present invention, there is provided a composition comprising as the active agent alvespimycin or a structural analog thereof for use in the treatment of a psychiatric stress disorder selected from the group consisting of acute stress disorder, post-traumatic stress disorder (PTSD), phobic anxiety disorder, separation anxiety disorder, obsessive-compulsive anxiety disorder, adjustment disorder with anxiety and selective mutism.

According to another aspect of the present invention, there is provided a method of treating a psychiatric stress disorder selected from the group consisting of acute stress disorder, post-traumatic stress disorder (PTSD), phobic anxiety disorder, separation anxiety disorder, obsessive-compulsive anxiety disorder, adjustment disorder with anxiety and selective mutism in a subject in need thereof, the method comprising administering to the subject a therapeutically effective amount of a composition comprising as the active agent alvespimycin or a structural analog thereof, thereby treating the psychiatric stress disorder in the subject.

According to specific embodiments, the composition consists of alvespimycin or a structural analog thereof as the active agent.

According to specific embodiments, a composition or a combination comprising alvespimycin or a structural analog thereof does not comprise a compound that degrades protein, such as disclosed in International Application Publication No. WO2013170147, the contents of which are fully incorporated herein by reference.

According to another aspect of the present invention, there is provided a combination of at least two agents selected from the group consisting of:
(i) an agent capable of down-regulating activity and/or expression of importin α5;
(ii) an agent capable of down-regulating activity and/or expression of MeCP2;
(iii) an agent capable of up-regulating activity and/or expression of liver X receptor (LXR);
(iv) an agent capable of up-regulating activity and/or expression of SIP receptor; and
(v) alvespimycin or a structural analog thereof;
for the treatment of a medical condition that can benefit from up-regulating activity of S1P receptor.

According to another aspect of the present invention, there is provided a method of treating a medical condition that can benefit from up-regulating activity of SIP receptor in a subject in need thereof, the method comprising administering to the subject a therapeutically effective amount of at least two agents selected from the group consisting of:
(i) an agent capable of down-regulating activity and/or expression of importin α5;
(ii) an agent capable of down-regulating activity and/or expression of MeCP2;
(iii) an agent capable of up-regulating activity and/or expression of liver X receptor (LXR);
(iv) an agent capable of up-regulating activity and/or expression of SIP receptor; and
(v) alvespimycin or a structural analog thereof;
thereby treating the medical condition in the subject.

According to specific embodiments, the at least two agents of the present invention comprise two, three, four or all of (i) - (v).

According to specific embodiments, the at least two agents of the present invention comprise (i) + (ii), (ii) + (iii), (iii) + (iv), (i) + (iii), (i) + (iv), (iii) + (v), (iv) + (v), (i) + (iii) + (iv), (ii) + (iii) + (iv) and/or (iii) + (iv) + (v), each possibility represents a separate embodiment of the present invention.

According to a specific embodiment, the at least two agents comprise (iii) + (iv).

According to specific embodiments, each of the at least two agents is packaged in a separate container.

According to other specific embodiments, the at least two agents are in a co-formulation.

According to specific embodiments, the combination of at least two agents described herein has a combined improved activity. As used herein the phrase "combined improved activity" refers to at least additive but preferably synergistically improved effect by the present invention. Assays for determining additive effect or synergy are well within the capabilities of the skilled artisan e.g., PCR, ELISA, Western blot analysis, immunoprecipitation, flow cytometry, immuno-staining, kinase assays, as well as behavioral assays such as the open field (OF) test, the Elevated plus maze (EPM) test and the Acoustic startle reflex (ASR) and standard pharmaceutical procedures for determining dosing, toxicity and therapeutic efficacy by *in-vitro*, in cell cultures or experimental animals.

According to specific embodiments, the combination of the at least two agent has a synergistic effect as compared to the administration of only one of the agents.

Alvespimycin, also known as 17-DMAG, UNII-001L2FE0M3, CHEBI:65324, 467214-20-6, KOS-1022, is an HSP90 inhibitor.

Analogs of alvespimycin can be selected using activity and structural assays that are well known to the skilled artisan, some of this are described herein e.g., behavioral assays such as the open field (OF) test, the Elevated plus maze (EPM) test and the Acoustic startle reflex (ASR) test.

According to specific embodiments, a structural analog of alvespimycin is selected from the group consisting of geldanamycin and tanespimycin (also known as 17-N-allylamino-17-demethoxygeldanamycin and 17-AAG).

The term "treating" or "treatment" refers to inhibiting, preventing or arresting the development of a pathology (disease, disorder or medical condition e.g. psychiatric stress disorder, multiple sclerosis, Rett syndrome, MeCP2 duplication syndrome) and/or causing the reduction, remission, or regression of a pathology or a symptom of a pathology. Those of skill in the art will understand that various methodologies and assays can be used to assess the development of a pathology, and similarly, various methodologies and assays may be used to assess the reduction, remission or regression of a pathology.

As used herein, the term "subject" includes mammals, e.g., human beings at any age and of any gender who suffer from the pathology (medical condition). According to specific embodiments, this term encompasses individuals who are at risk to develop the pathology.

According to specific embodiments, the subject is above 13 years old, above 18 years old or above 21 years old.

According to specific embodiments, the subject is below 80 years old, below 65 years old or below 45 years old.

According to specific embodiments, the pathology is a medical condition that can benefit from up-regulating activity of S1P receptor.

According to specific embodiments, the SIP receptor activity and/or expression is down-regulated in diseased cells as compared to control cells not afflicted with the medical condition.

Non-limiting examples of medical conditions that can benefit from up-regulating activity of SIP receptor include psychiatric stress disorder, multiple sclerosis, Rett syndrome, MeCP2 duplication syndrome, neuropathy, dermatitis, arthritis, allergy, diabetic nephropathy, renal protection, emphysema, neuroprotection, spontaneous autoimmune polyneuropathy, sepsis, transplantation, experimental autoimmune neuritis and dampening the cytokine response during influenza virus infection.

According to specific embodiments, the medical condition is selected from the group consisting of psychiatric stress disorder, multiple sclerosis, Rett syndrome and MeCP2 duplication syndrome.

According to specific embodiments, the medical condition is selected from the group consisting of psychiatric stress disorder, Rett syndrome and MeCP2 duplication syndrome.

As used herein the term "psychiatric stress disorder" refers to a stress disorder manifested by unusual behavioral or mental patterns that cause distress or disability to the individual and which can be alleviated by an anxiolytic agent. Non-limiting examples of psychiatric stress disorders include anxiety, acute stress disorder, post-traumatic stress disorder (PTSD), depression [e.g. psychosocial stress-related depression and major depressive disorder (MDD)].

According to specific embodiments, the psychiatric stress disorder is not post-traumatic stress disorder (PTSD) mediated by inflammation.

According to specific embodiments, the psychiatric stress disorder is anxiety.

Non-limiting examples of anxiety disorders include generalized anxiety disorder (GAD), panic attacks, phobic anxiety disorders (e.g., acrophobia, claustrophobia, agoraphobia, social phobia, and other phobias), social anxiety disorder, separation anxiety disorder, situational anxiety, psychoactive substance anxiety disorder, organic anxiety disorder, obsessive-compulsive anxiety disorder, adjustment disorder with anxiety and selective mutism.

According to specific embodiments, the psychiatric stress disorder (or the medical condition) is acute stress disorder, post-traumatic stress disorder (PTSD), phobic anxiety disorder, separation anxiety disorder, obsessive-compulsive anxiety disorder, adjustment disorder with anxiety and/or selective mutism; each possibility represents a separate embodiment of the present invention.

According to specific embodiments, when alvespimycin or a structural analog thereof is used then the psychiatric stress disorder or the medical condition) is selected from the group consisting of acute stress disorder, post-traumatic stress disorder (PTSD), phobic anxiety disorder, separation anxiety disorder, obsessive-compulsive anxiety disorder, adjustment disorder with anxiety and selective mutism.

Thus, according to specific embodiments, the at least two agent comprise alvespimycin or a structural analog thereof and the psychiatric stress disorder or the medical condition) is selected from the group consisting of acute stress disorder, post-traumatic stress disorder (PTSD), phobic anxiety disorder, separation anxiety disorder, obsessive-compulsive anxiety disorder, adjustment disorder with anxiety and selective mutism.

According to specific embodiments, the psychiatric stress disorder (or the medical condition) is not substance induced anxiety.

According to specific embodiments, when alvespimycin or a structural analog thereof is used then the psychiatric stress disorder is not substance induced anxiety.

Thus, according to specific embodiments, the at least two agents comprise alvespimycin or a structural analog thereof and the psychiatric stress disorder is not substance induced anxiety.

According to specific embodiments, the psychiatric stress disorder (or the medical condition) is not in comorbidity with other diseases e.g., cancer, IBD, diabetes, hypertension, ALS, Parkinson, Alzheimer's disease, Rett syndrome and/or MeCP2 duplication syndrome.

According to specific embodiments, the psychiatric stress disorder (or the medical condition) is not in co-morbidity with MeCP2 duplication syndrome.

According to specific embodiments, the psychiatric stress disorder (or the medical condition) is not in co-morbidity with Alzheimer's disease.

According to specific embodiments, when alvespimycin or a structural analog thereof is used then the psychiatric stress disorder is not in co-morbidity with Alzheimer's disease.

Thus, according to specific embodiments, the at least two agents comprise alvespimycin or a structural analog thereof and the psychiatric stress disorder is not in co-morbidity with Alzheimer's disease.

According to some embodiments, the multiple sclerosis (MS) relates to any form of the disease including relapsing remitting MS (RRMS), (SPMS), secondary progressive MS, primary progressive MS (PPMS) and progressive relapsing MS (PRMS).

According to specific embodiments, the medical condition is not multiple sclerosis or in co-morbidity with multiple sclerosis.

According to specific embodiments, when an agent capable of down-regulating activity and/or expression of importin α5, an agent capable of up-regulating activity and/or expression of liver X receptor (LXR) and/or an agent capable of up-regulating activity and/or expression of SIP receptor is used then the medical condition is not multiple sclerosis.

Thus, according to specific embodiments, the at least two agents comprise an agent capable of down-regulating activity and/or expression of importin α5, an agent capable of up-regulating activity and/or expression of liver X receptor (LXR) and/or an agent capable of up-regulating activity and/or expression of SIP receptor and the medical condition is not multiple sclerosis.

"Rett syndrome" refers to an X-linked disorder caused by a mutation(s) in the MECP2 gene resulting in reduced expression or expression of aberrant protein. Examples of MeCP2 mutations that are associated with Rett syndrome are described for examples in Guy et al, Nat Genet. (2001) Mar;27(3):322-6, Bienvenu et al., Hum Mol Genet. (2000) May 22;9(9): 1377-84, Wan et al., Am J Hum Genet. (1999) Dec;65(6): 1520-9, and Amir et al, Nat Genet. (1999) Oct;23(2): 185- 8.

According to specific embodiments, the "Rett syndrome" that can be treated according to specific embodiments of the present invention is caused by expression of an aberrant MeCP2 protein. According to specific embodiments, the aberrant protein has an altered interaction/activity of the functional domains such as the MBD (Methyl-binding domain - i.e. reduced interaction with the target DNA sequences) or the TRD (transcription repression domain - i.e. reduced ability to recruit a transcription repression complex) and eventually the NLS (in the case of altered localization).

According to specific embodiments, the medical condition is not Rett syndrome or in co-morbidity with Rett syndrome.

"MeCP2 duplication syndrome" refers to an X-linked disorder caused by over expression of the MECP2 protein.

According to specific embodiments, the medical condition is not MeCP2 duplication syndrome or in co-morbidity with MeCP2 duplication syndrome.

According to specific embodiments, when an agent capable of down-regulating activity and/or expression of MeCP2 is used the medical condition is not MeCP2 duplication syndrome or in co-morbidity with MeCP2 duplication syndrome.

Thus, according to specific embodiments, the at least two agents comprise an agent capable of down-regulating activity and/or expression of MeCP2 and the medical condition is not MeCP2 duplication syndrome or in co-morbidity with MeCP2 duplication syndrome.

As used herein, the term "MeCP2 (methyl CpG binding protein 2)" refers to the polynucleotide or polypeptide expression product of the MECP2 gene (Gene ID: 4204). According to specific embodiments, the MeCP2 refers to the human MeCP2, such as provided in the following Accession Numbers: NM_001110792, NM_004992, NM_001316337, NP_001104262, NP_001303266 and NP_004983. According to specific embodiments, the MeCP2 refers to the mouse MeCP2, such as provided in the following Accession Numbers: NM_001081979, NM_010788, NP_001075448, NP_034918, NP_001075448 and NP_034918.

According to specific embodiments, MeCP2 activity is at least one of (or two of or all of): binding importin α5, translocating into the nucleus, binding the Sphingosine kinase 1 (Sphkl) promoter (typically at the nucleic acid sequence TTAACTCCGG, SEQ ID NO: 1) and/or inhibiting Sphkl transcription.

Hence, according to specific embodiments, the agent which down-regulates activity of MeCP2 down-regulates MeCP2 translocation into the nucleus, as determined by e.g. immuno-staining.

According to other specific embodiments, the agent which down-regulates activity of MeCP2 down-regulates MeCP2 binding to importin α5, as determined by e.g. immunoprecipitation, ELISA, flow cytometry or other known binding assays.

According to other specific embodiments, the agent which down-regulates activity of MeCP2 down-regulates MeCP2 binding to the promoter of sphingosine kinase 1 (Sphkl), as determined by e.g.immunoprecipitation, ELISA, flow cytometry or other known binding assays.

According to a specific embodiment, the agent which down-regulates activity of MeCP2 down-regulates MeCP2 binding to a nucleic acid sequence comprising TTAACTCCGG, SEQ ID NO: 1.

As used herein the term "importin α5", also known as Karyopherin Subunit Alpha 1, refers to the polynucleotide or polypeptide expression product of the KPNA1 gene (Gene ID: 3836). According to specific embodiments, the importin α5 refers to the human importin α5, such as provided in the following Accession Numbers: NM_002264, and NP_002255. According to specific embodiments, the importin α5 refers to the mouse importin α5, such as provided in the following Accession Numbers: NM_008465, NP_032491 and NP_032491.

According to specific embodiments, importin α5 activity is at least one of (or two of or all of): forming a heterodimer with importin β, binding to the NLS sequence of MeCP2 and acting as a chaperone transporting the target protein (e.g. MeCP2) into the nucleus.

Hence, according to specific embodiments, the agent which down-regulates activity of importin α5 down-regulates importin α5 binding to the NLS sequence of MeCP2 (RKAEADPQAIPKKRGRK, SEQ ID NO: 2), as determined by e.g. immunoprecipitation, ELISA, flow cytometry or other known binding assays.

As used herein, the term "liver X receptor (LXR)" encompasses LXRα and LXRβ.

According to specific embodiments, LXR is LXRα.

"LXRα" refers to the polynucleotide or polypeptide expression product of the NR1H3 gene (Gene ID: 10062). According to specific embodiments, the LXRα refers to the human LXRα, such as provided in the following Accession Numbers: NM_001130101, NM_001130102, NM_001251934, NM_001251935, NM_005693, NP_001123573, NP_001123574, NP_001238863, NP_001238864, NP_005684. According to specific embodiments, the LXRα refers to the mouse LXRα, such as provided in the following Accession Numbers: NM_001177730, NM_013839, NP_001171201, NP_038867, NP_001171201 and NP_038867.

"LXRβ" refers to the polynucleotide or polypeptide expression product of the NR1H2 gene (Gene ID: 7376). According to specific embodiments, the LXRβ refers to the human LXRβ, such as provided in the following Accession Numbers: NM_007121, NM_001256647, NP_001243576, NP_009052 and NP_009052. According to specific embodiments, the LXRβ refers to the mouse LXRβ, such as provided in the following Accession Numbers: NM_001285517, NM_001285518, NM_001285519, NM_009473, P_001272446, NP_001272447, NP_001272448, NP_033499 and NP_001272446.

According to specific embodiments, LXR activity is at least one of (or two of or all of): translocating into the nucleus, binding the Sphingosine kinase 1 (Sphkl) promoter (typically at the nucleic acid sequence CCGGTTAGTCACGGACA, SEQ ID NO: 3) and activating Sphkl transcription.

Hence, according to specific embodiments, the agent which down-regulates activity of LXE down-regulates LXR translocation into the nucleus, as determined by e.g. immuno-staining.

According to other specific embodiments, the agent which down-regulates activity of LXR down-regulates LXR binding to the promoter of sphingosine kinase 1 (Sphkl), as determined by e.g. immunoprecipitation, ELISA, flow cytometry or other known binding assays.

According to a specific embodiment, the agent which down-regulates activity of LXR down-regulates LXR binding to a nucleic acid sequence comprising CCGGTTAGTCACGGACA, SEQ ID NO: 3.

As used herein, the term "sphingosine kinase 1 (Sphkl)" refers the polynucleotide or polypeptide expression product of the *SPHK1* gene (Gene ID: 8877). According to specific embodiments, the Sphkl refers to the human Sphkl, such as provided in the following Accession Numbers: NM_001142601, NM_001142602, NM_021972, NM_182965, NP_001136073, NP_001136074, NP_068807 and NP_892010. According to specific embodiments, the Sphkl refers to the mouse Sphkl, such as provided in the following Accession Numbers: NM_001172472, NM_001172473, NM_001172475, NM_011451, NM_025367, NP_001165943, NP_001165944, NP_001165946, NP_035581 and NP_079643.

As used herein the term "sphingosine-1-phosphate receptor (SIP receptor or S1PR)" refers to a class of G protein-coupled receptor that is a target of the lipid signaling molecule Sphingosine-1-phosphate (SIP) and encompasses S1PR1, S1PR2, S1PR3, S1PR4 and S1PR5.

According to specific embodiments, S1PR is S1PR1.

"S1PR1", also known as endothelial differentiation gene 1 (EDG1) refers to the polynucleotide or polypeptide expression product of the S1PR1 gene (Gene ID: 1901). According to specific embodiments, the S1PR1 refers to the human S1PR1, such as provided in the following Accession Numbers: NM_001400, NM_001320730, NP_001307659 and NP_001391. According to specific embodiments, the S1PR1 refers to the mouse S1PR1, such as provided in the following Accession Numbers: NM_007901, NP_031927 and NP_031927.

According to specific embodiments, S1PR is S1PR2.

"S1PR2" refers to the polynucleotide or polypeptide expression product of the S1PR2 gene (Gene ID: 9294). According to specific embodiments, the S1PR2 refers to the human S1PR2, such as provided in the following Accession Numbers: NM_004230 and NP_004221. According to specific embodiments, the S1PR2 refers to the mouse S1PR2, such as provided in the following Accession Numbers: NM_010333 and NP_034463.

According to specific embodiments, S1PR is S1PR3.

"S1PR3" refers to the polynucleotide or polypeptide expression product of the S1PR3 gene (Gene ID: 1903). According to specific embodiments, the S1PR3 refers to the human S1PR3, such as provided in the following Accession Numbers: NM_005226 and NP_005217. According to specific embodiments, the S1PR3 refers to the mouse S1PR3, such as provided in the following Accession Numbers: NM_010101 and NP_034231.

According to specific embodiments, S1PR is S1PR4.

"S1PR4" refers to the polynucleotide or polypeptide expression product of the S1PR4 gene (Gene ID: 8698). According to specific embodiments, the S1PR4 refers to the human S1PR4 such as provided in the following Accession Numbers: NM_003775 and NP_003766. According to specific embodiments, the S1PR4 refers to the mouse S1PR4, such as provided in the following Accession Numbers: NM_010102 and NP_034232.

According to specific embodiments, S1PR is S1PR5.

"S1PR5" refers to the polynucleotide or polypeptide expression product of the S1PR5 gene (Gene ID: 53637). According to specific embodiments, the S1PR5 refers to the human S1PR5 such as provided in the following Accession Numbers: NM_030760, NM_001166215, NP_001159687 and NP_110387. According to specific embodiments, the S1PR5 refers to the mouse S1PR5, such as provided in the following Accession Numbers: NM_053190 and NP_444420.

According to specific embodiments, S1P receptor activity is at least one of (or two of or all of): binding its ligand SIP, binding the agent capable of up-regulating SIP receptor activity of some embodiments of the invention; and transmitting a down-stream signal.

Hence, according to specific embodiments, the agent which down-regulates activity of SIP receptor down-regulates SIP receptor binding to its ligand SIP, as determined by e.g. immunoprecipitation, ELISA, flow cytometry or other known binding assays.

According to specific embodiments, the agent which down-regulates activity of SIP receptor down-regulates transmission of the receptor down-stream signal, as determined by e.g. enzymatic assays such as kinase activity assays, binding assays such as flow cytometry and immunoprecipitation or expression of molecules involved in the signaling cascade using e.g. PCR, Western blot, immunoprecipitation or immunohistochemistry. Additionally or alternatively, determining transmission of a signal can be effected by evaluating a biological outcome of same.

As mentioned, the agents disclosed herein are capable of modulating (up-regulating or down-regulating, depending on the target) activity and/or expression of a target expression product (e.g. importin α5, MeCP2, LXR, SIP receptor).

According to specific embodiments, the agent directly binds the target.

According to other specific embodiments, the agent indirectly binds the target by acting through an intermediary molecule, for example the agent binds to or modulates a molecule that in turn binds to or modulates the target.

As used herein, "modulating (i.e. up-regulating or down-regulating) activity and/or expression" refers to a change (i.e. increase or decrease, respectively) of at least 5 % in biological function and/or expression in the presence of the agent in comparison to same in the absence of the agent, as determined by e.g. PCR, ELISA, Western blot analysis, immunoprecipitation, flow cytometry, immuno-staining, kinase assays. As the agents of the present invention have an anxiolytic effect, the change can also be determined by behavioral assays such as the open field (OF) test, the Elevated plus maze (EPM) test and the Acoustic startle reflex (ASR) test, which are further described in details in the Examples section which follows. According to a specific embodiment, the change is in at least 10 %, 30 %, 40 % or even higher say, 50 %, 60 %, 70 %, 80 %, 90 % or more than 100 %. According to specific embodiments, the change is at least 1.5 fold, at least 2 fold, at least 3 fold, at least 5 fold, at least 10 fold, or at least 20 fold as compared to same in the absence of the agent.

According to specific embodiments, the agent up-regulates activity and/or expression of the target expression product.

Up-regulating activity and/or expression can be effected at the protein level (e.g., antibodies, small molecules, peptides and the like) but may also be effected at the genomic level (e.g., activation of transcription via promoters, enhancers, regulatory elements) and/or the transcript level using a variety of molecules which promote transcription and/or translation (e.g., correct splicing, polyadenylation, activation of translation) of a target expression product described herein.

Non-limiting examples of agents that can function as up-regulating agents are described in details hereinbelow.

### Up-regulation at the polypeptide level

According to specific embodiments, the agonist is the naturally occurring activator or a functional derivative or variant thereof which retain the ability to specifically bind to the target protein.

It will be appreciated that a functional analogue of at least a catalytic or binding portion of a target protein can be also used as an up-regulating agent. Thus, according to specific embodiments, the agent is an exogenous polypeptide including at least a functional portion (e.g. catalytic or interaction) of the target protein.

According to specific embodiments, the agonist is an antibody.

According to specific embodiments the antibody is capable of specifically binding a target protein described herein. According to specific embodiments, the antibody specifically binds at least one epitope of a target protein described herein.

As used herein, the term "epitope" refers to any antigenic determinant on an antigen to which the paratope of an antibody binds. Epitopic determinants usually consist of chemically active surface groupings of molecules such as amino acids or carbohydrate side chains and usually have specific three dimensional structural characteristics, as well as specific charge characteristics.

The term "antibody" as used in this invention includes intact molecules as well as functional fragments thereof, such as Fab, F(ab')2, Fv, scFv, dsFv, or single domain molecules such as VH and VL that are capable of binding to an epitope of an antigen. The antibody may be mono-specific (capable of recognizing one epitope or protein), bi-specific (capable of binding two epitopes or proteins) or multi-specific (capable of recognizing multiple epitopes or proteins).

Suitable antibody fragments for practicing some embodiments of the invention include a complementarity-determining region (CDR) of an immunoglobulin light chain (referred to herein as "light chain"), a complementarity-determining region of an immunoglobulin heavy chain (referred to herein as "heavy chain"), a variable region of a light chain, a variable region of a heavy chain, a light chain, a heavy chain, an Fd fragment, and antibody fragments comprising essentially whole variable regions of both light and heavy chains such as an Fv, a single chain Fv (scFv), a disulfide-stabilized Fv (dsFv), an Fab, an Fab', and an F(ab')2.

As used herein, the terms "complementarity-determining region" or "CDR" are used interchangeably to refer to the antigen binding regions found within the variable region of the heavy and light chain polypeptides. Generally, antibodies comprise three CDRs in each of the VH (CDR HI or HI; CDR H2 or H2; and CDR H3 or H3) and three in each of the VL (CDR LI or LI; CDR L2 or L2; and CDR L3 or L3).

The identity of the amino acid residues in a particular antibody that make up a variable region or a CDR can be determined using methods well known in the art and include methods such as sequence variability as defined by Kabat et al. (See, e.g., Kabat et al., 1992, Sequences of Proteins of Immunological Interest, 5th ed., Public Health Service, NIH, Washington D.C.), location of the structural loop regions as defined by Chothia et al. (see, e.g., Chothia et al., Nature 342:877-883, 1989.), a compromise between Kabat and Chothia using Oxford Molecular's AbM antibody modeling software (now Accelrys®, see, Martin et al., 1989, Proc. Natl Acad Sci USA. 86:9268; and world wide web site www(dot)bioinf-org(dot)uk/abs), available complex crystal structures as defined by the contact definition (see MacCallum et al., J. Mol. Biol. 262:732-745, 1996), the "conformational definition" (see, e.g., Makabe et al., Journal of Biological Chemistry, 283:1156-1166, 2008) and IMGT [Lefranc MP, et al. (2003) IMGT unique numbering for immunoglobulin and T cell receptor variable domains and Ig superfamily V-like domains. Dev Comp Immunol 27: 55-77].

As used herein, the "variable regions" and "CDRs" may refer to variable regions and CDRs defined by any approach known in the art, including combinations of approaches.

Functional antibody fragments comprising whole or essentially whole variable regions of both light and heavy chains are defined as follows:
(i) Fv, defined as a genetically engineered fragment consisting of the variable region of the light chain (VL) and the variable region of the heavy chain (VH) expressed as two chains;
(ii) single chain Fv ("scFv"), a genetically engineered single chain molecule including the variable region of the light chain and the variable region of the heavy chain, linked by a suitable polypeptide linker as a genetically fused single chain molecule.
(iii) disulfide-stabilized Fv ("dsFv"), a genetically engineered antibody including the variable region of the light chain and the variable region of the heavy chain, linked by a genetically engineered disulfide bond.
(iv) Fab, a fragment of an antibody molecule containing a monovalent antigen-binding portion of an antibody molecule which can be obtained by treating whole antibody with the enzyme papain to yield the intact light chain and the Fd fragment of the heavy chain which consists of the variable and CH1 domains thereof;
(v) Fab', a fragment of an antibody molecule containing a monovalent antigen-binding portion of an antibody molecule which can be obtained by treating whole antibody with the enzyme pepsin, followed by reduction (two Fab' fragments are obtained per antibody molecule);
(vi) F(ab')2, a fragment of an antibody molecule containing a monovalent antigen-binding portion of an antibody molecule which can be obtained by treating whole antibody with the enzyme pepsin (i.e., a dimer of Fab' fragments held together by two disulfide bonds); and
(vii) Single domain antibodies or nanobodies are composed of a single VH or VL domains which exhibit sufficient affinity to the antigen.

The antibody may be monoclonal or polyclonal.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988, incorporated herein by reference).

Antibody fragments according to some embodiments of the invention can be prepared by proteolytic hydrolysis of the antibody or by expression in E. coli or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331,647, and references contained therein, which patents are hereby incorporated by reference in their entirety. See also Porter, R. R. [Biochem. J. 73: 119-126 (1959)]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al. [Proc. Nat'l Acad. Sci. USA 69:2659-62 (19720]. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into a host cell such as E. coli. The recombinant host cells synthesize a single polypeptide chain with a linker peptide bridging the two V domains. Methods for producing sFvs are described, for example, by [Whitlow and Filpula, Methods 2: 97-105 (1991); Bird et al., Science 242:423-426 (1988); Pack et al., Bio/Technology 11:1271-77 (1993); and U.S. Pat. No. 4,946,778, which is hereby incorporated by reference in its entirety.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry [Methods, 2: 106-10 (1991)].

It will be appreciated that for human therapy or diagnostics, humanized antibodies are preferably used. Humanized forms of non-human (e.g., murine) antibodies are chimeric molecules of immunoglobulins, immunoglobulin chains or fragments thereof (such as Fv, Fab, Fab', F(ab').sub.2 or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

As some of the targets described herein are localized intracellularly, the antibody or antibody fragment capable can be an intracellular antibody (also known as "intrabodies"). Intracellular antibodies are essentially SCA to which intracellular localization signals have been added (e.g., ER, mitochondrial, nuclear, cytoplasmic). This technology has been successfully applied in the art (for review, see Richardson and Marasco, 1995, TIBTECH vol. 13). Intrabodies have been shown to virtually eliminate the expression of otherwise abundant cell surface receptors and to inhibit a protein function within a cell (See, for example, Richardson et al., 1995, Proc. Natl. Acad. Sci. USA 92: 3137-3141; Deshane et al., 1994, Gene Ther. 1: 332-337; Marasco et al., 1998 Human Gene Ther 9: 1627-42; Shaheen et al., 1996 J. Virol. 70: 3392-400; Werge, T. M. et al., 1990, FEBS Letters 274:193-198; Carlson, J.R. 1993 Proc. Natl. Acad. Sci. USA 90:7427-7428; Biocca, S. et al., 1994, Bio/Technology 12: 396-399; Chen, S-Y. et al., 1994, Human Gene Therapy 5:595-601; Duan, L et al., 1994, Proc. Natl. Acad. Sci. USA 91:5075-5079; Chen, S-Y. et al., 1994, Proc. Natl. Acad. Sci. USA 91:5932-5936; Beerli, R.R. et al., 1994, J. Biol. Chem. 269:23931-23936; Mhashilkar, A.M. et al., 1995, EMBO J. 14:1542-1551; PCT Publication No. WO 94/02610 by Marasco et al.; and PCT Publication No. WO 95/03832 by Duan et al.).

To prepare an intracellular antibody expression vector, the cDNA encoding the antibody light and heavy chains specific for the target protein of interest are isolated, typically from a hybridoma that secretes a monoclonal antibody specific for the marker. Hybridomas secreting anti-marker monoclonal antibodies, or recombinant monoclonal antibodies, can be prepared using methods known in the art. Once a monoclonal antibody specific for the marker protein is identified (e.g., either a hybridoma-derived monoclonal antibody or a recombinant antibody from a combinatorial library), DNAs encoding the light and heavy chains of the monoclonal antibody are isolated by standard molecular biology techniques. For hybridoma derived antibodies, light and heavy chain cDNAs can be obtained, for example, by PCR amplification or cDNA library screening. For recombinant antibodies, such as from a phage display library, cDNA encoding the light and heavy chains can be recovered from the display package (e.g., phage) isolated during the library screening process and the nucleotide sequences of antibody light and heavy chain genes are determined. For example, many such sequences are disclosed in Kabat, E. A., et al. (1991) Sequences of Proteins of Immunological Interest, Fifth Edition, U.S. Department of Health and Human Services, NIH Publication No. 91-3242 and in the "Vbase" human germline sequence database. Once obtained, the antibody light and heavy chain sequences are cloned into a recombinant expression vector using standard methods.

For cytoplasmic expression of the light and heavy chains, the nucleotide sequences encoding the hydrophobic leaders of the light and heavy chains are removed. An intracellular antibody expression vector can encode an intracellular antibody in one of several different forms. For example, in one embodiment, the vector encodes full-length antibody light and heavy chains such that a full-length antibody is expressed intracellularly. In another embodiment, the vector encodes a full-length light chain but only the VH/CH1 region of the heavy chain such that a Fab fragment is expressed intracellularly. In another embodiment, the vector encodes a single chain antibody (scFv) wherein the variable regions of the light and heavy chains are linked by a flexible peptide linker [e.g., (Gly₄Ser)₃ and expressed as a single chain molecule. To inhibit marker activity in a cell, the expression vector encoding the intracellular antibody is introduced into the cell by standard transfection methods, as discussed hereinbefore.

Once antibodies are obtained, they may be tested for activity, for example via ELISA.

Another up-regulating agent would be a molecule which promotes and/or increases the function (e.g. catalytic or interaction) of the target protein by binding to the target or an intermediate thereof. Such molecules can be, but are not limited to, small molecules, peptides and aptamers, wherein each possibility is a separate embodiment of the invention.

According to specific embodiments, the agent is a peptide.

According to specific embodiments, the agent is a small molecule.

Non-limiting examples of agents capable of up-regulating activity of SIP receptor include fingolimod (FTY720), siponimod, ozanimod, AUY954, AAL(R), sphingosine-1-phosphate, sphingosine analogues, 2-substituted 2-amino-propane-1,3-diol or 2-amino-propanol derivatives of sphingosine, (S)-phosphoric acid mono-[2-amino-3-(4-octyl-phenylamino)-propyl]ester (VPC 24191; Avanti® Polar Lipids, Inc., Alabaster, Ala.), (R)-phosphoric acid mono-[2-amino-2-(6-octyl-1H-benzoimiazol-2-yl)-ethyl]ester (VPC 23153, Avanti® Polar Lipids, Inc.), CID 2321431 (SID 3714904), CID 5309153 (SID 7967985), CID 665518 (SID 864271), CID 2842253 (SID 7977380), KM10340 (3-(6-tert-butyl-1,1-dimethyl-2,3-dihydro-1H-inden-4-yl)-5-(trifluoromethyl)-1H-pyrazole), TC-SP 14 (CAS# 1257093-40-5), SEW 2871 (CAS# 256414-75-2), RP 001 hydrochloride (CAS# 1306761-53-4), CYM 5442 hydrochloride (CAS# 1094042-01-9), CS 2100 (CAS# 913827-99-3), CYM 50260, CYM 50308, CYM 5520, CYM 5541, RP 001 hydrochloride, TC-G 1006, A 971432, TC-SP 14 and those agonists described in U.S. Patent Application Publication Nos. 2011/0124605 and 2017/0020826; U.S. Pat. Nos. 7,842,685; 7,064,217; 7,208,502; 7,241,790; and 7,638,637; Shurer et al. (2008) ACS Chem. Biol., 3:486-498); Sammani et al. (2011) Am. J. Respir. Cell Mol. Biol., 45:1022-7; and "MLSCN Probe Summary: S1P3 Agonist" by The Scripps Research Institute Molecular Screening Center (available at mli(dot)nih(dot)gov/mli/?dl_id=721).

According to specific embodiments, the agent capable of up-regulating activity of SIP receptor is fingolimod (FTY720), siponimod, AUY954, AAL(R), sphingosine-1-phosphate, sphingosine analogues, 2-substituted 2-amino-propane-1,3-diol or 2-amino-propanol derivatives of sphingosine, (S)-phosphoric acid mono-[2-amino-3-(4-octyl-phenylamino)-propyl]ester (VPC 24191; Avanti® Polar Lipids, Inc., Alabaster, Ala.), (R)-phosphoric acid mono-[2-amino-2-(6-octyl-1H-benzoimiazol-2-yl)-ethyl]ester (VPC 23153, Avanti® Polar Lipids, Inc.), CID 2321431 (SID 3714904), CID 5309153 (SID 7967985), CID 665518 (SID 864271), CID 2842253 (SID 7977380), KM10340 (3-(6-tert-butyl-1,1-dimethyl-2,3-dihydro-1H-inden-4-yl)-5-(trifluoromethyl)-1H-pyrazole), TC-SP 14 (CAS# 1257093-40-5), SEW 2871 (CAS# 256414-75-2), RP 001 hydrochloride (CAS# 1306761-53-4), CYM 5442 hydrochloride (CAS# 1094042-01-9), CS 2100 (CAS# 913827-99-3), CYM 50260, CYM 50308, CYM 5520, CYM 5541, RP 001 hydrochloride, TC-G 1006, A 971432, TC-SP 14 and/or those agonists described in U.S. Patent Application Publication Nos. 2011/0124605 and 2017/0020826; U.S. Pat. Nos. 7,842,685; 7,064,217; 7,208,502; 7,241,790; and 7,638,637; Shurer et al. (2008) ACS Chem. Biol., 3:486-498); Sammani et al. (2011) Am. J. Respir. Cell Mol. Biol., 45:1022-7; and "MLSCN Probe Summary: S1P3 Agonist" by The Scripps Research Institute Molecular Screening Center (available at mli(dot)nih(dot)gov/mli/?dl_id=721), each possibility represents a separate embodiments of the present invention.

According to specific embodiments, the agent capable of up-regulating activity of S1P receptor is fingolimod (FTY720), siponimod, TC-G 1006 and/or A 971432.

According to specific embodiments, the agent capable of up-regulating activity of SIP receptor is selected from the group consisting of fingolimod, siponimod and/or A 971432.

According to specific embodiments, the agent capable of up-regulating activity of SIP receptor is selected from the group consisting of fingolimod (FTY720), TC-G 1006 and A 971432.

According to specific embodiments, the agent capable of up-regulating activity of SIP receptor is fingolimod (FTY720) and/or siponimod.

According to a specific embodiment, the agent capable of up-regulating activity of SIP receptor is fingolimod (FTY720).

According to specific embodiments, the agent capable of up-regulating activity of LXR is selected from the group consisting of β-sitosterol, campesterol, stigmasterol, fucosterol and brassicasterol, cholic acid (CA) and 24,25 EC.

According to a specific embodiment, the agent capable of up-regulating activity of LXR is β-sitosterol.

### Up-regulating at the nucleic acid level

The up-regulating agent can also be a molecule which is capable of increasing the transcription and/or translation of an endogenous DNA or mRNA encoding the target protein.

Another up-regulating agent may be an exogenous polynucleotide (DNA or RNA) sequence designed and constructed to express at least a functional portion of the target protein. The coding sequences information for the targets described herein is available from several databases including the GenBank database available through www4(dot)ncbi(dot)nlm (dot)nih(dot)gov/, and is further described hereinabove.

To express an exogenous protein in mammalian cells, a polynucleotide sequence encoding a specific protein or a homologue thereof which exhibit the desired activity is preferably ligated into a nucleic acid construct suitable for mammalian cell expression. Such a nucleic acid construct includes a promoter sequence for directing transcription of the polynucleotide sequence in the cell in a constitutive [e.g. cytomegalovirus (CMV) and Rous sarcoma virus (RSV)] or inducible (e.g. the tetracycline-inducible promoter) manner. According to specific embodiments, the promoter utilized by the nucleic acid construct of some embodiments of the invention is active in a specific cell population.

The nucleic acid construct (also referred to herein as an "expression vector") of some embodiments of the invention includes additional sequences which render this vector suitable for replication and integration in prokaryotes, eukaryotes, or preferably both (e.g., shuttle vectors). In addition, a typical cloning vectors may also contain a transcription and translation initiation sequence, transcription and translation terminator and a polyadenylation signal. By way of example, such constructs will typically include a 5' LTR, a tRNA binding site, a packaging signal, an origin of second-strand DNA synthesis, and a 3' LTR or a portion thereof. The construct may also include an enhancer element which can stimulate transcription up to 1,000 fold from linked homologous or heterologous promoters. The vector may or may not include a eukaryotic replicon.

The nucleic acid construct of some embodiments of the invention can also include a signal sequence for secretion of the peptide from a host cell in which it is placed. Preferably the signal sequence for this purpose is a mammalian signal sequence or the signal sequence of the polypeptide variants of some embodiments of the invention.

Polyadenylation sequences can also be added to the expression vector in order to increase the efficiency of mRNA translation. Two distinct sequence elements are required for accurate and efficient polyadenylation: GU or U rich sequences located downstream from the polyadenylation site and a highly conserved sequence of six nucleotides, AAUAAA, located 11-30 nucleotides upstream. Termination and polyadenylation signals that are suitable for some embodiments of the invention include those derived from SV40.

The expression vector of some embodiments of the invention can further include additional polynucleotide sequences that allow, for example, the translation of several proteins from a single mRNA such as an internal ribosome entry site (IRES) and sequences for genomic integration of the promoter-chimeric polypeptide.

Other than containing the necessary elements for the transcription and translation of the inserted coding sequence, the expression construct of some embodiments of the invention can also include sequences engineered to enhance stability, production, or yield of the expressed peptide.

It will be appreciated that the individual elements comprised in the expression vector can be arranged in a variety of configurations.

The type of vector used by some embodiments of the invention will depend on the cell type transformed. The ability to select suitable vectors according to the cell type transformed is well within the capabilities of the ordinary skilled artisan and as such no general description of selection consideration is provided herein.

Recombinant viral vectors are useful for *in vivo* expression of a protein since they offer advantages such as lateral infection and targeting specificity. Viral vectors can also be produced that are unable to spread laterally.

Various methods can be used to introduce the expression vector of some embodiments of the invention into cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986]. Currently preferred *in vivo* nucleic acid transfer techniques include transfection with viral or non-viral constructs, such as adenovirus, lentivirus, Herpes simplex I virus, or adeno-associated virus (AAV) and lipid-based systems. Useful lipids for lipid-mediated transfer of the gene are, for example, DOTMA, DOPE, and DC-Chol [Tonkinson et al., Cancer Investigation, 14(1): 54-65 (1996)]. The most preferred constructs for use in gene therapy are viruses, most preferably adenoviruses, AAV, lentiviruses, or retroviruses. Other vectors can be used that are non-viral, such as cationic lipids, polylysine, and dendrimers.

According to specific embodiments, the agent down-regulates activity and/or expression of the target expression product.

Down-regulation activity and/or expression can be can be effected at the protein level (e.g., antibodies, small molecules, inhibitory peptides, enzymes that cleave the polypeptide, aptamers and the like) but may also be effected at the genomic (e.g. homologous recombination and site specific endonucleases) and/or the transcript level using a variety of molecules which interfere with transcription and/or translation (e.g., RNA silencing agents) of a target expression product described herein.

Down-regulation of expression may be either transient or permanent.

According to specific embodiments, down-regulating expression refers to the absence of mRNA and/or protein, as detected by RT-PCR or Western blot, respectively.

According to other specific embodiments down-regulating expression refers to a decrease in the level of mRNA and/or protein, as detected by RT-PCR or Western blot, respectively. The reduction may be by at least a 10 %, at least 20 %, at least 30 %, at least 40 %, at least 50 %, at least 60 %, at least 70 %, at least 80 %, at least 90 %, at least 95 % or at least 99 % reduction.

Non-limiting examples of down-regulating agents are described in details hereinbelow.

### Down-regulating at the polypeptide level

According to specific embodiments, the down-regulating agent is an antibody. A detailed description on antibodies that can be used according to specific embodiments of the present invention is provided hereinabove.

Another down-regulating agent which can be used along with some embodiments of the invention is an aptamer. As used herein, the term "aptamer" refers to double stranded or single stranded RNA molecule that binds to specific molecular target, such as a protein. Various methods are known in the art which can be used to design protein specific aptamers. The skilled artisan can employ SELEX (Systematic Evolution of Ligands by Exponential Enrichment) for efficient selection as described in Stoltenburg R, Reinemann C, and Strehlitz B (Biomolecular engineering (2007) 24(4):381-403).

Another down-regulating agent would be any molecule which interferes with the target protein activity (e.g., catalytic or interaction) by binding the target protein or intermediate thereof and/or cleaving the target protein. Such molecules can be a small molecule, antagonists, or inhibitory peptide.

Another down-regulating agent which can be used along with some embodiments of the invention is a molecule which prevents target activation or substrate binding.

According to a specific embodiment, the down-regulating agent is a small molecule.

According to a specific embodiment, the down-regulating agent is a peptide molecule.

It will be appreciated that a non-functional analogue of at least a catalytic or binding portion of the target can be also used as a down-regulating agent.

### Down-regulating at the nucleic acid level

Down-regulation at the nucleic acid level is typically effected using a nucleic acid agent, having a nucleic acid backbone, DNA, RNA, mimetics thereof or a combination of same. The nucleic acid agent may be encoded from a DNA molecule or provided to the cell *per se.*

Thus, down-regulation can be achieved by RNA silencing. As used herein, the phrase "RNA silencing" refers to a group of regulatory mechanisms [e.g. RNA interference (RNAi), transcriptional gene silencing (TGS), post-transcriptional gene silencing (PTGS), quelling, co-suppression, and translational repression] mediated by RNA molecules which result in the inhibition or "silencing" of the expression of a corresponding protein-coding gene. RNA silencing has been observed in many types of organisms, including plants, animals, and fungi.

As used herein, the term "RNA silencing agent" refers to an RNA which is capable of specifically inhibiting or "silencing" the expression of a target gene. In certain embodiments, the RNA silencing agent is capable of preventing complete processing (e.g., the full translation and/or expression) of an mRNA molecule through a post-transcriptional silencing mechanism. RNA silencing agents include non-coding RNA molecules, for example RNA duplexes comprising paired strands, as well as precursor RNAs from which such small non-coding RNAs can be generated. Exemplary RNA silencing agents include dsRNAs such as siRNAs, miRNAs and shRNAs.

In one embodiment, the RNA silencing agent is capable of inducing RNA interference.

In another embodiment, the RNA silencing agent is capable of mediating translational repression.

According to an embodiment of the invention, the RNA silencing agent is specific to the target RNA (e.g., importin α5, MeCP2) and does not cross inhibit or silence other targets or a splice variant which exhibits 99% or less global homology to the target gene, e.g., less than 98%, 97%, 96%, 95%, 94%, 93%, 92%, 91%, 90%, 89%, 88%, 87%, 86%, 85%, 84%, 83%, 82%, 81% global homology to the target gene; as determined by PCR, Western blot, Immunohistochemistry and/or flow cytometry.

RNA interference refers to the process of sequence-specific post-transcriptional gene silencing in animals mediated by short interfering RNAs (siRNAs).

Following is a detailed description on RNA silencing agents that can be used according to specific embodiments of the present invention.

*DsRNA, siRNA and shRNA* - The presence of long dsRNAs in cells stimulates the activity of a ribonuclease III enzyme referred to as dicer. Dicer is involved in the processing of the dsRNA into short pieces of dsRNA known as short interfering RNAs (siRNAs). Short interfering RNAs derived from dicer activity are typically about 21 to about 23 nucleotides in length and comprise about 19 base pair duplexes. The RNAi response also features an endonuclease complex, commonly referred to as an RNA-induced silencing complex (RISC), which mediates cleavage of single-stranded RNA having sequence complementary to the antisense strand of the siRNA duplex. Cleavage of the target RNA takes place in the middle of the region complementary to the antisense strand of the siRNA duplex.

Accordingly, some embodiments of the invention contemplate use of dsRNA to down-regulate protein expression from mRNA.

According to one embodiment dsRNA longer than 30 bp are used. Various studies demonstrate that long dsRNAs can be used to silence gene expression without inducing the stress response or causing significant off-target effects - see for example [Strat et al., Nucleic Acids Research, 2006, Vol. 34, No. 13 3803-3810; Bhargava A et al. Brain Res. Protoc. 2004;13:115-125; Diallo M., et al., Oligonucleotides. 2003;13:381-392; Paddison P.J., et al., Proc. Natl Acad. Sci. USA. 2002;99:1443-1448; Tran N., et al., FEBS Lett. 2004;573:127-134].

According to some embodiments of the invention, dsRNA is provided in cells where the interferon pathway is not activated, see for example Billy et al., PNAS 2001, Vol 98, pages 14428-14433; and Diallo et al, Oligonucleotides, October 1, 2003, 13(5): 381-392. doi:10.1089/154545703322617069.

According to an embodiment of the invention, the long dsRNA are specifically designed not to induce the interferon and PKR pathways for down-regulating gene expression. For example, Shinagwa and Ishii [Genes & Dev. 17 (11): 1340-1345, 2003] have developed a vector, named pDECAP, to express long double-strand RNA from an RNA polymerase II (Pol II) promoter. Because the transcripts from pDECAP lack both the 5'-cap structure and the 3'-poly(A) tail that facilitate ds-RNA export to the cytoplasm, long ds-RNA from pDECAP does not induce the interferon response.

Another method of evading the interferon and PKR pathways in mammalian systems is by introduction of small inhibitory RNAs (siRNAs) either via transfection or endogenous expression.

The term "siRNA" refers to small inhibitory RNA duplexes (generally between 18-30 base pairs) that induce the RNA interference (RNAi) pathway. Typically, siRNAs are chemically synthesized as 21mers with a central 19 bp duplex region and symmetric 2-base 3'-overhangs on the termini, although it has been recently described that chemically synthesized RNA duplexes of 25-30 base length can have as much as a 100-fold increase in potency compared with 21mers at the same location. The observed increased potency obtained using longer RNAs in triggering RNAi is suggested to result from providing Dicer with a substrate (27mer) instead of a product (21mer) and that this improves the rate or efficiency of entry of the siRNA duplex into RISC.

It has been found that position of the 3'-overhang influences potency of an siRNA and asymmetric duplexes having a 3'-overhang on the antisense strand are generally more potent than those with the 3'-overhang on the sense strand (Rose et al., 2005). This can be attributed to asymmetrical strand loading into RISC, as the opposite efficacy patterns are observed when targeting the antisense transcript.

The strands of a double-stranded interfering RNA (e.g., an siRNA) may be connected to form a hairpin or stem-loop structure (e.g., an shRNA). Thus, as mentioned, the RNA silencing agent of some embodiments of the invention may also be a short hairpin RNA (shRNA).

The term "shRNA", as used herein, refers to an RNA agent having a stem-loop structure, comprising a first and second region of complementary sequence, the degree of complementarity and orientation of the regions being sufficient such that base pairing occurs between the regions, the first and second regions being joined by a loop region, the loop resulting from a lack of base pairing between nucleotides (or nucleotide analogs) within the loop region. The number of nucleotides in the loop is a number between and including 3 to 23, or 5 to 15, or 7 to 13, or 4 to 9, or 9 to 11. Some of the nucleotides in the loop can be involved in base-pair interactions with other nucleotides in the loop. Examples of oligonucleotide sequences that can be used to form the loop include 5'-CAAGAGA-3' and 5'-UUACAA-3' (International Patent Application Nos. WO2013126963 and WO2014107763). It will be recognized by one of skill in the art that the resulting single chain oligonucleotide forms a stem-loop or hairpin structure comprising a double-stranded region capable of interacting with the RNAi machinery.

Synthesis of RNA silencing agents suitable for use with some embodiments of the invention can be effected as follows. First, the mRNA sequence is scanned downstream of the AUG start codon for AA dinucleotide sequences. Occurrence of each AA and the 3' adjacent 19 nucleotides is recorded as potential siRNA target sites. Preferably, siRNA target sites are selected from the open reading frame, as untranslated regions (UTRs) are richer in regulatory protein binding sites. UTR-binding proteins and/or translation initiation complexes may interfere with binding of the siRNA endonuclease complex [Tuschl ChemBiochem. 2:239-245]. It will be appreciated though, that siRNAs directed at untranslated regions may also be effective, as demonstrated for GAPDH wherein siRNA directed at the 5' UTR mediated about 90 % decrease in cellular GAPDH mRNA and completely abolished protein level (www(dot)ambion(dot)com/techlib/tn/91/912(dot)html).

Second, potential target sites are compared to an appropriate genomic database (e.g., human, mouse, rat etc.) using any sequence alignment software, such as the BLAST software available from the NCBI server (www(dot)ncbi(dot)nlm(dot)nih(dot)gov/BLAST/). Putative target sites which exhibit significant homology to other coding sequences are filtered out.

Qualifying target sequences are selected as template for siRNA synthesis. Preferred sequences are those including low G/C content as these have proven to be more effective in mediating gene silencing as compared to those with G/C content higher than 55 %. Several target sites are preferably selected along the length of the target gene for evaluation. For better evaluation of the selected siRNAs, a negative control is preferably used in conjunction. Negative control siRNA preferably include the same nucleotide composition as the siRNAs but lack significant homology to the genome. Thus, a scrambled nucleotide sequence of the siRNA is preferably used, provided it does not display any significant homology to any other gene.

It will be appreciated that, and as mentioned hereinabove, the RNA silencing agent of some embodiments of the invention need not be limited to those molecules containing only RNA, but further encompasses chemically-modified nucleotides and non-nucleotides.

*miRNA and miRNA mimics* - According to another embodiment the RNA silencing agent may be a miRNA.

The term "microRNA", "miRNA", and "miR" are synonymous and refer to a collection of non-coding single-stranded RNA molecules of about 19-28 nucleotides in length, which regulate gene expression. miRNAs are found in a wide range of organisms (viruses.fwdarw.humans) and have been shown to play a role in development, homeostasis, and disease etiology.

Below is a brief description of the mechanism of miRNA activity.

Genes coding for miRNAs are transcribed leading to production of an miRNA precursor known as the pri-miRNA. The pri-miRNA is typically part of a polycistronic RNA comprising multiple pri-miRNAs. The pri-miRNA may form a hairpin with a stem and loop. The stem may comprise mismatched bases.

The hairpin structure of the pri-miRNA is recognized by Drosha, which is an RNase III endonuclease. Drosha typically recognizes terminal loops in the pri-miRNA and cleaves approximately two helical turns into the stem to produce a 60-70 nucleotide precursor known as the pre-miRNA. Drosha cleaves the pri-miRNA with a staggered cut typical of RNase III endonucleases yielding a pre-miRNA stem loop with a 5' phosphate and ∼2 nucleotide 3' overhang. It is estimated that approximately one helical turn of stem (∼10 nucleotides) extending beyond the Drosha cleavage site is essential for efficient processing. The pre-miRNA is then actively transported from the nucleus to the cytoplasm by Ran-GTP and the export receptor Ex-portin-5.

The double-stranded stem of the pre-miRNA is then recognized by Dicer, which is also an RNase III endonuclease. Dicer may also recognize the 5' phosphate and 3' overhang at the base of the stem loop. Dicer then cleaves off the terminal loop two helical turns away from the base of the stem loop leaving an additional 5' phosphate and ∼2 nucleotide 3' overhang. The resulting siRNA-like duplex, which may comprise mismatches, comprises the mature miRNA and a similar-sized fragment known as the miRNA*. The miRNA and miRNA* may be derived from opposing arms of the pri-miRNA and pre-miRNA. miRNA* sequences may be found in libraries of cloned miRNAs but typically at lower frequency than the miRNAs.

Although initially present as a double-stranded species with miRNA*, the miRNA eventually becomes incorporated as a single-stranded RNA into a ribonucleoprotein complex known as the RNA-induced silencing complex (RISC). Various proteins can form the RISC, which can lead to variability in specificity for miRNA/miRNA* duplexes, binding site of the target gene, activity of miRNA (repress or activate), and which strand of the miRNA/miRNA* duplex is loaded in to the RISC.

When the miRNA strand of the miRNA:miRNA* duplex is loaded into the RISC, the miRNA* is removed and degraded. The strand of the miRNA:miRNA* duplex that is loaded into the RISC is the strand whose 5' end is less tightly paired. In cases where both ends of the miRNA:miRNA* have roughly equivalent 5' pairing, both miRNA and miRNA* may have gene silencing activity.

The RISC identifies target nucleic acids based on high levels of complementarity between the miRNA and the mRNA, especially by nucleotides 2-7 of the miRNA.

A number of studies have looked at the base-pairing requirement between miRNA and its mRNA target for achieving efficient inhibition of translation (reviewed by Bartel 2004, Cell 116-281). In mammalian cells, the first 8 nucleotides of the miRNA may be important (Doench & Sharp 2004 GenesDev 2004-504). However, other parts of the microRNA may also participate in mRNA binding. Moreover, sufficient base pairing at the 3' can compensate for insufficient pairing at the 5' (Brennecke et al, 2005 PLoS 3-e85). Computation studies, analyzing miRNA binding on whole genomes have suggested a specific role for bases 2-7 at the 5' of the miRNA in target binding but the role of the first nucleotide, found usually to be "A" was also recognized (Lewis et at 2005 Cell 120-15). Similarly, nucleotides 1-7 or 2-8 were used to identify and validate targets by Krek et al. (2005, Nat Genet 37-495).

The target sites in the mRNA may be in the 5' UTR, the 3' UTR or in the coding region. Interestingly, multiple miRNAs may regulate the same mRNA target by recognizing the same or multiple sites. The presence of multiple miRNA binding sites in most genetically identified targets may indicate that the cooperative action of multiple RISCs provides the most efficient translational inhibition.

miRNAs may direct the RISC to down-regulate gene expression by either of two mechanisms: mRNA cleavage or translational repression. The miRNA may specify cleavage of the mRNA if the mRNA has a certain degree of complementarity to the miRNA. When a miRNA guides cleavage, the cut is typically between the nucleotides pairing to residues 10 and 11 of the miRNA. Alternatively, the miRNA may repress translation if the miRNA does not have the requisite degree of complementarity to the miRNA. Translational repression may be more prevalent in animals since animals may have a lower degree of complementarity between the miRNA and binding site.

It should be noted that there may be variability in the 5' and 3' ends of any pair of miRNA and miRNA*. This variability may be due to variability in the enzymatic processing of Drosha and Dicer with respect to the site of cleavage. Variability at the 5' and 3' ends of miRNA and miRNA* may also be due to mismatches in the stem structures of the pri-miRNA and pre-miRNA. The mismatches of the stem strands may lead to a population of different hairpin structures. Variability in the stem structures may also lead to variability in the products of cleavage by Drosha and Dicer.

The term "microRNA mimic" or "miRNA mimic" refers to synthetic non-coding RNAs that are capable of entering the RNAi pathway and regulating gene expression. miRNA mimics imitate the function of endogenous miRNAs and can be designed as mature, double stranded molecules or mimic precursors (e.g., or pre-miRNAs). miRNA mimics can be comprised of modified or unmodified RNA, DNA, RNA-DNA hybrids, or alternative nucleic acid chemistries (e.g., LNAs or 2'-O,4'-C-ethylene-bridged nucleic acids (ENA)). For mature, double stranded miRNA mimics, the length of the duplex region can vary between 13-33, 18-24 or 21-23 nucleotides. The miRNA may also comprise a total of at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides. The sequence of the miRNA may be the first 13-33 nucleotides of the pre-miRNA. The sequence of the miRNA may also be the last 13-33 nucleotides of the pre-miRNA.

Preparation of miRNAs mimics can be effected by any method known in the art such as chemical synthesis or recombinant methods.

It will be appreciated from the description provided herein above that contacting cells with a miRNA may be effected by transfecting the cells with e.g. the mature double stranded miRNA, the pre-miRNA or the pri-miRNA.

The pre-miRNA sequence may comprise from 45-90, 60-80 or 60-70 nucleotides.

The pri-miRNA sequence may comprise from 45-30,000, 50-25,000, 100-20,000, 1,000-1,500 or 80-100 nucleotides.

*Antisense* - Antisense is a single stranded RNA designed to prevent or inhibit expression of a gene by specifically hybridizing to its mRNA. Down-regulation can be effected using an antisense polynucleotide capable of specifically hybridizing with an mRNA transcript encoding the target (e.g. importin α5, MeCP2).

Design of antisense molecules which can be used to efficiently down-regulate a target must be effected while considering two aspects important to the antisense approach. The first aspect is delivery of the oligonucleotide into the cytoplasm of the appropriate cells, while the second aspect is design of an oligonucleotide which specifically binds the designated mRNA within cells in a way which inhibits translation thereof.

The prior art teaches of a number of delivery strategies which can be used to efficiently deliver oligonucleotides into a wide variety of cell types [see, for example, Jääskeläinen et al. Cell Mol Biol Lett. (2002) 7(2):236-7; Gait, Cell Mol Life Sci. (2003) 60(5):844-53; Martino et al. J Biomed Biotechnol. (2009) 2009:410260; Grijalvo et al. Expert Opin Ther Pat. (2014) 24(7):801-19; Falzarano et al, Nucleic Acid Ther. (2014) 24(1):87-100; Shilakari et al. Biomed Res Int. (2014) 2014: 526391; Prakash et al. Nucleic Acids Res. (2014) 42(13):8796-807 and Asseline et al. J Gene Med. (2014) 16(7-8):157-65]

In addition, algorithms for identifying those sequences with the highest predicted binding affinity for their target mRNA based on a thermodynamic cycle that accounts for the energetics of structural alterations in both the target mRNA and the oligonucleotide are also available [see, for example, Walton et al. Biotechnol Bioeng 65: 1-9 (1999)]. Such algorithms have been successfully used to implement an antisense approach in cells.

In addition, several approaches for designing and predicting efficiency of specific oligonucleotides using an in vitro system were also published (Matveeva et al., Nature Biotechnology 16: 1374 - 1375 (1998)].

Thus, the generation of highly accurate antisense design algorithms and a wide variety of oligonucleotide delivery systems, enable an ordinarily skilled artisan to design and implement antisense approaches suitable for down-regulating expression of known sequences without having to resort to undue trial and error experimentation.

Nucleic acid agents can also operate at the DNA level as summarized infra.

Down-regulation can also be achieved by inactivating the gene (e.g., KPNA1, MECP2) via introducing targeted mutations involving loss-of function alterations (e.g. point mutations, deletions and insertions) in the gene structure.

As used herein, the phrase "loss-of-function alterations" refers to any mutation in the DNA sequence of a gene which results in down-regulation of the expression level and/or activity of the expressed product, i.e., the mRNA transcript and/or the translated protein. Non-limiting examples of such loss-of-function alterations include a missense mutation, *i.e.,* a mutation which changes an amino acid residue in the protein with another amino acid residue and thereby abolishes the enzymatic activity of the protein; a nonsense mutation, *i.e.,* a mutation which introduces a stop codon in a protein, e.g., an early stop codon which results in a shorter protein devoid of the enzymatic activity; a frame-shift mutation, *i.e.,* a mutation, usually, deletion or insertion of nucleic acid(s) which changes the reading frame of the protein, and may result in an early termination by introducing a stop codon into a reading frame (e.g., a truncated protein, devoid of the enzymatic activity), or in a longer amino acid sequence (e.g., a readthrough protein) which affects the secondary or tertiary structure of the protein and results in a non-functional protein, devoid of the enzymatic activity of the non-mutated polypeptide; a readthrough mutation due to a frame-shift mutation or a modified stop codon mutation (*i.e*., when the stop codon is mutated into an amino acid codon), with an abolished enzymatic activity; a promoter mutation, *i.e.,* a mutation in a promoter sequence, usually 5' to the transcription start site of a gene, which results in down-regulation of a specific gene product; a regulatory mutation, *i.e.,* a mutation in a region upstream or downstream, or within a gene, which affects the expression of the gene product; a deletion mutation, *i.e.,* a mutation which deletes coding nucleic acids in a gene sequence and which may result in a frame-shift mutation or an in-frame mutation (within the coding sequence, deletion of one or more amino acid codons); an insertion mutation, *i.e.,* a mutation which inserts coding or non-coding nucleic acids into a gene sequence, and which may result in a frame-shift mutation or an in-frame insertion of one or more amino acid codons; an inversion, *i.e.,* a mutation which results in an inverted coding or non-coding sequence; a splice mutation *i.e.,* a mutation which results in abnormal splicing or poor splicing; and a duplication mutation, *i.e*., a mutation which results in a duplicated coding or non-coding sequence, which can be in-frame or can cause a frame-shift.

According to specific embodiments loss-of-function alteration of a gene may comprise at least one allele of the gene.

The term "allele" as used herein, refers to any of one or more alternative forms of a gene locus, all of which alleles relate to a trait or characteristic. In a diploid cell or organism, the two alleles of a given gene occupy corresponding loci on a pair of homologous chromosomes.

According to other specific embodiments loss-of-function alteration of a gene comprises both alleles of the gene. In such instances the e.g. KPNA1, MECP2 may be in a homozygous form or in a heterozygous form.

Methods of introducing nucleic acid alterations to a gene of interest are well known in the art [see for example Menke D. Genesis (2013) 51: - 618; Capecchi, Science (1989) 244:1288-1292; Santiago et al. Proc Natl Acad Sci USA (2008) 105:5809-5814; International Patent Application Nos. WO 2014085593, WO 2009071334 and WO 2011146121; US Patent Nos. 8771945, 8586526, 6774279 and UP Patent Application Publication Nos. 20030232410, 20050026157, US20060014264; the contents of which are incorporated by reference in their entireties] and include targeted homologous recombination, site specific recombinases, PB transposases and genome editing by engineered nucleases. Agents for introducing nucleic acid alterations to a gene of interest can be designed publically available sources or obtained commercially from Transposagen, Addgene and Sangamo Biosciences.

Following is a description of various exemplary methods used to introduce nucleic acid alterations to a gene of interest and agents for implementing same that can be used according to specific embodiments of the present invention.

Genome Editing using engineered endonucleases - this approach refers to a reverse genetics method using artificially engineered nucleases to cut and create specific double-stranded breaks at a desired location(s) in the genome, which are then repaired by cellular endogenous processes such as, homology directed repair (HDR) and non-homologous end-joining (NHEJ). NHEJ directly joins the DNA ends in a double-stranded break, while HDR utilizes a homologous sequence as a template for regenerating the missing DNA sequence at the break point. In order to introduce specific nucleotide modifications to the genomic DNA, a DNA repair template containing the desired sequence must be present during HDR. Genome editing cannot be performed using traditional restriction endonucleases since most restriction enzymes recognize a few base pairs on the DNA as their target and the probability is very high that the recognized base pair combination will be found in many locations across the genome resulting in multiple cuts not limited to a desired location. To overcome this challenge and create site-specific single- or double-stranded breaks, several distinct classes of nucleases have been discovered and bioengineered to date. These include the meganucleases, Zinc finger nucleases (ZFNs), transcription-activator like effector nucleases (TALENs) and CRISPR/Cas system.

*Meganucleases* - Meganucleases are commonly grouped into four families: the LAGLIDADG family, the GIY-YIG family, the His-Cys box family and the HNH family. These families are characterized by structural motifs, which affect catalytic activity and recognition sequence. For instance, members of the LAGLIDADG family are characterized by having either one or two copies of the conserved LAGLIDADG motif. The four families of meganucleases are widely separated from one another with respect to conserved structural elements and, consequently, DNA recognition sequence specificity and catalytic activity. Meganucleases are found commonly in microbial species and have the unique property of having very long recognition sequences (>14bp) thus making them naturally very specific for cutting at a desired location. This can be exploited to make site-specific double-stranded breaks in genome editing. One of skill in the art can use these naturally occurring meganucleases, however the number of such naturally occurring meganucleases is limited. To overcome this challenge, mutagenesis and high throughput screening methods have been used to create meganuclease variants that recognize unique sequences. For example, various meganucleases have been fused to create hybrid enzymes that recognize a new sequence. Alternatively, DNA interacting amino acids of the meganuclease can be altered to design sequence specific meganucleases (see e.g., US Patent 8,021,867). Meganucleases can be designed using the methods described in e.g., Certo, MT et al. Nature Methods (2012) 9:073-975; U.S. Patent Nos. 8,304,222; 8,021,867; 8, 119,381; 8, 124,369; 8, 129,134; 8,133,697; 8,143,015; 8,143,016; 8, 148,098; or 8, 163,514, the contents of each are incorporated herein by reference in their entirety. Alternatively, meganucleases with site specific cutting characteristics can be obtained using commercially available technologies e.g., Precision Biosciences' Directed Nuclease Editor™ genome editing technology.

*ZFNs and TALENs* - Two distinct classes of engineered nucleases, zinc-finger nucleases (ZFNs) and transcription activator-like effector nucleases (TALENs), have both proven to be effective at producing targeted double-stranded breaks (Christian *et al.*, 2010; Kim *et al.*, 1996; Li *et al.*, 2011; Mahfouz *et al.*, 2011; Miller *et al.*, 2010).

Basically, ZFNs and TALENs restriction endonuclease technology utilizes a non-specific DNA cutting enzyme which is linked to a specific DNA binding domain (either a series of zinc finger domains or TALE repeats, respectively). Typically a restriction enzyme whose DNA recognition site and cleaving site are separate from each other is selected. The cleaving portion is separated and then linked to a DNA binding domain, thereby yielding an endonuclease with very high specificity for a desired sequence. An exemplary restriction enzyme with such properties is Fokl. Additionally Fokl has the advantage of requiring dimerization to have nuclease activity and this means the specificity increases dramatically as each nuclease partner recognizes a unique DNA sequence. To enhance this effect, Fokl nucleases have been engineered that can only function as heterodimers and have increased catalytic activity. The heterodimer functioning nucleases avoid the possibility of unwanted homodimer activity and thus increase specificity of the double-stranded break.

Thus, for example to target a specific site, ZFNs and TALENs are constructed as nuclease pairs, with each member of the pair designed to bind adjacent sequences at the targeted site. Upon transient expression in cells, the nucleases bind to their target sites and the FokI domains heterodimerize to create a double-stranded break. Repair of these double-stranded breaks through the non-homologous end-joining (NHEJ) pathway most often results in small deletions or small sequence insertions. Since each repair made by NHEJ is unique, the use of a single nuclease pair can produce an allelic series with a range of different deletions at the target site. The deletions typically range anywhere from a few base pairs to a few hundred base pairs in length, but larger deletions have successfully been generated in cell culture by using two pairs of nucleases simultaneously (Carlson *et al.*, 2012; Lee *et al.*, 2010). In addition, when a fragment of DNA with homology to the targeted region is introduced in conjunction with the nuclease pair, the double-stranded break can be repaired via homology directed repair to generate specific modifications (Li *et al.*, 2011; Miller *et al.*, 2010; Urnov *et al.*, 2005).

Although the nuclease portions of both ZFNs and TALENs have similar properties, the difference between these engineered nucleases is in their DNA recognition peptide. ZFNs rely on Cys2- His2 zinc fingers and TALENs on TALEs. Both of these DNA recognizing peptide domains have the characteristic that they are naturally found in combinations in their proteins. Cys2-His2 Zinc fingers typically found in repeats that are 3 bp apart and are found in diverse combinations in a variety of nucleic acid interacting proteins. TALEs on the other hand are found in repeats with a one-to-one recognition ratio between the amino acids and the recognized nucleotide pairs. Because both zinc fingers and TALEs happen in repeated patterns, different combinations can be tried to create a wide variety of sequence specificities. Approaches for making site-specific zinc finger endonucleases include, e.g., modular assembly (where Zinc fingers correlated with a triplet sequence are attached in a row to cover the required sequence), OPEN (low-stringency selection of peptide domains vs. triplet nucleotides followed by high-stringency selections of peptide combination vs. the final target in bacterial systems), and bacterial one-hybrid screening of zinc finger libraries, among others. ZFNs can also be designed and obtained commercially from e.g., Sangamo Biosciences™ (Richmond, CA).

Method for designing and obtaining TALENs are described in e.g. Reyon et al. Nature Biotechnology 2012 May;30(5):460-5; Miller et al. Nat Biotechnol. (2011) 29: 143-148; Cermak et al. Nucleic Acids Research (2011) 39 (12): e82 and Zhang et al. Nature Biotechnology (2011) 29 (2): 149-53. A recently developed web-based program named Mojo Hand was introduced by Mayo Clinic for designing TAL and TALEN constructs for genome editing applications (can be accessed through www(dot)talendesign(dot)org). TALEN can also be designed and obtained commercially from e.g., Sangamo Biosciences™ (Richmond, CA).

*CRISPR-Cas system* - Many bacteria and archea contain endogenous RNA-based adaptive immune systems that can degrade nucleic acids of invading phages and plasmids. These systems consist of clustered regularly interspaced short palindromic repeat (CRISPR) genes that produce RNA components and CRISPR associated (Cas) genes that encode protein components. The CRISPR RNAs (crRNAs) contain short stretches of homology to specific viruses and plasmids and act as guides to direct Cas nucleases to degrade the complementary nucleic acids of the corresponding pathogen. Studies of the type II CRISPR/Cas system of *Streptococcus pyogenes* have shown that three components form an RNA/protein complex and together are sufficient for sequence-specific nuclease activity: the Cas9 nuclease, a crRNA containing 20 base pairs of homology to the target sequence, and a trans-activating crRNA (tracrRNA) (Jinek et al. Science (2012) 337: 816-821.). It was further demonstrated that a synthetic chimeric guide RNA (gRNA) composed of a fusion between crRNA and tracrRNA could direct Cas9 to cleave DNA targets that are complementary to the crRNA in vitro. It was also demonstrated that transient expression of Cas9 in conjunction with synthetic gRNAs can be used to produce targeted double-stranded brakes in a variety of different species (Cho *et al.*, 2013; Cong *et al.*, 2013; DiCarlo *et al.*, 2013; Hwang *et al.*, 2013a,b; Jinek *et al.*, 2013; Mali *et al.*, 2013).

The CRIPSR/Cas system for genome editing contains two distinct components: a gRNA and an endonuclease e.g. Cas9.

The gRNA is typically a 20 nucleotide sequence encoding a combination of the target homologous sequence (crRNA) and the endogenous bacterial RNA that links the crRNA to the Cas9 nuclease (tracrRNA) in a single chimeric transcript. The gRNA/Cas9 complex is recruited to the target sequence by the base-pairing between the gRNA sequence and the complement genomic DNA. For successful binding of Cas9, the genomic target sequence must also contain the correct Protospacer Adjacent Motif (PAM) sequence immediately following the target sequence. The binding of the gRNA/Cas9 complex localizes the Cas9 to the genomic target sequence so that the Cas9 can cut both strands of the DNA causing a double-strand break. Just as with ZFNs and TALENs, the double-stranded brakes produced by CRISPR/Cas can undergo homologous recombination or NHEJ.

The Cas9 nuclease has two functional domains: RuvC and HNH, each cutting a different DNA strand. When both of these domains are active, the Cas9 causes double strand breaks in the genomic DNA.

A significant advantage of CRISPR/Cas is that the high efficiency of this system coupled with the ability to easily create synthetic gRNAs enables multiple genes to be targeted simultaneously. In addition, the majority of cells carrying the mutation present biallelic mutations in the targeted genes.

However, apparent flexibility in the base-pairing interactions between the gRNA sequence and the genomic DNA target sequence allows imperfect matches to the target sequence to be cut by Cas9.

Modified versions of the Cas9 enzyme containing a single inactive catalytic domain, either RuvC- or HNH-, are called 'nickases'. With only one active nuclease domain, the Cas9 nickase cuts only one strand of the target DNA, creating a single-strand break or 'nick'. A single-strand break, or nick, is normally quickly repaired through the HDR pathway, using the intact complementary DNA strand as the template. However, two proximal, opposite strand nicks introduced by a Cas9 nickase are treated as a double-strand break, in what is often referred to as a 'double nick' CRISPR system. A double-nick can be repaired by either NHEJ or HDR depending on the desired effect on the gene target. Thus, if specificity and reduced off-target effects are crucial, using the Cas9 nickase to create a double-nick by designing two gRNAs with target sequences in close proximity and on opposite strands of the genomic DNA would decrease off-target effect as either gRNA alone will result in nicks that will not change the genomic DNA.

Modified versions of the Cas9 enzyme containing two inactive catalytic domains (dead Cas9, or dCas9) have no nuclease activity while still able to bind to DNA based on gRNA specificity. The dCas9 can be utilized as a platform for DNA transcriptional regulators to activate or repress gene expression by fusing the inactive enzyme to known regulatory domains. For example, the binding of dCas9 alone to a target sequence in genomic DNA can interfere with gene transcription.

There are a number of publically available tools available to help choose and/or design target sequences as well as lists of bioinformatically determined unique gRNAs for different genes in different species such as the Feng Zhang lab's Target Finder, the Michael Boutros lab's Target Finder (E-CRISP), the RGEN Tools: Cas-OFFinder, the CasFinder: Flexible algorithm for identifying specific Cas9 targets in genomes and the CRISPR Optimal Target Finder.

In order to use the CRISPR system, both gRNA and Cas9 should be expressed in a target cell. The insertion vector can contain both cassettes on a single plasmid or the cassettes are expressed from two separate plasmids. CRISPR plasmids are commercially available such as the px330 plasmid from Addgene.

"Hit and run" or "in-out" - involves a two-step recombination procedure. In the first step, an insertion-type vector containing a dual positive/negative selectable marker cassette is used to introduce the desired sequence alteration. The insertion vector contains a single continuous region of homology to the targeted locus and is modified to carry the mutation of interest. This targeting construct is linearized with a restriction enzyme at a one site within the region of homology, electroporated into the cells, and positive selection is performed to isolate homologous recombinants. These homologous recombinants contain a local duplication that is separated by intervening vector sequence, including the selection cassette. In the second step, targeted clones are subjected to negative selection to identify cells that have lost the selection cassette via intrachromosomal recombination between the duplicated sequences. The local recombination event removes the duplication and, depending on the site of recombination, the allele either retains the introduced mutation or reverts to wild type. The end result is the introduction of the desired modification without the retention of any exogenous sequences.

The "double-replacement" or "tag and exchange" strategy - involves a two-step selection procedure similar to the hit and run approach, but requires the use of two different targeting constructs. In the first step, a standard targeting vector with 3' and 5' homology arms is used to insert a dual positive/negative selectable cassette near the location where the mutation is to be introduced. After electroporation and positive selection, homologously targeted clones are identified. Next, a second targeting vector that contains a region of homology with the desired mutation is electroporated into targeted clones, and negative selection is applied to remove the selection cassette and introduce the mutation. The final allele contains the desired mutation while eliminating unwanted exogenous sequences.

Site-Specific Recombinases - The Cre recombinase derived from the PI bacteriophage and Flp recombinase derived from the yeast *Saccharomyces cerevisiae are* site-specific DNA recombinases each recognizing a unique 34 base pair DNA sequence (termed "Lox" and "FRT", respectively) and sequences that are flanked with either Lox sites or FRT sites can be readily removed via site-specific recombination upon expression of Cre or Flp recombinase, respectively. For example, the Lox sequence is composed of an asymmetric eight base pair spacer region flanked by 13 base pair inverted repeats. Cre recombines the 34 base pair lox DNA sequence by binding to the 13 base pair inverted repeats and catalyzing strand cleavage and religation within the spacer region. The staggered DNA cuts made by Cre in the spacer region are separated by 6 base pairs to give an overlap region that acts as a homology sensor to ensure that only recombination sites having the same overlap region recombine.

Basically, the site specific recombinase system offers means for the removal of selection cassettes after homologous recombination. This system also allows for the generation of conditional altered alleles that can be inactivated or activated in a temporal or tissue-specific manner. Of note, the Cre and Flp recombinases leave behind a Lox or FRT "scar" of 34 base pairs. The Lox or FRT sites that remain are typically left behind in an intron or 3' UTR of the modified locus, and current evidence suggests that these sites usually do not interfere significantly with gene function.

Thus, Cre/Lox and Flp/FRT recombination involves introduction of a targeting vector with 3' and 5' homology arms containing the mutation of interest, two Lox or FRT sequences and typically a selectable cassette placed between the two Lox or FRT sequences. Positive selection is applied and homologous recombinants that contain targeted mutation are identified. Transient expression of Cre or Flp in conjunction with negative selection results in the excision of the selection cassette and selects for cells where the cassette has been lost. The final targeted allele contains the Lox or FRT scar of exogenous sequences.

Transposases - As used herein, the term "transposase" refers to an enzyme that binds to the ends of a transposon and catalyzes the movement of the transposon to another part of the genome.

As used herein the term "transposon" refers to a mobile genetic element comprising a nucleotide sequence which can move around to different positions within the genome of a single cell. In the process the transposon can cause mutations and/or change the amount of a DNA in the genome of the cell.

A number of transposon systems that are able to also transpose in cells e.g. vertebrates have been isolated or designed, such as Sleeping Beauty [Izsvák and Ivics Molecular Therapy (2004) 9, 147-156], piggyBac [Wilson et al. Molecular Therapy (2007) 15, 139-145], Tol2 [Kawakami et al. PNAS (2000) 97 (21): 11403-11408] or Frog Prince [Miskey et al. Nucleic Acids Res. Dec 1, (2003) 31(23): 6873-6881]. Generally, DNA transposons translocate from one DNA site to another in a simple, cut-and-paste manner. Each of these elements has their own advantages, for example, Sleeping Beauty is particularly useful in region-specific mutagenesis, whereas Tol2 has the highest tendency to integrate into expressed genes. Hyperactive systems are available for Sleeping Beauty and piggyBac. Most importantly, these transposons have distinct target site preferences, and can therefore introduce sequence alterations in overlapping, but distinct sets of genes. Therefore, to achieve the best possible coverage of genes, the use of more than one element is particularly preferred. The basic mechanism is shared between the different transposases, therefore we will describe piggyBac (PB) as an example.

PB is a 2.5 kb insect transposon originally isolated from the cabbage looper moth, *Trichoplusia ni.* The PB transposon consists of asymmetric terminal repeat sequences that flank a transposase, PBase. PBase recognizes the terminal repeats and induces transposition via a "cut-and-paste" based mechanism, and preferentially transposes into the host genome at the tetranucleotide sequence TTAA. Upon insertion, the TTAA target site is duplicated such that the PB transposon is flanked by this tetranucleotide sequence. When mobilized, PB typically excises itself precisely to reestablish a single TTAA site, thereby restoring the host sequence to its pretransposon state. After excision, PB can transpose into a new location or be permanently lost from the genome.

Typically, the transposase system offers an alternative means for the removal of selection cassettes after homologous recombination quit similar to the use Cre/Lox or Flp/FRT. Thus, for example, the PB transposase system involves introduction of a targeting vector with 3' and 5' homology arms containing the mutation of interest, two PB terminal repeat sequences at the site of an endogenous TTAA sequence and a selection cassette placed between PB terminal repeat sequences. Positive selection is applied and homologous recombinants that contain targeted mutation are identified. Transient expression of PBase removes in conjunction with negative selection results in the excision of the selection cassette and selects for cells where the cassette has been lost. The final targeted allele contains the introduced mutation with no exogenous sequences.

For PB to be useful for the introduction of sequence alterations, there must be a native TTAA site in relatively close proximity to the location where a particular mutation is to be inserted.

Genome editing using recombinant adeno-associated virus (rAAV) platform - this genome-editing platform is based on rAAV vectors which enable insertion, deletion or substitution of DNA sequences in the genomes of live mammalian cells. The rAAV genome is a single-stranded deoxyribonucleic acid (ssDNA) molecule, either positive- or negative-sensed, which is about 4.7 kb long. These single-stranded DNA viral vectors have high transduction rates and have a unique property of stimulating endogenous homologous recombination in the absence of double-strand DNA breaks in the genome. One of skill in the art can design a rAAV vector to target a desired genomic locus and perform both gross and/or subtle endogenous gene alterations in a cell. rAAV genome editing has the advantage in that it targets a single allele and does not result in any off-target genomic alterations. rAAV genome editing technology is commercially available, for example, the rAAV GENESIS™ system from Horizon™ (Cambridge, UK).

It will be appreciated that the agent can be a mutagen that causes random mutations and the cells exhibiting down-regulation of the expression level and/or activity of the target may be selected.

The mutagens may be, but are not limited to, genetic, chemical or radiation agents. For example, the mutagen may be ionizing radiation, such as, but not limited to, ultraviolet light, gamma rays or alpha particles. Other mutagens may include, but not be limited to, base analogs, which can cause copying errors; deaminating agents, such as nitrous acid; intercalating agents, such as ethidium bromide; alkylating agents, such as bromouracil; transposons; natural and synthetic alkaloids; bromine and derivatives thereof; sodium azide; psoralen (for example, combined with ultraviolet radiation). The mutagen may be a chemical mutagen such as, but not limited to, ICR191, 1,2,7,8-diepoxy-octane (DEO), 5-azaC, N-methyl-N-nitrosoguanidine (MNNG) or ethyl methane sulfonate (EMS).

Methods for qualifying efficacy and detecting sequence alteration are well known in the art and include, but not limited to, DNA sequencing, electrophoresis, an enzyme-based mismatch detection assay and a hybridization assay such as PCR, RT-PCR, RNase protection, in-situ hybridization, primer extension, Southern blot, Northern Blot and dot blot analysis.

Sequence alterations in a specific gene can also be determined at the protein level using e.g. chromatography, electrophoretic methods, immunodetection assays such as ELISA and western blot analysis and immunohistochemistry.

In addition, one ordinarily skilled in the art can readily design a knock-in/knock-out construct including positive and/or negative selection markers for efficiently selecting transformed cells that underwent a homologous recombination event with the construct. Positive selection provides a means to enrich the population of clones that have taken up foreign DNA. Non-limiting examples of such positive markers include glutamine synthetase, dihydrofolate reductase (DHFR), markers that confer antibiotic resistance, such as neomycin, hygromycin, puromycin, and blasticidin S resistance cassettes. Negative selection markers are necessary to select against random integrations and/or elimination of a marker sequence (e.g. positive marker). Non-limiting examples of such negative markers include the herpes simplex-thymidine kinase (HSV-TK) which converts ganciclovir (GCV) into a cytotoxic nucleoside analog, hypoxanthine phosphoribosyltransferase (HPRT) and adenine phosphoribosytransferase (ARPT).

Agents which can be implemented in the present teachings can be identified according to the following aspect.

According to an aspect of the present invention, there is provided a method of identifying an agent that inhibits activity of importin α5, the method comprising:
(i) contacting a polypeptide sequence containing an NLS sequence of MeCP2 with a importin α5 polypeptide under conditions which allow binding of said importin α5 polypeptide to said polypeptide containing said NLS sequence;
(ii) determining an effect of a test agent on binding of said importin α5 polypeptide to said polypeptide containing said NLS sequence;
wherein a decrease in said binding as compared to same in the absence of said test agent indicates said test agent inhibits activity of importin α5.

According to specific embodiments, the contacting is effected *in-vitro* or *ex-vivo.*

According to other specific embodiments, the contacting is effected *in-vivo.*

As used herein, the term "binding" refers to the interaction of importin α5 polypeptide with an NLS sequence of MeCP2. The binding can be determined *per se* (e.g., binding affinity e.g., using plasmon resonance BIAcore assay) or by determining the effect of binding on expression of a reporter gene translationally fused to the polypeptide sequence containing an NLS sequence of MeCP2. Alternatively or additionally, the binding can be evaluated by determining the effect of binding on translocation of the polypeptide sequence containing an NLS sequence of MeCP2 to the nucleus (typically, as disclosed herein, binding of importin α5 to MeCP2 induces translocation into the nucleus) and/or the effect of binding on Sphkl level of expression (typically, as disclosed herein, binding of importin α5 to MeCP2 reduces Sphkl expression).

According to another aspect of the present invention, there is provided a method of identifying an agent that inhibits activity of importin α5, the method comprising:
(i) contacting a polypeptide sequence containing an NLS sequence of MeCP2 with a importin α5 polypeptide under conditions which allow binding of said importin α5 polypeptide to said polypeptide containing said NLS sequence;
(ii) determining an effect of a test agent on translocation of said polypeptide sequence containing an NLS sequence of MeCP2 to the nucleus;
wherein a decrease in said translocation as compared to same in the absence of said test agent indicates said test agent inhibits activity of importin α5.

According to another aspect of the present invention, there is provided a method of identifying an agent that inhibits activity of importin α5, the method comprising:
(i) contacting a polypeptide sequence containing an NLS sequence of MeCP2 with a importin α5 polypeptide under conditions which allow binding of said importin α5 polypeptide to said polypeptide containing said NLS sequence;
(ii) determining an effect of a test agent on expression of sphingosine kinase 1 (Sphkl);
wherein an increase in said expression as compared to same in the absence of said test agent indicates said test agent inhibits activity of importin α5.

According to specific embodiments, determining is effected *in-vitro* or *ex-vivo.*

According to specific embodiments, the decrease is of at least 5 %, at least 10 %, 20 %, 30 %, 40 % or even higher say, 50 %, 60 %, 70 %, 80 %, 90 %, 99 % or even 100 % as compared to same in the absence of the test agent. According to specific embodiments the decrease is at least 1.5 fold, at least 2 fold, at least 3 fold, at least 5 fold, at least 10 fold, or at least 20 fold as compared to same in the absence of the test agent.

According to specific embodiments, the increase is of at least 5 %, at least 10 %, 20 %, 30 %, 40 % or even higher say, 50 %, 60 %, 70 %, 80 %, 90 %, 99 % or even 100 % as compared to same in the absence of the test agent. According to specific embodiments the increase is at least 1.5 fold, at least 2 fold, at least 3 fold, at least 5 fold, at least 10 fold, or at least 20 fold as compared to same in the absence of the test agent.

According to specific embodiments, the NLS sequence of MeCP2 comprises an amino acid sequence as set forth in SEQ ID NO: 2 (RKAEADPQAIPKKRGRK).

According to specific embodiments, the polypeptide sequence containing an NLS sequence of MeCP2 can be naturally occurring molecule or a chimeric molecule.

Thus, according to specific embodiments, the polypeptide sequence containing an NLS sequence of MeCP2 is a MeCP2 polypeptide such as set forth in the following Accession Numbers: NP_001104262, NP_001303266, NP_004983, NP_001075448, NP_034918, NP_001075448 or NP_034918.

According to specific embodiments, the naturally occurring molecule can be a full protein sequence or a fragment thereof. According to specific embodiment, the naturally occurring molecule is comprised, either endogenously or exogenously, in a cell.

According to other specific embodiments, the polypeptide sequence containing an NLS sequence of MeCP2 is a chimeric molecule, such as an induced synthetic molecule comprising an amino acid sequence containing an NLS sequence of MeCP2 and a reporter gene which are heterologous. According to a specific embodiment, the chimeric molecule is comprised in a cell.

According to specific embodiments, the reporter gene comprises (attached or conjugated to) a detectable moiety.

According to specific embodiments, determining the effect of a test agent on binding of importin α5 to a polypeptide sequence containing an NLS sequence of MeCP2and/or on expression of a reporter gene comprising a polypeptide sequence containing an NLS sequence of MeCP2 comprises the detection of the detectable moiety.

Various types of detectable moieties may be conjugated to the polypeptide sequence containing an NLS sequence of MeCP2. These include, but not are limited to, a phosphorescent chemical, a hemiluminescent chemical such as luciferase and galactosidase, a fluorescent chemical (fluorophore) such as GFP, an enzyme, a fluorescent polypeptide, an affinity tag, and molecules (contrast agents) detectable by Positron Emission Tomagraphy (PET) or Magnetic Resonance Imaging (MRI).

Detection of the detectable moiety can be effected by methods and apparatuses well known in the art including, but not limited to flow cytometer, fluorescent plate reader, immunofluorescence confocal microscopy, fluorescence *in-situ* hybridization (FISH) and fluorescence resonance energy transfer (FRET) and luminescence plate reader.

The following non-limiting screening method can be used:
Recombinant importin α5 mutated to cancel self-binding of the auto-inhibitory IBB domain is produced and binding of fluorescently labeled polypeptide sequence containing an NLS sequence of MeCP2 (flNLS) to importin α5 is used to establish a fluorescence polarization (FP) based assay for small molecule HTS. Changes in FP of importin α5/flNLS upon formation or dissociation of the complex with test agents are then determined.

According to some embodiments, candidate agent selected according to the methods described above are tested for their biological activity, specificity and toxicity *in-vitro* in cell cultures or *in-vivo* in animal models. Such assays are well known in the art and are further described in details hereinabove and below and in the Examples section which follows.

Typically, the agents identified by the method described herein are anxiolytic agents.

Thus, according to specific embodiments, the screening method further comprising providing said test agent and testing an anxiolytic activity of same.

Typically, the agents identified by the method described herein are suitable for treating a disease that can benefit from up-regulating activity of SIP receptor, e.g. psychiatric stress disorder, MS, Rett syndrome, MeCP2 duplication syndrome.

Thus, according to specific embodiments, the screening method further comprising treating a medical condition that can benefit from up-regulating activity of SIP receptor with said test agent wherein said determining indicates said test agent inhibits activity of importin α5.

The agents, compounds and compositions of some embodiments of the invention can be administered to an organism *per se*, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the agent or compound accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, transmucosal, especially transnasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, intraperiotoneal, intranasal, or intraocular injections.

Conventional approaches for drug delivery to the central nervous system (CNS) include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Thus, according to specific embodiments, the agent or the composition disclosed herein is formulated for penetrating the blood brain barrier (BBB).

According to a specific embodiment, the agent capable of up-regulating activity and/or expression of LXR is formulated for penetrating the blood brain barrier (BBB).

Alternately, one may administer the pharmaceutical composition in a local rather than systemic manner, for example, via injection of the pharmaceutical composition directly into a tissue region of a patient.

Pharmaceutical compositions of some embodiments of the invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with some embodiments of the invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to some embodiments of the invention are conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of some embodiments of the invention may also be formulated in rectal compositions such as suppositories or retention enemas, using, e.g., conventional suppository bases such as cocoa butter or other glycerides.

Pharmaceutical compositions suitable for use in context of some embodiments of the invention include compositions wherein the active ingredients are contained in an amount effective to achieve the intended purpose. More specifically, a therapeutically effective amount means an amount of active ingredients effective to prevent, alleviate or ameliorate symptoms of a medical condition (e.g., psychiatric stress disorder, MS, Rett syndrome, MeCP2 duplication syndrome) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from *in vitro* and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

It will be appreciated that the agents and compositions comprising same of the instant invention can be co-administered (sequentially and/or simultaneously) with other therapeutic for the treatment of the indicated medical conditions. Thus, for example other therapeutics that can be administered in combination with the agents of the present invention for the treatment of psychiatric stress disorder include, but not limited to, selective serotonin reuptake inhibitors [SSRIs, e.g., sertraline (Zoloft®), paroxetine (Paxil®), or fluoxetine (Prozac®)]; noradrenergic drugs; monoamine oxidase inhibitors (MAOIs; e.g., ipronizad, phenylzine and pheniprazine); tricyclic antidepressants (TCAs; e.g., clomipramine, desipramine, imipramine, and serotonin); and norepinephrine re-uptake inhibitors (SNRIs; e.g., venalafazine, dluoxetine, and sibutramine). A non-limiting example of a therapeutic that can be administered in combination with the agents of the present invention for the treatment of MS is Copaxone.

Thus, according to an additional or an alternative aspect of the present invention there is provided a method of treating a psychiatric stress disorder in a subject in need thereof, the method comprising administering to the subject a therapeutically effective amount of an agent capable of up-regulating activity and/or expression of LXR and a selective serotonin reuptake inhibitor (SSRI), thereby treating the psychiatric stress disorder in the subject.

According to an additional or an alternative aspect of the present invention there is provided an agent capable of up-regulating activity and/or expression of LXR and a selective serotonin reuptake inhibitor (SSRI), for use in treating a psychiatric stress disorder in a subject.

According to specific embodiments, the SSRI is fluoxetine (Prozac®).

According to specific embodiments, the combination of the agent capable of up-regulating activity and/or expression of LXR and SSRI described herein has a combined improved activity. Assays for determining additive effect or synergy are well within the capabilities of the skilled artisan and are further described hereinabove and bellow.

According to specific embodiments, the combination of the agent capable of up-regulating activity and/or expression of LXR and SSRI has a synergistic effect as compared to the administration of only one of the agents.

Thus, according to specific embodiments, the agent capable of up-regulating activity and/or expression of LXR and/or the SSRI is administered in less than the FDA recommended does for treatment as a monotherapy.

According to specific embodiments, the SSRI is administered in a dose below 10 mg/kg.

According to specific embodiments, the SSRI is administered in a dose below 5 mg/kg.

According to specific embodiments, the SSRI is administered in a dose of 0.5-5 mg/kg.

According to specific embodiments, agent capable of up-regulating activity and/or expression of LXR is administered in a dose below 25 mg/kg.

According to specific embodiments, agent capable of up-regulating activity and/or expression of LXR is administered in a dose of 2-20mg/kg.

Compositions of some embodiments of the invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

According to another aspect of the present invention there is provided an article of manufacture or a kit identified for the treatment of a disease that can benefit from up-regulating activity of SIP receptor comprising a packaging material packaging at least two agents selected from the group consisting of:
(i) an agent capable of down-regulating activity and/or expression of importin α5;
(ii) an agent capable of down-regulating activity and/or expression of MeCP2;
(iii) an agent capable of up-regulating activity and/or expression of liver X receptor (LXR);
(iv) an agent capable of up-regulating activity and/or expression of SIP receptor; and
(v) alvespimycin or a structural analog thereof.

The packaging material may comprise at least one, at least two or at least three containers for packaging the agents.

According to specific embodiments, each container contains one agent.

According to other specific embodiments, several agents are packaged in a co-formulation.

The article of manufacture or kit may be accompanied by instructions for use.

As used herein the term "about" refers to ± 10 %.

The terms "comprises", "comprising", "includes", "including", "having" and their conjugates mean "including but not limited to".

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.
Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### MATERIALS AND METHODS

***Animals*** - All animal procedures were performed in accordance with Weizmann IACUC regulations. Importin α single mutants for importin α1, α3, α4, α5 and α7 were generated by conventional gene deletion strategies [Shmidt, T. et al. Nat. Cell Biol. 9, 1337-8; author reply 1339 (2007); Gabriel, G. et al. Nat. Commun. 2, 156 (2011); and Rother, F. et al. PLoS One 6, e18310 (2011)]. C57BL/6 and BALB/c mice were purchased from Harlan (Israel). Mice were kept at 24.0±0.5 °C in a humidity-controlled room under a 12 hours light-dark cycle with free access to food and water. Experiments were carried out on 2-5 months old animals.

***Pharmacological treatments*** - All drugs were administered by intraperitoneal injection (I.P.). FG7142 (Sigma-Aldrich, #E006-100MG) was administered as described previously [Evans, A. K. & Lowry, C. A. CNS Drug Rev. 13, 475-501 (2007)] at a final concentration of 5 mg / kg body weight 30 minutes before behavioral testing. β-Sitosterol (Sigma-Aldrich, #S1270-25MG) was dissolved in corn oil at a final concentration of 100 mg / kg. Dose-response data are shown in Figure 9A-B. PF-543 (Cayman Chemicals, #17034) was dissolved in 2 % DMSO/PBS 0.01 M and administered at a final dosage of 10 mg / kg. Fingolimod (FTY-720, Sigma-Aldrich, #SML0700-5MG) and Siponimod (Cayman Chemical, Cat# 22057) were dissolved in saline solution and administered at a final dosage of 0.1 mg / kg or 0.05 mg/kg when indicated, as described in Deogracias, R. et al. Proc. Natl. Acad. Sci. U. S. A. 109, 14230-5 (2012). Fluoxetine (Fluoxetine hydrochloride, Sigma-Aldrich, #F132), a serotonin reuptake inhibitor (SSRI), was dissolved in saline solution; and anxiety-reducing properties were tested following three weeks of treatment.

***RNA expression analysis (RNA-Seq)*** - Total RNA was extracted from hippocampi dissected from either importin α5 or wild type animals treated with vehicle or FG7142 injection 30 minutes prior to dissection (4 experimental groups, 3 animals per each group and replicate, 3 replicates of the entire set) using the RNAqueous-Micro Kit (Ambion) according to manufacturer's instructions. Replicates of high RNA integrity (RIN ≥ 7) were processed for RNA-Seq on an Illumina HiSeq (read type: SR60 V4) at the Crown Institute for Genomics (G-INCPM, Weizmann Institute of Science). Data was analyzed using DESeq software.

***Gene expression analysis by RT-qPCR*** - Total RNA from mouse brain tissue was extracted using the Ambion RNAqueous-Micro total RNA isolation kit (Life Technologies Corp.). RNA purity, integrity (RIN > 7) and concentration was determined, and 100 ng of total RNA was then used to synthesize cDNA using SuperScript III (Invitrogen). RT-qPCR was performed on a ViiA7 System (Applied Biosystems) using PerfeCTa SYBR Green (Quanta Biosciences, Gaithersburg, USA). Forward/Reverse primers were designed for different exons, and the RNA was treated with DNase H to avoid false-positives. Amplicon specificity was verified by melting curve analysis. All RT-qPCR reactions were conducted in technical triplicates and the results were averaged for each sample, normalized to *Actb* levels, and analyzed using the comparative ΔΔCt method [Livak, K. J. & Schmittgen, T. D. Methods 25, 402-8 (2001)]. The following primers (*Mus musculus*) were used: *Sphkl (F:* CAGTGGGCACCTTCTTTC, SEQ ID NO: 4 and R: CTTCTGCACCAGTGTAGAGGC, SEQ ID NO: 5), *Sphk2 (F:* TAGATGGGGAGTTAGTGGAGTATG, SEQ ID NO: 6 and R: TGCTTTTAGGCTCGTTCAGG, SEQ ID NO: 7), *Actb (F:* GGCTGTATTCCCCTCCATCG, SEQ ID NO: 8 and R: CCAGTTGGTAACAATGCCATGT, SEQ ID NO: 9).

***Transcription Factor Binding Site (TFBS) analysis*** - TFBS enrichment was evaluated using FMatch (geneXplain) on gene sets with fold changes of 1.5 or more and their corresponding background sets. Promoter sequences were scanned from 600 base pairs (bp) upstream to 100 bp downstream of the predicted transcription start site for each such gene, and TFBS were identified with TRANSFAC 9. TFBS enrichment in test versus background sets was assessed by t-test with p-value threshold of 0.05.

***Immunofluorescence*** - Mice were deeply anaesthetized with pentobarbital (100 mg / kg) and transcardially perfused (NaCl for 1 min, followed by 4 % paraformaldehyde/PBS 1x for 10 minutes). Brains were removed, fixed for 5 hours, cryoprotected in 20 % sucrose for additional 24 hours and then frozen at -80 °C followed by cryostat sectioning (20 µm coronal sections). Sections were collected in separate sets for immunohistochemistry, so that each set contained every fifth serial section. Briefly, sections were rehydrated, permeabilized (0.1 % Triton X-100 PBS IX), blocked (7 % normal goat serum) and incubated overnight at room temperature with the indicated primary antibody. Sections were subsequently incubated with the respective secondary antibody and mounted with an aqueous-based mounting solution (Immumount, Sigma). Anti-LXR-a (1:000, Abcam ab3585), anti-LXR-β (1:000, ThermoFisher Scientific PA1-333), Rabbit anti-MECP2-e1 [1:1000, Kaddoum et al. F1000Research 2, 204 (2013)] were used as primary antibodies. Goat anti-rabbit or goat anti-mouse Alexa 488 and goat anti-rabbit or anti-mouse Alexa 546 (1:200, Molecular Probes) were used as secondary antibodies. DAPI (4.6-diamino-2-phenolindol dihydrochloride) counterstaining was performed on all sections, in order to visualize the nucleus.

***Image processing, cell counting and colocalization analysis*** - Images were acquired on an Olympus FV1000 inverted confocal (Olympus, Tokyo) with Fluoview (FV10-ASW 4.1) software. In general, brain slices were scanned using camera settings identical for all genotypes in a given experiment. Images were imported into the *Fiji* version (www(dot)fiji(dot)sc) of the *ImageJ* software for threshold subtraction and subsequent analyses as further described hereinbelow.
Cell counting: The number of MECP2/DAPI double-positive cells was counted on 60X magnification pictures in the CA3 field of the hippocampus and the M1/M2 cortical areas using the nuclear-biased sampling procedure [Weibel, E. R. in Stereological methods. Vol.1 (Academic Press, London, 1979)]. A neuron was considered to be MECP2-positive if it met 3 criteria: 1) the nucleus size (diameter) ranged above 10 µm; 2) the nucleus (DAPI-positive object) was located inside the counting frame; and 3) a portion of the nucleolus was in focus between the top and bottom boundaries of the counting frame or outside the guard zone. Coronal sections of 20 µm thickness were processed to avoid potential double counting. Briefly, the images were converted to 8-bit, then thresholded and a region of interest (ROI) counting area was drawn on the image. From each animal, 5 regularly spaced sections were selected for quantitative analysis. Cell numbers are expressed as the total number of MECP2/DAPI-positive neurons counted in the CA3 hippocampal region and the motor cortical areas M1/M2. For each animal, the total neuron number was counted and the average value calculated. Colocalization analysis: Fiji contains a number of preinstalled plugins including a procedure for colocalization analysis, designated as "Colocalization Threshold", which calculates a variety of colocalization parameters such as the Mander's colocalization coefficient [Costes, S. V et al. Biophys. J. 86, 3993-4003 (2004)] based on pixel intensity correlation measurements. The background of both channels was subtracted. The channels were equalized to the intensity range to compensate for potential intensity differences between the channels. Following, the "Colocalization Threshold" plugin was run without a region of interest or mask. Numerical correlation parameters were recorded, as well as the 2D intensity histogram for visualization of the correlation between the two channels.

***Behavioral profiling*** - All data acquisition and analyses were performed during "subjective" nights (i.e. in rooms put under a reverse cycle illumination condition) to fit with the active phase of the mice. Mice were maintained under reverse cycle for at least 2 weeks before the tests. Mice were then habituated to the test rooms (∼10lx, 65dB white noise unless otherwise stated) 2 hours prior to each tests. A recovery period of at least 1 day was taken between different behavioral assays.

***Home-cage locomotion test*** - Home-cage locomotor activity was monitored over 72 consecutive hours using the InfraMot system (TSE Systems, Germany), which tracks the spatial displacement of body-heat images (infra-red radiation) over time [Neufeld-Cohen, A. et al. Proc. Natl. Acad. Sci. U. S. A. 107, 19020-19025 (2010); and Cooper, A. et al. Proc. Natl. Acad. Sci. U. S. A. 109, 2642-7 (2012)]. The first 24 hours were used as a habituation phase. The last 48 hours (2 consecutive dark/light cycles) were analyzed and the corresponding mouse activity calculated.

***Open field (OF)*** - Assessment of motility and anxiety-like behaviors were assaying in OF [Neufeld-Cohen, A. et al. Proc. Natl. Acad. Sci. U. S. A. 107, 19020-19025 (2010); and Cooper, A. et al. Proc. Natl. Acad. Sci. U. S. A. 109, 2642-7 (2012)]. The total distance moved (cm), the time spent (global, center, border; s), center/border ratio, mean speed (cm/s), percentage of time spent moving vs. rest in the different defined area were recorded using VideoMot2 (TSE System, Germany) or Ethovision XT11 (Noldus Information Technology, The Netherlands). OF was performed under 120lx for the assessment of anxiety-related behaviors or 6lx for the study of general locomotion. OF raw data were further analyzed with *COLOR*cation [Dagan, S. Y. et al. J. Neurosci. Methods 270, 9-16 (2016)], allowing the unbiased study of mice activity based on group heat-maps.

***Elevated plus maze (EPM)*** - The EPM contains two open arms and two enclosed ones connected by a central square. Exploration on the open arms is reduced in high anxiety states while it is increased in low anxiety states [Walf, A. A. & Frye, C. A. Nat. Protoc. 2, 322-8 (2007)]. Mice were placed on the central platform facing one of the "open" arms to initiate a 5-minutes test session and the time spent in each arm was measured. The test was performed under 7lx and 65dB white noise. The number of entries into, the time spent/distance on the open arms and the percentage of the open arm entries compared with total number of arm entries were recorded using the VideoMot2 software (TSE System, Germany) or the Ethovision XT11 software (Noldus Information Technology, The Netherlands).

***Acoustic startle reflex (ASR)*** - Rodent ASR is a major body muscle contraction response to a loud and abrupt sound. Enhanced ASR is a hallmark of increased fear and/or sustained anxiety [Koch, M. Prog. Neurobiol. 59, 107-28 (1999)]. An ASR apparatus (StartleResponse, TSE Systems, Germany) consisting of a sound-attenuated, well-ventilated cabinet was used. Sessions started with a 5-minutes acclimation period with background white noise (65 dB) maintained throughout. During the last 2 minutes of this period an individual activity baseline was recorded. Overall, 32 startle stimuli (120 dB, 40ms; inter-trials interval: randomly varying, 12-30 s) were presented; the stimuli presentation is divided into three 'Blocks': Blocks 1 and 3 consisted of 6 startle stimuli each, whereas Block 2 consisted of 10 startle stimuli and 10 'no stimuli' (65dB (A), 40ms.; i.e. equivalent to the background white noise) that were presented in a quasi-random manner. Three indices were recorded for each of the blocks: (1) RT ASR (ms) =mean reaction time to respond to the startle stimuli (latency to exceed the individual activity baseline); (2) The average response amplitude produced in response to the startling stimuli; and (3) the latency to reach the maximal response (ms).

***Fear conditioning*** - Contextual fear conditioning was carried out in a computer-controlled system (TSE Systems, Germany) over three days as previously described [Neufeld-Cohen, A. et al. Proc. Natl. Acad. Sci. U. S. A. 107, 19020-19025 (2010)], briefly:
1) Habituation - on the first day, mice were habituated for 5 minutes to the fear conditioning chamber, a clear Plexiglas cage (21 cm × 20 cm × 36 cm) with a stainless steel floor grid within a constantly illuminated (250 lx) fear-conditioning housing.
2) Conditioning - conditioning took place on day 2 in one 5-minutes training session. Mice initially explored the context for 2 minutes. Thereafter, mice were exposed to two pairings of a co-terminating tone [conditioned stimulus (CS): 30 s, 3,000 Hz, pulsed 10 HZ, 80 dB] and shock [unconditioned stimulus (US): 0.7 mA, 2 s, constant current) with a fixed ITI of 60 s. The US was delivered through the metal grid floor. Mice were removed from this chamber 1 minute following the last CS-US pairing. The chamber was cleaned with 10 % ethanol prior to each session. The ventilating fan of the conditioning box housing provided a constant auditory background noise [white noise, 62 dB].
3) Testing - Context dependent memory was tested 24 hours following the conditioning by re-exposure to the conditioning box for 5 minutes without any stimuli.

***Accelerated rotarod*** - The ROTOR-ROD™ system (83x91x61 - SD Instruments, San Diego) was used to measure motor coordination and balance in mice [Crawley, J. N. Neuron 57, 809-18 (2008)]. Mice were subjected to 3 trials, with 5 minutes inter-trial intervals. Rotarod acceleration was set at 20rpm in 240 s. Latency to fall was recorded in number of seconds (s). An average of the 3 trials was calculated.

***Wire hanging*** - The wire hanging test examines motor neuromuscular impairment and motor coordination [Gomez, C. M. et al. J. Neurosci. 17, 4170-9 (1997); and Rafael, J. A. et al. Mamm. Genome 11, 725-728 (2000)]. Forepaws of the tested mouse were hung on the middle of an elevated metal wire (diameter = 2 mm, length = 90 cm), 80 cm above of a water-filled tank. Traction was determined as the ability not to drop from the wire and to remain stable and hanging. The time (s) until the mouse completely released its grip was recorded.

***Pole test*** - The pole test assesses basal ganglia-related movement disorders in mice [Matsuura, K., et al. J. Neurosci. Methods 73, 45-8 (1997)]. Briefly, mice were placed head-up on top of a 50 cm-long horizontal pole (1 cm in diameter). The base of the pole was placed in the home cage so that when the pole is flipped downward, animals orient themselves (turn) and descend the length of the pole back into their home cage. Mice received 2 days of training that consisted of five trials for each session. On the test day, animals received five trials, and the time to orient downward T_{Turn} was measured. If the mouse was not able to turn or dropped, a cutoff value of 120 s was attributed.

***EAE Induction and monitoring* -** All animal protocols were approved by the Animal Research Committee (IACUC) of The Weizmann Institute of Science. EAE was induced by a subcutaneous injection of 200 µg emulsion of MOG peptide 35-55 [MEVGWYRSPFSRVVHLYRNGK (SEQ ID NO: 10), 98 % purity; Genscript) and Complete Freund Adjuvant (CFA; Sigma Aldrich, ref. F5881-10ML) in a skin fold at the back of the neck of C57BL6 mice, followed by intraperitoneal injection of 400 ng B. pertussis toxin (PTX) on days 0 and 2, in order to allow the breach of the blood brain barrier and the immune cells to reach the CNS. EAE-Induced mice were monitored daily from day 7 following disease induction (d9) until sacrifice on day 15. EAE severity was assessed by the following scoring method: 0 = healthy mouse, 1 = flaccid tail, 2 = hind limb weakness, 3 = paralysis of one or both hind limbs, 4 = forelimb paralysis, and 5 = death. Mice were withdrawn from the experiment when significant clinical signs (i.e. average score of 3) were manifested.

***Statistical analysis*** - A normality test (Shapiro-Wilk test) was applied to all data before analysis for statistical significance. Datasets which passed the normality test were subjected to parametric analysis. For 2-groups analyses, unpaired Student's t test was used. Analysis of multiple groups was made using the ANOVA method. The choice between one- or two-way ANOVA was based on the requirements for identification of specific factors' contribution to statistical differences between groups and were followed as specified in the figure legends by the Tukey, the Sidak or the Dunnett's post hoc analysis tests. Datasets that did not pass the normality test were subjected to the Mann-Whitney or the Kruskal-Wallis test followed by Dunn's multiple comparisons test. Potential outliers were identified and discarded using the ROUT method with a Q (maximum desired false discovery rate) of 1%. All analyses were performed using GraphPad Prism version 8.00 for Windows (GraphPad Software, La Jolla, California, USA, https://www(dot)graphpad(dot)com/). All statistical parameters and P-values for specific analyses are reported in the figure legends.

### EXAMPLE 1

### IMPORTIN α5 KNOCKOUT MICE PRESENT AN ANXIOLYTIC PHENOTYPE

Behavioral phenotyping was performed on five single gene importin α knockout mouse lines, all of which are viable and reach adulthood. A panel of assays was used to follow home cage locomotion and circadian activity, novelty induced locomotion, anxiety behaviors, and associative memory capacities (Figures 1A-C). Strikingly, importin α5 KO animals displayed a specific phenotype characterized by reduced anxiety in multiple tests, including open field (OF), elevated plus maze (EPM) and acoustic startle response (ASR). OF and EPM are based on the monitoring of mouse locomotion activity when placed in a novel environment offering both secured and anxiogenic areas^{18,19}. In contrast, the ASR monitors an involuntary reflex-based response to a sudden loud sound²⁰, in this case to a loud auditory stimulation of 120 dB.

Importin α5 KO mice explored and reared significantly more than their wild type (WT) littermates in the central anxiogenic area of the OF (Figure 2A-C). The specificity of the result was further confirmed by the absence of effects on total distance travelled or velocity of movement, as well as time spent in the OF anxiogenic area under low illumination (6 lux, assessing general motor activity²¹), ruling out possible general movement phenotypes in these mice (Figure 3A). In line with the OF results, importin α5 KO mice showed a significant increase in distance covered, time spent and number of visits in the open arms of the EPM compared to their WT littermates (Figures 2D-F and 3B). In the ASR test, Importin α5 KO mice exhibited both a significant increase in reaction time and a significantly reduced response amplitude compared to their WT littermates (Figures 2G-I). The importin α5 KO was the only line out of the five importin α mutants tested that revealed a consonant phenotype in all three anxiety tests, further emphasizing the robustness of the findings (Figures 1A-C). Moreover importin α5 KO animals did not reveal any differences in home cage activity or performance on rotarod, wire hanging, pole tests or in the fear conditioning paradigm (Figures 3C-G), further supporting a specific reduction in anxiety levels in importin α5 null mice, without changes in other physiological parameters of overall motor performance, coordination or activity.

### EXAMPLE 2

### THE EFFECTS OF IMPORTIN α5 KO ON TRASNCRIPTION AND NUCLEAR LOCALIZATION: THE IDENTIFICATION OF THE IMPORTIN α5- MeCP2/LXR- Sphkl PATHWAY

To obtain further insights on the mechanisms underlying the reduction in anxiety in importin α5 KO mice, their response to the anxiogenic drug FG7142²⁴ was tested. FG7142 increases anxiety by a partial agonist effect on the GABA-A receptor, hence provides a convenient tool to test if anxiolysis in importin α5 KO animals can be pharmacologically modulated. As shown in Figures 4A-F, FG-7142 indeed reversed anxiolysis in importin α5 KO animals in OF tests, without affecting overall movement velocity in the test. In the next step, hippocampal transcriptomes were analyzed by RNA-seq. Specifically, RNA was extracted from total hippocampi, dissected from either importin α5 or WT animals injected with vehicle or FG-7142. Analyses of the resulting datasets revealed significant changes in the expression of 121 genes under basal conditions in importin α5 null mice (Figures 5A-B), and a larger cohort of almost 600 transcripts that were differentially regulated by FG7142 treatment in KO versus WT mice (Figures 6A-C). A computational analyses of these differential groups was performed to reveal the underlying transcriptional networks, using the TRANSFAC FMatch promoter analysis tool, and the enriched pathways and signaling networks using the Ingenuity tool (Figures 6C-D). The resulting list of transcription factors (TF) that may control expression of importin α5 KO differentially expressed genes (DEGs) features a number of interesting candidates, including MeCP2 and the liver X receptor (LXR) family of nuclear receptors (Figure 5C). The Ingenuity pathway analyses shown in Figure 6D directed attention to a lipid signaling network containing the Sphingosine kinase 1 (Sphkl), the promoter of which possesses overlapping binding sites for both MeCP2 and LXRs (Figure 5D). The up-regulation of Sphkl in importin α5 KO hippocampus is intriguing, since previous studies have implicated Sphkl, its product sphingosine 1 phosphate (SIP), and its receptors in synaptic plasticity and presynaptic functions²⁵⁻²⁷.

MeCP2 is a transcriptional modulator best known as the gene mutated in Rett syndrome²⁸. The protein is widely expressed and abundant in the brain, primarily in mature neurons²⁹ where it colocalizes with methylated heterochromatic foci in the nucleus³⁰. LXRs are ligand-activated TFs activated by naturally occurring cholesterol derivatives³¹ and have been implicated in control of neurogenesis and survival of mature neurons^{32,33}. A series of immunofluorescent stainings was performed to assess MeCP2 expression in hippocampal and cortical neurons in the brain of importin α5 KOs and their WT littermates. As shown in Figure 5E, MeCP2 localization was strictly nuclear and highly co-localized with DAPI-positive heterochromatin in WT neurons (on average 78 % of nuclei with MeCP2/DAPI colocalization in the hippocampus CA3 region and 68 % in the motor cortex). In contrast, importin α5 KO brains revealed a significant reduction of MeCP2/DAPI double-positive nuclei in hippocampus CA3 (22 %, *p* < 0.01) and to a lesser extent in the motor cortex (39.2 %, *p* < 0.05) (Figures 5F-G) while the total number of DAPI neuronal nuclei remained unchanged (Figure 5H). MeCP2 was significantly excluded from nuclei in KO hippocampal neurons, as confirmed by Mander's coefficient analyses in CA3 (Figure 5I), whereas in the cortex the reduction was not significant (Figure 5J). This may reflect diverse importin ensembles in specific neuronal subtypes, since multiple importins can carry out nuclear import of MeCP2³⁴. Nonetheless, importin α5 is critical for MeCP2 nuclear import in specific brain regions such as the hippocampus.

Previous gene expression mapping revealed low LXRα and high LXRβ expression in the hippocampus³⁵. Immunostaining with isoform-specific antibodies confirmed the predominance of LXRβ over LXRα in the hippocampal CA3 region and showed that both isoforms are predominantly cytoplasmic (Figure 7A). The nuclear-localized fraction of LXRβ did not differ significantly between WT and importin α5 KO hippocampus or cortex (Figure 7B-C).

Taken together, the above data suggest the following model for importin α5 involvement in anxiety regulation (Figure 8A): Expression levels of Sphkl are determined by the relative amounts of MeCP2 and LXRs in neuronal nuclei. Removal of importin α5 reduces MeCP2 levels in the nucleus, thereby enabling LXR-induced up-regulation of Sphkl and subsequent increase of SIP activation of SIP receptors.

### EXAMPLE 3

### PHARMACOLOGICAL TARGETING OF THE IMPORTIN α5- MeCP2/LXR-Sphkl PATHWAY FOR TREATING ANXIETY

The model presented in Figure 8A predicts that LXR and S1P receptor agonists should have anxiolytic effects, while Sphkl antagonists should be anxiogenic. Moreover, an effective Sphkl antagonist should attenuate the anxiolytic phenotype in importin α5 KO mice. Hence, the effects of three such molecules - β-sitosterol (a plant-derived nutraceutical that can target LXRs³⁶), fingolimod (FTY720, an SIP receptor agonist currently used as a drug in multiple sclerosis³⁷) and PF-543 (a selective inhibitor of Sphk1³⁸) were tested. β-sitosterol, applied by injection, had clear dose-dependent anxiolytic effects in the OF test (Figure 9A-C) that were apparent 1 hour post-injection and persisted until 6 hours following injection (Figure 8C-D). Similar results were obtained in the EPM assay (Figure 9D-F). In addition, co-administration of β-sitosterol with a conventional anxiolytic drug, fluoxetine, may serve to reduce dose requirements and minimize possible side effects. A dose-response analysis with fluoxetine showed that doses of 10 mg / kg and above had significant anxiolytic effects in mice, while doses of 5 mg / kg and below had no apparent effect (Figures 16A-D). Strikingly, a synergic anxiolytic effect of sub-efficacious dosage combinations of 5mg / kg fluoxetine and 10, 20 or 50 mg / kg β-sitosterol were observed (Figures 17A-G).

Following, the effects of the SIP receptor agonist fingolimod were tested, using a dose regime previously employed for trophic factor studies in the hippocampus³⁹. Fingolimod indeed markedly reduced anxiety levels in both the EPM (Figure 10A-C) and the OF assays (Figures 11A-C). These assays were repeated on BALB/c mice, a line with intrinsically higher anxiety levels⁴⁰ than the C57Bl6 mice used thus far. Most strikingly, BALB/c mice likewise exhibited significantly reduced anxiety responses upon fingolimod treatment, despite their clearly apparent higher basal anxiety measures (Figures 10D-F and 11D-F). Similarly, the SIP receptor agonist siponimod markedly reduced anxiety levels in both the OF (Figure 13A) and EPM (Figure 13B) assays. Importantly, a combined treatment with fingolimod and β-sitosterol had an additive anxiolytic effect (Figure 14). Finally, the effects of the Sphkl inhibitor PF-543 upon i.p. injection in WT and importin α5 KO animals were evaluated. Sphkl inhibition did not influence anxiety-related behavior of WT mice in the EPM, but significantly decreased the distance travelled and the time spent in the open arms of importin α5 KO animals (Figures 10H-I). Hence, Sphkl inhibition attenuated the anxiolytic phenotype of importin α5 KO mice.

### EXAMPLE 4

### ALVESPIMYCIN FOR TREATING ANXIETY

The importin α5 KO differentially expressed gene lists were compared to the Broad Institute Connectivity Map (CMap; www(dot)portals(dot)broadinstitute(dot)org/cmap/), a database of drug-affected cell line transcriptomes [Lamb, J. et al. Science 313, 1929-35 (2006)]. CMap enables identification of approved drugs that may mimic the compared activity due to similarities with the query transcriptome profile. 19 drug candidates with high CMap scores (>0.75) were found and further filtered by penetrability across the blood-brain barrier, possibility of oral delivery, and the likelihood of side effects due to pleiotropic activities of the drug. Three of the compounds that met the desired criteria were alvespimycin, β-sitosterol and fluspirilene. β-sitosterol anxiolytic effects are described in Example 3 herein above. Fluspirilene is a neuropsychiatric drug previously indicated as anxiolytic.

All three drugs were tested for their effects on behavior in the OF test 1 hour following i.p. injections in comparison with vehicle controls. All three drugs affected mouse behavior in the OF test. Specifically, alvespimycin-treated animals showed marked yet variable differences from vehicle-treated controls, reaching statistical significance for rearing activity in the center in the single dosage regime tested so far (Figure 12A). A larger cohort of animals (n=10 mice per group, figure 12B) confirmed an effect of alvespimycin on increasing the number of rearings in the OF center (WT: 0.6 ± 0.34; Importin α5 KO: 6.2 ± 2.07; *P* < 0.01). In addition, alvespimycin increased the number of visit (*P*=0.09) and the time spent in the center area (*P*=0.052) and the latency to access it (*P*=0.08) (Figure 12B).

### EXAMPLE 5

### PHARMACOLOGICAL TARGETING OF THE IMPORTIN α5- MeCP2/LXR-Sphkl PATHWAY FOR TREATING MULTIPLE SCLEROSIS

The data presented in the Examples hereinabove provided both molecular and pharmacological evidence for the involvement of the importin α5 - MeCP2/LXR - Sphkl - SIP receptor pathway in anxiety. Hence, it was suggested that targeting the importin α5 - MeCP2/LXR - Sphkl - SIP receptor pathway can be used for the treatment of medical disorders that can benefit from up-regulating SIP receptor activity such as multiple sclerosis.

To this end, the effect of a combined treatment with fingolimod and β-sitosterol on the severity of an EAE mouse model of multiple sclerosis was evaluated. As shown in Figures 15A-C, Fingolimod and β-sitosterol treatment reduced EAE severity and body weight loss and increased survival, compared to the vehicle control.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting. In addition, any priority document(s) of this application is/are hereby incorporated herein by reference in its/their entirety.

### REFERENCES

### (other references are cited throughout the application)

1. Dias, B. G., Banerjee, S. B., Goodman, J. V. & Ressler, K. J. Towards new approaches to disorders of fear and anxiety. Curr Opin Neurobiol 23, 346-352 (2013).
2. Griebel, G. & Holmes, A. 50 years of hurdles and hope in anxiolytic drug discovery. Nat Rev Drug Discov 12, 667-687 (2013).
3. West, A. E. & Greenberg, M. E. Neuronal activity-regulated gene transcription in synapse development and cognitive function. Cold Spring Harbor perspectives in biology 3 (2011).
4. Lim, A. F., Lim, W. L. & Ch'ng, T. H. Activity-dependent synapse to nucleus signaling. Neurobiol Learn Mem 138, 78-84 (2017).
5. Panayotis, N., Karpova, A., Kreutz, M. R. & Fainzilber, M. Macromolecular transport in synapse to nucleus communication. Trends Neurosci 38, 108-116 (2015).
6. Miyamoto, Y., Yamada, K. & Yoneda, Y. Importin alpha: a key molecule in nuclear transport and non-transport functions. J Biochem 160, 69-75 (2016).
7. Pumroy, R. A. & Cingolani, G. Diversification of importin-alpha isoforms in cellular trafficking and disease states. Biochem J 466, 13-28 (2015).
8. Yasuhara, N. et al. Importin alpha subtypes determine differential transcription factor localization in embryonic stem cells maintenance. Dev Cell 26, 123-135 (2013).
9. Yasuhara, N. et al. Triggering neural differentiation of ES cells by subtype switching of importin-alpha. Nat Cell Biol 9, 72-79 (2007).
10. Perry, R. B. et al. Subcellular knockout of importin beta1 perturbs axonal retrograde signaling. Neuron 75, 294-305 (2012).
11. Ch'ng, T. H. et al. Activity-dependent transport of the transcriptional coactivator CRTC1 from synapse to nucleus. Cell 150, 207-221 (2012).
12. Rishal, I. & Fainzilber, M. Axon-soma communication in neuronal injury. Nat Rev Neurosci 15, 32-42 (2014).
13. Friedrich, B., Quensel, C., Sommer, T., Hartmann, E. & Kohler, M. Nuclear localization signal and protein context both mediate importin alpha specificity of nuclear import substrates. Mol Cell Biol 26, 8697-8709 (2006).
14. Ushijima, R. et al. Extracellular signal-dependent nuclear import of STAT3 is mediated by various importin alphas. Biochem Biophys Res Commun 330, 880-886 (2005).
15. Shmidt, T. et al. Normal brain development in importin-alpha5 deficient-mice. Nat Cell Biol 9, 1337-1338; author reply 1339 (2007).
16. Rother, F. et al. Importin alpha7 is essential for zygotic genome activation and early mouse development. PLoS One 6, e18310 (2011).
17. Gabriel, G. et al. Differential use of importin-alpha isoforms governs cell tropism and host adaptation of influenza virus. Nat Commun 2, 156 (2011).
18. Prut, L. & Belzung, C. The open field as a paradigm to measure the effects of drugs on anxiety-like behaviors: a review. European journal of pharmacology 463, 3-33 (2003).
19. Walf, A. A. & Frye, C. A. The use of the elevated plus maze as an assay of anxiety-related behavior in rodents. Nature protocols 2, 322-328 (2007).
20. Koch, M. The neurobiology of startle. Prog Neurobiol 59, 107-128 (1999).
21. Trullas, R. & Skolnick, P. Differences in fear motivated behaviors among inbred mouse strains. Psychopharmacology 111, 323-331 (1993).
22. Bannerman, D. M. et al. Hippocampal synaptic plasticity, spatial memory and anxiety. Nat Rev Neurosci 15, 181-192 (2014).
23. Tovote, P., Fadok, J. P. & Luthi, A. Neuronal circuits for fear and anxiety. Nat Rev Neurosci 16, 317-331 (2015).
24. Evans, A. K. & Lowry, C. A. Pharmacology of the beta-carboline FG-7,142, a partial inverse agonist at the benzodiazepine allosteric site of the GABA A receptor: neurochemical, neurophysiological, and behavioral effects. CNS drug reviews 13, 475-501 (2007).
25. Kanno, T. et al. Regulation of synaptic strength by sphingosine 1-phosphate in the hippocampus. Neuroscience 171, 973-980 (2010).
26. Chan, J. P. & Sieburth, D. Localized sphingolipid signaling at presynaptic terminals is regulated by calcium influx and promotes recruitment of priming factors. J Neurosci 32, 17909-17920 (2012).
27. Kempf, A. et al. The sphingolipid receptor S1PR2 is a receptor for Nogo-a repressing synaptic plasticity. PLoS Biol 12, e1001763 (2014).
28. Lyst, M. J. & Bird, A. Rett syndrome: a complex disorder with simple roots. Nat Rev Genet 16, 261-275 (2015).
29. Jung, B. P. et al. The expression of methyl CpG binding factor MeCP2 correlates with cellular differentiation in the developing rat brain and in cultured cells. J Neurobiol 55, 86-96 (2003).
30. Bertulat, B. et al. MeCP2 dependent heterochromatin reorganization during neural differentiation of a novel Mecp2-deficient embryonic stem cell reporter line. PLoS One 7, e47848 (2012).
31. Hong, C. & Tontonoz, P. Liver X receptors in lipid metabolism: opportunities for drug discovery. Nat Rev Drug Discov 13, 433-444 (2014).
32. Theofilopoulos, S. et al. Cholestenoic acids regulate motor neuron survival via liver X receptors. J Clin Invest 124, 4829-4842 (2014).
33. Theofilopoulos, S. et al. Brain endogenous liver X receptor ligands selectively promote midbrain neurogenesis. Nat Chem Biol 9, 126-133 (2013).
34. Baker, S. A., Lombardi, L. M. & Zoghbi, H. Y. Karyopherin alpha 3 and karyopherin alpha 4 proteins mediate the nuclear import of methyl-CpG binding protein 2. J Biol Chem 290, 22485-22493 (2015).
35. Gofflot, F. et al. Systematic gene expression mapping clusters nuclear receptors according to their function in the brain. Cell 131, 405-418 (2007).
36. Vanmierlo, T. et al. Plant sterols: Friend or foe in CNS disorders? Progress in lipid research 58, 26-39 (2015).
37. Chew, W. S., Wang, W. & Herr, D. R. To fingolimod and beyond: The rich pipeline of drug candidates that target S1P signaling. Pharmacological research 113, 521-532 (2016).
38. Schnute, M. E. et al. Modulation of cellular S1P levels with a novel, potent and specific inhibitor of sphingosine kinase-1. Biochem J 444, 79-88 (2012).
39. Deogracias, R. et al. Fingolimod, a sphingosine-1 phosphate receptor modulator, increases BDNF levels and improves symptoms of a mouse model of Rett syndrome. Proc Natl Acad Sci U S A 109, 14230-14235 (2012).
40. Oliverio, A., Eleftheriou, B. E. & Bailey, D. W. A gene influencing active avoidance performance in mice. Physiol Behav 11, 497-501 (1973).
41. Barnes, K. V. et al. Anxiety-like behavior in Rett syndrome: characteristics and assessment by anxiety scales. JNeurodev Disord 7, 30 (2015).
42. Ramocki, M. B. et al. Autism and other neuropsychiatric symptoms are prevalent in individuals with MECP2 duplication syndrome. Annals of Neurology 66, 771-782 (2009).
43. Stearns, N. A. et al. Behavioral and anatomical abnormalities in Mecp2 mutant mice: A model for Rett syndrome. Neuroscience 146, 907-921 (2007).
44. Samaco, R. C. et al. A partial loss of function allele of Methyl-CpG-binding protein 2 predicts a human neurodevelopmental syndrome. Human Molecular Genetics 17, 1718-1727 (2008).
45. Goffin, D. et al. Rett syndrome mutation MeCP2 T158A disrupts DNA binding, protein stability and ERP responses. Nature Neuroscience 15, 274-283 (2011).
46. Na, E. S. et al. A Mouse Model for MeCP2 Duplication Syndrome: MeCP2 Overexpression Impairs Learning and Memory and Synaptic Transmission. Journal of Neuroscience 32, 3109-3117 (2012).
47. Muller, C. P. et al. Brain membrane lipids in major depression and anxiety disorders. Biochim Biophys Acta 1851, 1052-1065 (2015).
48. Muhle, C., Reichel, M., Gulbins, E. & Kornhuber, J. Sphingolipids in psychiatric disorders and pain syndromes. Handbook of experimental pharmacology, 431-456 (2013).
49. Moreau, T. et al. Anxiety and Coping Strategy Changes in Multiple Sclerosis Patients Initiating Fingolimod: The GRACE Prospective Study. European neurology 77, 47-55 (2017).
50. Murrough, J. W., Yaqubi, S., Sayed, S. & Charney, D. S. Emerging drugs for the treatment of anxiety. Expert Opinion on Emerging Drugs 20, 393-406 (2015).

## Claims

1. A combination of an agent capable of up-regulating activity and/or expression of liver X receptor (LXR) and a selective serotonin reuptake inhibitor (SSRI), for use in treating a psychiatric stress disorder in a subject in need thereof.

2. The combination for use of claim 1, wherein said SSRI is fluoxetine.

3. The combination for use of any one of claims 1-2, wherein said agent capable of up-regulating activity and/or expression of LXR and/or said SSRI is administered in less than the FDA recommended dose for treatment as a monotherapy.

4. The combination for use of any one of claims 1-3, wherein said SSRI is administered at a dose of 0.5-5 mg/kg.

5. The combination for use of any one of claims 1-4, wherein said agent capable of up-regulating activity and/or expression of LXR is administered at a dose of 2-20 mg/kg.

6. The combination for use of any one of claims 1-5, wherein said agent capable of up-regulating activity and/or expression of LXR is formulated for penetrating the blood brain barrier (BBB).

7. The combination for use of any one of claims 1-6, wherein said LXR activity is translocation into the nucleus.

8. The combination for use of any one of claims 1-6, wherein said LXR activity is binding to the promoter of sphingosine kinase 1 (Sphkl).

9. The combination for use of any one of claims 1-6, wherein said agent capable of up-regulating activity of LXR is selected from the group consisting of β-sitosterol, campesterol, stigmasterol, fucosterol and brassicasterol, cholic acid (CA) and 24,25 EC.

10. The combination for use of any one of claims 1-6, wherein said agent capable of up-regulating activity of LXR is β-sitosterol.

11. The combination for use of any one of claims 1-10, wherein said psychiatric stress disorder is anxiety.

12. The combination for use of any one of claims 1-11, wherein said agent capable of up-regulating activity and/or expression of LXR and said SSRI are provided in a co-formulation.

13. The combination for use of any one of claims 1-11, wherein said agent capable of up-regulating activity and/or expression of LXR and said SSRI are provided in separate formulations.


SEQUENCE LISTING

<110>  Yeda Research and Development Co. Ltd.
       FAINZILBER, Michael
       PANAYOTIS, Nicolas

<120>  METHODS OF TREATING PSYCHIATRIC STRESS DISORDERS

<130>  84750

<150>  US 16/676,495
<151>  2019-11-07

<160>  12

<170>  PatentIn version 3.5

<210>  1
<211>  10
<212>  DNA
<213>  Artificial Sequence

<220>
<223>  Sphk1 promoter region which binds MeCP2

<400>  1
ttaactccgg                                                              10


<210>  2
<211>  17
<212>  PRT
<213>  Artificial Sequence

<220>
<223>  NLS sequence of MeCP2

<400>  2

Arg Lys Ala Glu Ala Asp Pro Gln Ala Ile Pro Lys Lys Arg Gly Arg
1               5                   10                  15


Lys




<210>  3
<211>  17
<212>  DNA
<213>  Artificial Sequence

<220>
<223>  Sphk1 promoter region which binds LXR

<400>  3
ccggttagtc acggaca                                                      17


<210>  4
<211>  18
<212>  DNA
<213>  Artificial Sequence

<220>
<223>  Single strand DNA oligonucleotide

<400>  4
cagtgggcac cttctttc                                                    18


<210>  5
<211>  21
<212>  DNA
<213>  Artificial Sequence

<220>
<223>  Single strand DNA oligonucleotide

<400>  5
cttctgcacc agtgtagagg c                                                21


<210>  6
<211>  24
<212>  DNA
<213>  Artificial Sequence

<220>
<223>  Single strand DNA oligonucleotide

<400>  6
tagatgggga gttagtggag tatg                                             24


<210>  7
<211>  20
<212>  DNA
<213>  Artificial Sequence

<220>
<223>  Single strand DNA oligonucleotide

<400>  7
tgcttttagg ctcgttcagg                                                  20


<210>  8
<211>  20
<212>  DNA
<213>  Artificial Sequence

<220>
<223>  Single strand DNA oligonucleotide

<400>  8
ggctgtattc ccctccatcg                                                  20


<210>  9
<211>  22
<212>  DNA
<213>  Artificial Sequence

<220>
<223>  Single strand DNA oligonucleotide

<400>  9
ccagttggta acaatgccat gt                                                22

<210>  10
<211>  21
<212>  PRT
<213>  Artificial Sequence

<220>
<223>  Synthetic peptide

<400>  10

Met Glu Val Gly Trp Tyr Arg Ser Pro Phe Ser Arg Val Val His Leu
1               5                   10                  15

Tyr Arg Asn Gly Lys
            20

<210>  11
<211>  9
<212>  PRT
<213>  Artificial Sequence

<220>
<223>  Meganuclease family motif

<400>  11

Leu Ala Gly Leu Ile Asp Ala Asp Gly
1               5

<210>  12
<211>  6
<212>  PRT
<213>  Artificial Sequence

<220>
<223>  Meganuclease family motif

<400>  12

Gly Ile Tyr Tyr Ile Gly
1               5